# EUROPEAN PATENT APPLICATION

(11) **EP 1 743 943 A1**
(43) Date of publication of application: **17.01.2007**
(21) Application number: 05720936.3
(22) Date of filing: 16.03.2005
(51) Int. Cl.: C12P 19/02, C12N 15/09

(54) **PROCESS FOR PRODUCING L-FUCULOSE AND PROCESS FOR PRODUCING L-FUCOSE**

(30) Priority: 17.03.2004 JP 2004077117
(71) Applicant: Ajinomoto Co., Inc., Tokyo 104-8315 (JP)
(72) Inventor: SUZUKI, Shunichi, Ajinomoto Co., Inc., Kawasaki-shi, Kanagawa 2108681 (JP); WATANABE, Kunihiko, Ajinomoto Co., Inc., Kawasaki-shi, Kanagawa 2108681 (JP)
(74) Representative: HOFFMANN EITLE
(86) International application number: PCT/JP2005/004701
(87) International publication number: WO 2005/087941

(57) **Abstract**

The present invention provides a method for producing L-fuculose and L-fucose which is suitable as an industrial method. L-Fuculose is synthesized from L-fucitol in the presence of a microorganism-derived protein having a dehydrogenase activity to produce L-fuculose from L-fucitol. The reaction system preferably contain NADH oxidase. L-Fuculose thus synthesized is then converted into L-fucose.

SEQUENCE LISTING

<110> AJINOMOTO CO., INC.

<120> Method for producing L-fuculose and L-fucose

<130> PAMA-06097

<140> JP2004-077117
<141> 2004-03-17

<160> 29

<170> PatentIn version 3.1

<210> 1
<211> 30
<212> DNA
<213> Artificial

<220>
<223> 5' primer to obtain fucI gene

<400> 1
gaagcatgca tgaaaaaaat cagcttaccg 30

<210> 2
<211> 30
<212> DNA
<213> artificial

<220>
<223> 3' primer to obtain fucI gene

<400> 2

tgttcaggcc cggaagcttg agcgaccggg                                    30

<210>  3
<211>  1818
<212>  DNA
<213>  Escherichia coli

<220>
<221>  CDS
<222>  (1)..(1818)
<223>  fucIH6

<400>  3
atg aga gga tcg cat cac cat cac cat cac gga tcc gca tgc atg aaa      48
Met Arg Gly Ser His His His His His His Gly Ser Ala Cys Met Lys
1               5                   10                  15
aaa atc agc tta ccg aaa att ggt atc cgc ccg gtt att gac ggt cgt      96
Lys Ile Ser Leu Pro Lys Ile Gly Ile Arg Pro Val Ile Asp Gly Arg
            20                  25                  30
cgc atg ggt gtt cgt gag tcg ctt gaa gaa caa aca atg aat atg gcg     144
Arg Met Gly Val Arg Glu Ser Leu Glu Glu Gln Thr Met Asn Met Ala
        35                  40                  45
aaa gct acg gcc gca ctg ctg acc gag aaa ctg cgc cat gcc tgc gga     192
Lys Ala Thr Ala Ala Leu Leu Thr Glu Lys Leu Arg His Ala Cys Gly
    50                  55                  60
gct gcc gtc gag tgt gtc att tcc gat acc tgt atc gcg ggt atg gct     240
Ala Ala Val Glu Cys Val Ile Ser Asp Thr Cys Ile Ala Gly Met Ala
65                  70                  75                  80
gaa gcc gct gct tgc gaa gaa aaa ttc agc agt cag aat gta ggc ctc     288
Glu Ala Ala Ala Cys Glu Glu Lys Phe Ser Ser Gln Asn Val Gly Leu
                85                  90                  95
acc att acg gta acg cct tgc tgg tgc tat ggc agt gaa acc atc gac     336
Thr Ile Thr Val Thr Pro Cys Trp Cys Tyr Gly Ser Glu Thr Ile Asp
            100                 105                 110
atg gat cca acc cgc ccg aag gcc att tgg ggc ttt aac ggc act gaa     384

Met Asp Pro Thr Arg Pro Lys Ala Ile Trp Gly Phe Asn Gly Thr Glu
        115                 120                 125
cgc ccc ggc gct gtt tac ctg gca gcg gct ctg gca gct cac agc cag 432
Arg Pro Gly Ala Val Tyr Leu Ala Ala Ala Leu Ala Ala His Ser Gln
    130                 135                 140
aaa ggc atc cca gca ttc tcc att tac ggt cat gac gtt cag gat gcc 480
Lys Gly Ile Pro Ala Phe Ser Ile Tyr Gly His Asp Val Gln Asp Ala
145                 150                 155                 160
gat gac aca tcg att cct gcc gat gtt gaa gaa aaa ctg ctg cgc ttt 528
Asp Asp Thr Ser Ile Pro Ala Asp Val Glu Glu Lys Leu Leu Arg Phe
                165                 170                 175
gcc cgc gcc ggt ttg gcc gtc gcc agc atg aaa ggt aaa agc tat ctg 576
Ala Arg Ala Gly Leu Ala Val Ala Ser Met Lys Gly Lys Ser Tyr Leu
            180                 185                 190
tcg ctg ggc ggc gtt tcg atg ggt atc gcc ggt tcc att gtt gat cac 624
Ser Leu Gly Gly Val Ser Met Gly Ile Ala Gly Ser Ile Val Asp His
        195                 200                 205
aac ttc ttt gaa tcc tgg ctg gga atg aaa gtc cag gcg gtg gat atg 672
Asn Phe Phe Glu Ser Trp Leu Gly Met Lys Val Gln Ala Val Asp Met
    210                 215                 220
acc gaa ctg cgt cgc cgt atc gat cag aag att tac gac gaa gcc gaa 720
Thr Glu Leu Arg Arg Arg Ile Asp Gln Lys Ile Tyr Asp Glu Ala Glu
225                 230                 235                 240
ttg gaa atg gca ctg gcc tgg gct gat aaa aac ttc cgc tat ggc gaa 768
Leu Glu Met Ala Leu Ala Trp Ala Asp Lys Asn Phe Arg Tyr Gly Glu
                245                 250                 255
gat gaa aat aac aaa cag tat caa cgt aat gcc gag caa agc cgc gca 816
Asp Glu Asn Asn Lys Gln Tyr Gln Arg Asn Ala Glu Gln Ser Arg Ala
            260                 265                 270
gtt ctg cgc gaa agt tta ctg atg gcg atg tgt atc cgc gac atg atg 864
Val Leu Arg Glu Ser Leu Leu Met Ala Met Cys Ile Arg Asp Met Met
        275                 280                 285
caa ggc aac agc aaa ctg gcc gat att ggt cgc gtg gaa gaa tca ctt 912
Gln Gly Asn Ser Lys Leu Ala Asp Ile Gly Arg Val Glu Glu Ser Leu
    290                 295                 300
ggc tac aac gcc atc gct gcg ggc ttc cag ggg caa cgt cac tgg acc 960

Gly Tyr Asn Ala Ile Ala Ala Gly Phe Gln Gly Gln Arg His Trp Thr
305                 310                 315                 320
gat caa tat ccc aat ggt gac acc gcc gaa gcg atc ctc aac agt tca      1008
Asp Gln Tyr Pro Asn Gly Asp Thr Ala Glu Ala Ile Leu Asn Ser Ser
                325                 330                 335
ttt gac tgg aat ggc gtg cgc gaa ccc ttt gtc gtg gcg acc gaa aac      1056
Phe Asp Trp Asn Gly Val Arg Glu Pro Phe Val Val Ala Thr Glu Asn
            340                 345                 350
gac agt ctt aac ggc gtg gca atg cta atg ggt cac cag ctc acc ggc      1104
Asp Ser Leu Asn Gly Val Ala Met Leu Met Gly His Gln Leu Thr Gly
        355                 360                 365
acc gct cag gta ttt gcc gat gtg cgt acc tac tgg tca cca gaa gca      1152
Thr Ala Gln Val Phe Ala Asp Val Arg Thr Tyr Trp Ser Pro Glu Ala
    370                 375                 380
att gag cgt gta acg ggg cat aaa ctg gat gga ctg gca gaa cac ggc      1200
Ile Glu Arg Val Thr Gly His Lys Leu Asp Gly Leu Ala Glu His Gly
385                 390                 395                 400
atc atc cat ttg atc aac tcc ggt tct gct gcg ctg gac ggt tcc tgt      1248
Ile Ile His Leu Ile Asn Ser Gly Ser Ala Ala Leu Asp Gly Ser Cys
                405                 410                 415
aaa caa cgc gac agc gaa ggt aac ccg acg atg aag cca cac tgg gaa      1296
Lys Gln Arg Asp Ser Glu Gly Asn Pro Thr Met Lys Pro His Trp Glu
            420                 425                 430
atc tct cag caa gag gct gac gct tgc ctc gcc gct acc gaa tgg tgc      1344
Ile Ser Gln Gln Glu Ala Asp Ala Cys Leu Ala Ala Thr Glu Trp Cys
        435                 440                 445
ccg gcg atc cac gaa tac ttc cgt ggc ggc ggt tac tct tcc cgc ttc      1392
Pro Ala Ile His Glu Tyr Phe Arg Gly Gly Gly Tyr Ser Ser Arg Phe
    450                 455                 460
ctt acc gaa ggc ggc gtc ccg ttc acc atg act cgt gtc aac atc atc      1440
Leu Thr Glu Gly Gly Val Pro Phe Thr Met Thr Arg Val Asn Ile Ile
465                 470                 475                 480
aaa ggc ctg gga ccg gta ctg caa atc gcg gaa ggc tgg agc gtg gaa      1488
Lys Gly Leu Gly Pro Val Leu Gln Ile Ala Glu Gly Trp Ser Val Glu
                485                 490                 495
ttg ccg aag gat gtg cat gac atc ctc aac aaa cgc acc aac tca acc      1536

Leu Pro Lys Asp Val His Asp Ile Leu Asn Lys Arg Thr Asn Ser Thr
            500                 505                 510
tgg cca acc acc tgg ttt gca ccg cgc ctc acc ggt aaa ggg ccg ttt      1584
Trp Pro Thr Thr Trp Phe Ala Pro Arg Leu Thr Gly Lys Gly Pro Phe
        515                 520                 525
acg gat gtg tac tcg gta atg gcg aac tgg ggc gct aac cat ggg gtt      1632
Thr Asp Val Tyr Ser Val Met Ala Asn Trp Gly Ala Asn His Gly Val
    530                 535                 540
ctg acc atc ggc cac gtt ggc gca gac ttt atc act ctc gcc tcc atg      1680
Leu Thr Ile Gly His Val Gly Ala Asp Phe Ile Thr Leu Ala Ser Met
545                 550                 555                 560
ctg cgt atc ccg gta tgt atg cac aac gtt gaa gag acc aaa gtg tat      1728
Leu Arg Ile Pro Val Cys Met His Asn Val Glu Glu Thr Lys Val Tyr
                565                 570                 575
cgt cct tct gcc tgg gct gcg cac ggc atg gat att gaa ggc cag gat      1776
Arg Pro Ser Ala Trp Ala Ala His Gly Met Asp Ile Glu Gly Gln Asp
            580                 585                 590
tac cgc gct tgc cag aac tac ggt ccg ttg tac aag cgt taa              1818
Tyr Arg Ala Cys Gln Asn Tyr Gly Pro Leu Tyr Lys Arg
        595                 600                 605

<210>  4
<211>  605
<212>  PRT
<213>  Escherichia coli

<400>  4
Met Arg Gly Ser His His His His His His Gly Ser Ala Cys Met Lys
1               5                   10                  15
Lys Ile Ser Leu Pro Lys Ile Gly Ile Arg Pro Val Ile Asp Gly Arg
            20                  25                  30
Arg Met Gly Val Arg Glu Ser Leu Glu Glu Gln Thr Met Asn Met Ala
        35                  40                  45
Lys Ala Thr Ala Ala Leu Leu Thr Glu Lys Leu Arg His Ala Cys Gly
    50                  55                  60

Ala Ala Val Glu Cys Val Ile Ser Asp Thr Cys Ile Ala Gly Met Ala
65                  70                  75                  80
Glu Ala Ala Ala Cys Glu Glu Lys Phe Ser Ser Gln Asn Val Gly Leu
                85                  90                  95
Thr Ile Thr Val Thr Pro Cys Trp Cys Tyr Gly Ser Glu Thr Ile Asp
            100                 105                 110
Met Asp Pro Thr Arg Pro Lys Ala Ile Trp Gly Phe Asn Gly Thr Glu
        115                 120                 125
Arg Pro Gly Ala Val Tyr Leu Ala Ala Ala Leu Ala Ala His Ser Gln
    130                 135                 140
Lys Gly Ile Pro Ala Phe Ser Ile Tyr Gly His Asp Val Gln Asp Ala
145                 150                 155                 160
Asp Asp Thr Ser Ile Pro Ala Asp Val Glu Glu Lys Leu Leu Arg Phe
                165                 170                 175
Ala Arg Ala Gly Leu Ala Val Ala Ser Met Lys Gly Lys Ser Tyr Leu
            180                 185                 190
Ser Leu Gly Gly Val Ser Met Gly Ile Ala Gly Ser Ile Val Asp His
        195                 200                 205
Asn Phe Phe Glu Ser Trp Leu Gly Met Lys Val Gln Ala Val Asp Met
    210                 215                 220
Thr Glu Leu Arg Arg Arg Ile Asp Gln Lys Ile Tyr Asp Glu Ala Glu
225                 230                 235                 240
Leu Glu Met Ala Leu Ala Trp Ala Asp Lys Asn Phe Arg Tyr Gly Glu
                245                 250                 255
Asp Glu Asn Asn Lys Gln Tyr Gln Arg Asn Ala Glu Gln Ser Arg Ala
            260                 265                 270
Val Leu Arg Glu Ser Leu Leu Met Ala Met Cys Ile Arg Asp Met Met
        275                 280                 285
Gln Gly Asn Ser Lys Leu Ala Asp Ile Gly Arg Val Glu Glu Ser Leu
    290                 295                 300
Gly Tyr Asn Ala Ile Ala Ala Gly Phe Gln Gly Gln Arg His Trp Thr
305                 310                 315                 320
Asp Gln Tyr Pro Asn Gly Asp Thr Ala Glu Ala Ile Leu Asn Ser Ser
                325                 330                 335
Phe Asp Trp Asn Gly Val Arg Glu Pro Phe Val Val Ala Thr Glu Asn
            340                 345                 350

Asp Ser Leu Asn Gly Val Ala Met Leu Met Gly His Gln Leu Thr Gly
        355                 360                 365
Thr Ala Gln Val Phe Ala Asp Val Arg Thr Tyr Trp Ser Pro Glu Ala
    370                 375                 380
Ile Glu Arg Val Thr Gly His Lys Leu Asp Gly Leu Ala Glu His Gly
385                 390                 395                 400
Ile Ile His Leu Ile Asn Ser Gly Ser Ala Ala Leu Asp Gly Ser Cys
                405                 410                 415
Lys Gln Arg Asp Ser Glu Gly Asn Pro Thr Met Lys Pro His Trp Glu
            420                 425                 430
Ile Ser Gln Gln Glu Ala Asp Ala Cys Leu Ala Ala Thr Glu Trp Cys
        435                 440                 445
Pro Ala Ile His Glu Tyr Phe Arg Gly Gly Gly Tyr Ser Ser Arg Phe
    450                 455                 460
Leu Thr Glu Gly Gly Val Pro Phe Thr Met Thr Arg Val Asn Ile Ile
465                 470                 475                 480
Lys Gly Leu Gly Pro Val Leu Gln Ile Ala Glu Gly Trp Ser Val Glu
                485                 490                 495
Leu Pro Lys Asp Val His Asp Ile Leu Asn Lys Arg Thr Asn Ser Thr
            500                 505                 510
Trp Pro Thr Thr Trp Phe Ala Pro Arg Leu Thr Gly Lys Gly Pro Phe
        515                 520                 525
Thr Asp Val Tyr Ser Val Met Ala Asn Trp Gly Ala Asn His Gly Val
    530                 535                 540
Leu Thr Ile Gly His Val Gly Ala Asp Phe Ile Thr Leu Ala Ser Met
545                 550                 555                 560
Leu Arg Ile Pro Val Cys Met His Asn Val Glu Glu Thr Lys Val Tyr
                565                 570                 575
Arg Pro Ser Ala Trp Ala Ala His Gly Met Asp Ile Glu Gly Gln Asp
            580                 585                 590
Tyr Arg Ala Cys Gln Asn Tyr Gly Pro Leu Tyr Lys Arg
        595                 600                 605

<210>  5
<211>  30

<212>  DNA
<213>  Artificial

<220>
<223>  primer for obtaining fucI gene

<400>  5
aactgaattc attttccgaa taaagtgagg                                        30

<210>  6
<211>  30
<212>  DNA
<213>  Artificial

<220>
<223>  primer for obtaining fucI gene

<400>  6
gttcaggccc tgcagccgga gcgaccgggc                                        30

<210>  7
<211>  35
<212>  DNA
<213>  artificial

<220>
<223>  5' primer to obtain a internal sequence of sldA

<400>  7
gccggcggta ccttctagca acagccgcag gactc                                  35

<210>  8
<211>  35

<212>  DNA
<213>  artificial

<220>
<223>  3' primer to obtain a internal sequence of sldA

<400>  8
acaccttgtc tgcagcatca ctacgcaggg cgctg                                35

<210>  9
<211>  35
<212>  DNA
<213>  artificial

<220>
<223>  5' primer to obtain kmr gene

<400>  9
taaaactgga tccttacata aacagtaata caagg                                35

<210>  10
<211>  35
<212>  DNA
<213>  artificial

<220>
<223>  3' primer to obtain Kmr gene

<400>  10
atgctctgcc agatctacaa ccaattaacc aattc                                35

<210>  11
<211>  12

<212>  PRT
<213>  Gluconobacter oxydans

<400>  11

Ser Leu Ala Ala Glu Trp Ala Pro Tyr Gly Ile Arg
1               5                   10

<210>  12
<211>  15
<212>  PRT
<213>  Gluconobacter oxydans

<400>  12

Phe Gly Met Glu Lys Pro Glu Leu Tyr Asp Ala Trp Ala Ile Gly
1               5                   10                  15

<210>  13
<211>  15
<212>  PRT
<213>  Gluconobacter oxydans

<400>  13

Phe Gly Leu Ser Ile Leu Asn Asn Ser His Lys Asp Val Ala Met
1               5                   10                  15

<210>  14
<211>  15
<212>  PRT
<213>  Gluconobacter oxydans

<400> 14

Pro Thr Ser Ile Thr Val Arg Asp Glu Ala Pro Leu Leu Tyr Phe
1               5                   10                  15

<210> 15
<211> 774
<212> DNA
<213> Gluconobacter oxydans

<220>
<221> CDS
<222> (1)..(774)
<223> gene for fudh

<400> 15
atg tac atg gaa aaa ctt cgt ctc gat ggc cgc acc gca gtt gtc act      48
Met Tyr Met Glu Lys Leu Arg Leu Asp Gly Arg Thr Ala Val Val Thr
1               5                   10                  15
ggc ggc gca cag aac atc ggt ctg gcc tgc gtg acg gca ctg gcc gaa      96
Gly Gly Ala Gln Asn Ile Gly Leu Ala Cys Val Thr Ala Leu Ala Glu
            20                  25                  30
gcc ggg gcg cgt gtt gtg att gcg gat ctg gat gag gcc atg gcc gca      144
Ala Gly Ala Arg Val Val Ile Ala Asp Leu Asp Glu Ala Met Ala Ala
        35                  40                  45
caa tct gcg gag gaa ctc tgc gca gag ggc ctg gac gtc aga agc atc      192
Gln Ser Ala Glu Glu Leu Cys Ala Glu Gly Leu Asp Val Arg Ser Ile
    50                  55                  60
cgc atg gat gtc acg agc atg gaa aat gtt cag gca gcc atc aag acc      240
Arg Met Asp Val Thr Ser Met Glu Asn Val Gln Ala Ala Ile Lys Thr
65                  70                  75                  80
ctg cac gag cag gaa ggc cat ctg gat att ctg gtg gcc tgt gcg ggg      288
Leu His Glu Gln Glu Gly His Leu Asp Ile Leu Val Ala Cys Ala Gly
                85                  90                  95

atc tgc att tcc gaa gtc aaa gct gag gac atg acg gaa ggt cag tgg 336
Ile Cys Ile Ser Glu Val Lys Ala Glu Asp Met Thr Glu Gly Gln Trp
            100                 105                 110
ctc aag cag gtc gat atc aac ctg aac ggc atg ttc cgt tgc tgt cag 384
Leu Lys Gln Val Asp Ile Asn Leu Asn Gly Met Phe Arg Cys Cys Gln
        115                 120                 125
gcc gtg ggt cgc atc atg ctt gag cag aag aaa ggc gcg att gtc gcc 432
Ala Val Gly Arg Ile Met Leu Glu Gln Lys Lys Gly Ala Ile Val Ala
    130                 135                 140
atc gga tcc atg tcc ggg caa atc gtc aac cgc cca cag cag cag gcc 480
Ile Gly Ser Met Ser Gly Gln Ile Val Asn Arg Pro Gln Gln Gln Ala
145                 150                 155                 160
gcc tat aat gcc tcc aag gcg ggt gtg cac cag tat atc cgc tca ctt 528
Ala Tyr Asn Ala Ser Lys Ala Gly Val His Gln Tyr Ile Arg Ser Leu
                165                 170                 175
gcg gcg gaa tgg gcg cct tat ggt atc cgt gcc aat gca gtt gct ccg 576
Ala Ala Glu Trp Ala Pro Tyr Gly Ile Arg Ala Asn Ala Val Ala Pro
            180                 185                 190
acc tac atc gaa aca aca ctg aca cgc ttc ggt atg gaa aag ccg gaa 624
Thr Tyr Ile Glu Thr Thr Leu Thr Arg Phe Gly Met Glu Lys Pro Glu
        195                 200                 205
ctg tat gat gcg tgg att gcc gga aca ccg atg ggg cgc gtg ggg cag 672
Leu Tyr Asp Ala Trp Ile Ala Gly Thr Pro Met Gly Arg Val Gly Gln
    210                 215                 220
ccc gac gaa gtc gcc tcc gtc gtg cac ttt ctg gcc tcg gat gcc gca 720
Pro Asp Glu Val Ala Ser Val Val His Phe Leu Ala Ser Asp Ala Ala
225                 230                 235                 240
agc ctg atg acg ggt tcc atc gtc aac gtg gat gct ggt ttc acc gtc 768
Ser Leu Met Thr Gly Ser Ile Val Asn Val Asp Ala Gly Phe Thr Val
                245                 250                 255
tgg taa 774
Trp

<210> 16
<211> 257

<212>  PRT
<213>  Gluconobacter oxydans

<400>  16

Met Tyr Met Glu Lys Leu Arg Leu Asp Gly Arg Thr Ala Val Val Thr
1               5                   10                  15
Gly Gly Ala Gln Asn Ile Gly Leu Ala Cys Val Thr Ala Leu Ala Glu
            20                  25                  30
Ala Gly Ala Arg Val Val Ile Ala Asp Leu Asp Glu Ala Met Ala Ala
        35                  40                  45
Gln Ser Ala Glu Glu Leu Cys Ala Glu Gly Leu Asp Val Arg Ser Ile
    50                  55                  60
Arg Met Asp Val Thr Ser Met Glu Asn Val Gln Ala Ala Ile Lys Thr
65                  70                  75                  80
Leu His Glu Gln Glu Gly His Leu Asp Ile Leu Val Ala Cys Ala Gly
                85                  90                  95

Ile Cys Ile Ser Glu Val Lys Ala Glu Asp Met Thr Glu Gly Gln Trp
            100                 105                 110
Leu Lys Gln Val Asp Ile Asn Leu Asn Gly Met Phe Arg Cys Cys Gln
        115                 120                 125
Ala Val Gly Arg Ile Met Leu Glu Gln Lys Lys Gly Ala Ile Val Ala
    130                 135                 140
Ile Gly Ser Met Ser Gly Gln Ile Val Asn Arg Pro Gln Gln Gln Ala
145                 150                 155                 160
Ala Tyr Asn Ala Ser Lys Ala Gly Val His Gln Tyr Ile Arg Ser Leu
                165                 170                 175
Ala Ala Glu Trp Ala Pro Tyr Gly Ile Arg Ala Asn Ala Val Ala Pro
            180                 185                 190
Thr Tyr Ile Glu Thr Thr Leu Thr Arg Phe Gly Met Glu Lys Pro Glu
        195                 200                 205
Leu Tyr Asp Ala Trp Ile Ala Gly Thr Pro Met Gly Arg Val Gly Gln
    210                 215                 220
Pro Asp Glu Val Ala Ser Val Val His Phe Leu Ala Ser Asp Ala Ala

225                 230                 235                 240
Ser Leu Met Thr Gly Ser Ile Val Asn Val Asp Ala Gly Phe Thr Val
                245                 250                 255
Trp

<210>  17
<211>  474
<212>  DNA
<213>  Gluconobacter oxydans

<220>
<221>  CDS
<222>  (1)..(474)
<223>  gene for nox

<400>  17
atg acg ata ctg aat gag cag gac ttt cgc cgg gcc atg tcc cat ttc        48
Met Thr Ile Leu Asn Glu Gln Asp Phe Arg Arg Ala Met Ser His Phe
1               5                   10                  15
gcg acg ggt gtg gct gtt gtg aca gcc aag gga agc gaa ggc gag caa        96
Ala Thr Gly Val Ala Val Val Thr Ala Lys Gly Ser Glu Gly Glu Gln
            20                  25                  30
ggg gcg acc atc agc gcc ctg aca tct gtt tcc cta gac ccg ctg ctg       144
Gly Ala Thr Ile Ser Ala Leu Thr Ser Val Ser Leu Asp Pro Leu Leu
        35                  40                  45
ctg ctg atc tgc ctg cat cgt ggc agc tcg act tac aag gcg att gct       192
Leu Leu Ile Cys Leu His Arg Gly Ser Ser Thr Tyr Lys Ala Ile Ala
    50                  55                  60
gag gca ggc cgt ttc ggt ctc agc att ctg aac aac agc cat aag gac       240
Glu Ala Gly Arg Phe Gly Leu Ser Ile Leu Asn Asn Ser His Lys Asp
65                  70                  75                  80
gtg gcc atg ctg ttc gcc agc cgc acg gca gac aaa ttt gga tca gac       288
Val Ala Met Leu Phe Ala Ser Arg Thr Ala Asp Lys Phe Gly Ser Asp
                85                  90                  95
gtt gtg gta cgg gca gaa gat ggt acg gct ttc att gat ggc gca ctg       336

Val Val Val Arg Ala Glu Asp Gly Thr Ala Phe Ile Asp Gly Ala Leu
            100                 105                 110
gta cag atg cat tgt gag ctt gtg gag acg ttc cag ggt ggc aca cat 384
Val Gln Met His Cys Glu Leu Val Glu Thr Phe Gln Gly Gly Thr His
        115                 120                 125
gcg ctc ttc atg gcg cgt cca acc agc atc acc gtc cgg gat gaa gca 432
Ala Leu Phe Met Ala Arg Pro Thr Ser Ile Thr Val Arg Asp Glu Ala
    130                 135                 140
ccg ctt ctg tac ttc cag gga cag ctg ggg gtt gag gcg taa 474
Pro Leu Leu Tyr Phe Gln Gly Gln Leu Gly Val Glu Ala
145                 150                 155

<210> 18
<211> 157
<212> PRT
<213> Gluconobacter oxydans

<400> 18

Met Thr Ile Leu Asn Glu Gln Asp Phe Arg Arg Ala Met Ser His Phe
1               5                   10                  15
Ala Thr Gly Val Ala Val Val Thr Ala Lys Gly Ser Glu Gly Glu Gln
            20                  25                  30
Gly Ala Thr Ile Ser Ala Leu Thr Ser Val Ser Leu Asp Pro Leu Leu
        35                  40                  45
Leu Leu Ile Cys Leu His Arg Gly Ser Ser Thr Tyr Lys Ala Ile Ala
    50                  55                  60
Glu Ala Gly Arg Phe Gly Leu Ser Ile Leu Asn Asn Ser His Lys Asp
65                  70                  75                  80
Val Ala Met Leu Phe Ala Ser Arg Thr Ala Asp Lys Phe Gly Ser Asp
                85                  90                  95
Val Val Val Arg Ala Glu Asp Gly Thr Ala Phe Ile Asp Gly Ala Leu
            100                 105                 110
Val Gln Met His Cys Glu Leu Val Glu Thr Phe Gln Gly Gly Thr His
        115                 120                 125

Ala Leu Phe Met Ala Arg Pro Thr Ser Ile Thr Val Arg Asp Glu Ala
    130                 135                 140
Pro Leu Leu Tyr Phe Gln Gly Gln Leu Gly Val Glu Ala
145                 150                 155

<210> 19
<211> 30
<212> DNA
<213> Artificial

<220>
<223> 5'primer to obtain fcdh

<400> 19
gcctgaattc aggaatattc aagatgtaca 30

<210> 20
<211> 35
<212> DNA
<213> Artificial

<220>
<223> 3'primer to obtain fcdh

<400> 20
gatgagccgc tgcagttaat atgtccggac ctgac 35

<210> 21
<211> 35
<212> DNA
<213> Artificial

<220>

<223>  5'primer to obtain nox

<400>  21
ctgtaaggct gcaggaacca aggattccga cgatg                                35

<210>  22
<211>  35
<212>  DNA
<213>  Artificial

<220>
<223>  3'primer to obtain nox

<400>  22
attccgaaaa agcttcgtaa tttccatcac ttttc                                35

<210>  23
<211>  35
<212>  DNA
<213>  Artificial

<220>
<223>  3'primer to obtain fcdh + nox

<400>  23
gcaccgcatg gtacccagac ggaaacgcct gctta                                35

<210>  24
<211>  35
<212>  DNA
<213>  Artificial

<220>

<223>  5'primer to obtain fucI

<400>  24
cggcaactgg taccacatat tttccgaata aagtg                                35

<210>  25
<211>  30
<212>  DNA
<213>  Artificial

<220>
<223>  3'primer to obtain fucI

<400>  25
gttcaggccg tcgacccgga gcgaccgggc                                      30

<210>  26
<211>  34
<212>  DNA
<213>  Artificial

<220>
<223>  5' primer to obtain fucI

<400>  26
aactgaattc acatattttc cgaataaagt gagg                                 34

<210>  27
<211>  30
<212>  DNA
<213>  Artificial

<220>

<223>  3'primer to obtain fucI

<400>  27
gttcaggccc tgcagcccga gcgaccgggc                                        30


<210>  28
<211>  35
<212>  DNA
<213>  Artificial

<220>
<223>  5'Primer to obtain KatE

<400>  28
ggcttcacta gtcgactatt aaaaatcaga aaaac                                  35


<210>  29
<211>  35
<212>  DNA
<213>  Artificial

<220>
<223>  3'Primer to obtain KatE

<400>  29
atgtaaatcc tgcaggcggc gcaattgcgc gctcc                                  35

## Description

### TECHNICAL FIELD

The present invention relates to a method for producing L-fuculose and a method for producing L-fucose, and more particularly relates to a method for producing L-fuculose and a method for producing L-fucose, which are easy and efficient.

### BACKGROUND ART

L-Fucose (L-6-deoxygalactose) is ubiquitously present in living world, mostly as a non-reducing terminal sugar of various carbohydrates. As a polysaccharide having L-fucose as a component, fucoidan in seaweeds has been known. L-Fucose is believed to be involved in uptake of serum glycoprotein into liver, and constitution of receptors for macrophage migration inhibitory factor as its roles *in vivo.* L-Fucose has been also studied on its association with diseases. For example, the research for utilizing L-fucose as a pharmaceutical intermediate has been advanced. Specifically, it is anticipated to establish diagnosis of cancer on the basis of ratio of L-fucose in glycoprotein or glycolipid or change of urinary free L-fucose amount as an indicator in patients with cancer. It is also anticipated to develop a cancer metastasis inhibitory agent and an antivirus agent. It is further anticipated to utilize L-fucose for controlling leukocytes and treating rheumatoid arthritis.

As described above, it is anticipated to develop various utilizations of L-fucose in future. For obtaining and producing L-fucose, some methods have been already developed. For example, a method for extracting fucoidan contained in *Nemacystus decipiens* (Patent Document 1), and a method for isolating L-fucose from fucoidan derived from *Nemacystus decipiens* (Patent Document 2) have been known. However, a large amount of *Nemacystus decipiens* is required for performing these methods. In addition, it is difficult to practically isolate and purify the product with these methods, and the yield given by these methods is small.

As another attempt, there is a method in which polysaccharide produced by a microorganism is hydrolyzed and L-fucose is isolated therefrom (Patent Document 3). However, practical isolation and purification with this method are also technically difficult and the yield is also very small. There is also known a chemical synthesis method using D-galactose as a raw material (Patent Document 1). However, this method is not industrially practical because of many steps and the small yield.

As a synthetic method using an enzyme, there is known a method in which L-fuculose-1-phosphate is converted into L-fucose using acid phosphatase and further converted into L-fucose using L-fucose isomerase (Patent Document 4). However, this method uses expensive L-fuculose-1-phosphate as a starting material. There is thus a demand for a method which enables production at lower cost.

Oxidation of L-fucitol using NAD-dependent dehydrogenase derived from a plant (Rhodophyta, red algae) has been reported (e.g., Patent Document 5, Non-patent Document 5). However, it is unclear which site of L-fucitol is oxidized, and resulting products are not definitely identified. In addition, it is generally difficult to produce an enzyme derived from a plant on a large scale for industrial application, and this method is thus accompanied by inconvenience in handling the enzyme derived from the plant.

Furthermore, as the oxidation of L-fucitol using a microorganism, experiments using acetobacterium have been reported (e.g., Non-patent Documents 2 and 3). However, no enzyme has been identified in these reports. The resulting products by the oxidation of L-fucitol are different depending on the oxidized site of L-fucitol. In these experimental reports, the major component of the resulting oxide has been reported to be not L-fucose or L-fuculose but a substance as a result of oxidation at position 4 of L-fucitol (L-fuco-4-ketose in Non-patent Document 2). The acetobacterium has been extensively studied in relation to the enzyme acting on a sugar as a substrate (e.g., Patent Document 6, Non-patent Documents 3 and 4).

Patent Document 1: JP S61-57520 A,
Patent Document 2: JP H11-35591 A,
Patent Document 3: JP S59-51798 A,
Patent Document 4: International Publication WO97/15683 Pamphlet,
Patent Document 5: International Publication WO02/06506 Pamphlet,
Patent Document 6: JP H8-242850 A,
Nonpatent Document 1: Carbohydrate research 270; 93-96 (1995),
Nonpatent Document 2: Journal of American Chemical Society; 4934-4937 (1950),
Nonpatent Document 3: Canadian Journal of Chemistry 45: 741-744 (1967),
Nonpatent Document 4: Biosci. Biotechnol. Biochem. 65: 2755-2762 (2001),
Nonpatent Document 5: Planta 202: 487-493 (1997).

### DISCLOSURE OF INVENTION

### PROBLEM TO BE SOLVED BY THE INVENTION

Although various methods for utilizing L-fucose are anticipated as described above, none of the methods for producing L-fucose are industrially available. The object of the present invention is to provide a method for producing L-fucose, which is suitable as the industrial production method in terms of easiness and cost.

### MEANS FOR SOLVING PROBLEM

The present inventors have conducted studies as shown in Examples described in detail below, and then have found out the presence of a protein having a fucitol dehydrogenase activity derived from a microorganism. The present inventors have also found various techniques to synthesize L-fuculose from L-fucitol under a condition where there is no protein having an activity to synthesize ketohexose other than L-fuculose from L-fucitol or the activity thereof is inhibited. The present inventors have then completed an industrially suitable method for producing L-fuculose which is an intermediate in the production of L-fucose, and have completed a method for producing L-fucose, which is industrially advantageous. That is, the present invention provides the following methods for producing 1-fuculose and methods for producing L-fucose.

(1) A method for producing L-fuculose comprising contacting L-fucitol with a protein derived from a microorganism, the protein having a dehydrogenase activity to synthesize L-fuculose from L-fucitol.
(2) The method for producing L-fuculose according to (1) wherein said microorganism is an acetobacterium.
(3) A method for producing L-fuculose comprising contacting L-fucitol with one or more selected from the group consisting of a microorganism having an ability to synthesize L-fuculose from L-fucitol to an extent so that the amount of L-fuculose thus produced accounts for 50% by weight or more of total L-fucitol oxide, a culture of the microorganism and a treated microbial cell product of the microorganism.
(4) A method for producing L-fuculose comprising contacting L-fucitol with one or more selected from the group consisting of: at least one microorganism belonging to *Gluconobacter xylinus subsp. xylinus* and *Gluconobacter oxydans,* a culture of the microorganism, and a treated microbial cell product of the microorganism.
(5) A method for producing L-fuculose comprising contacting L-fucitol with one or more selected from the group consisting of: a non-genetically engineered microorganism having a protein having a dehydrogenase activity to synthesize L-fuculose from L-fucitol, the microorganism not substantially having an ability to synthesize ketohexose other than L-fuculose from L-fucitol, a culture of the non-genetically engineered microorganism, and a treated microbial cell product of the non-genetically engineered microorganism.
(6) A method for producing L-fuculose comprising contacting L-fucitol with one or more selected from the group consisting of: a microorganism having a first protein derived from a microorganism having a dehydrogenase activity to synthesize L-fuculose from L-fucitol, wherein a gene encoding a second protein having an activity to synthesize ketohexose other than L-fuculose from L-fucitol has been knocked out, a culture of the microorganism, and a treated microbial cell product of the microorganism.
(7) A method for producing L-fuculose comprising contacting L-fucitol with one or more selected from the group consisting of: a microorganism substantially lacking an ability to synthesize ketohexose other than L-fuculose from fucitol, the microorganism being transformed to be capable of expressing a first microorganism-derived protein having a dehydrogenase activity to synthesize L-fuculose from L-fucitol, a culture of the microorganism, and a treated microbial cell product of the microorganism.
(8) The method for producing L-fuculose according to (7) wherein said microorganism is a microorganism wherein a gene encoding a second protein having an activity to synthesize ketohexose other than L-fuculose from L-fucitol has been knocked out.
(9) The method for producing L-fuculose according to any one of (1) to (4) wherein L-fuculose is synthesized from L-fucitol under a condition where synthesis of ketohexose other than L-fuculose from L-fucitol is inhibited.
(10) The method for producing L-fuculose according to any one of (1) to (4) wherein L-fuculose is synthesized from L-fucitol under a pH condition where synthesis of ketohexose other than L-fuculose from L-fucitol is inhibited.
(11) The method for producing L-fuculose according to any one of (1) to (4) wherein L-fuculose is synthesized from L-fucitol in the coexistence of a bivalent ion chelator under the condition where synthesis of ketohexose other than L-fuculose from L-fucitol is inhibited.
(12) The method for producing L-fuculose according to any one of (1) to (8) wherein L-fuculose is synthesized from L-fucitol in the presence of a protein having an activity to produce NAD from NADH.
(13) A method for producing L-fucose comprising: a step of synthesizing L-fuculose wherein L-fucitol is contacted with a protein derived from a microorganism having a dehydrogenase activity to synthesize L-fuculose from L-fucitol, and a step synthesizing L-fucose wherein L-fuculose is contacted with a protein having an activity to synthesize L-fucose from L-fuculose.
(14) A method for producing L-fucose comprising: a step of synthesizing L-fuculose wherein L-fucitol is contacted with one or more selected from the group consisting of a microorganism having an ability to synthesize L-fuculose from L-fucitol to an extent so that the amount of L-fuculose thus produced accounts for 50% by weight or more of total L-fucitol oxide, a culture of the microorganism and a treated microbial cell product of the microorganism, and a step synthesizing L-fucose wherein L-fuculose is contacted with a protein having an activity to synthesize L-fucose from L-fuculose.
(15) A method for producing L-fucose comprising: a step of synthesizing L-fuculose wherein L-fucitol is contacted with one or more selected from the group consisting of a non-genetically engineered microorganism having a protein having a dehydrogenase activity to synthesize L-fuculose from L-fucitol, the microorganism not substantially having an ability to synthesize ketohexose other than L-fuculose from L-fucitol; a culture of the non-genetically engineered microorganism; and a treated microbial cell product of the non-genetically engineered microorganism, and a step synthesizing L-fucose wherein L-fuculose is contacted with a protein having an activity to synthesize L-fucose from L-fuculose.
(16) A method for producing L-fucose comprising: a step of synthesizing L-fuculose wherein L-fucitol is contacted with one or more selected from the group consisting of a microorganism having a protein having a dehydrogenase activity to synthesize L-fuculose from L-fucitol, wherein a gene encoding a protein having an activity to synthesize ketohexose other than L-fuculose from L-fucitol has been knocked out; a culture of the microorganism; and a treated microbial cell product of the microorganism, and a step synthesizing L-fucose wherein L-fuculose is contacted with a protein having an activity to synthesize L-fucose from L-fuculose.
(17) A method for producing L-fucose comprising: a step of synthesizing L-fuculose wherein L-fucitol is contacted with one or more selected from the group consisting of a microorganism substantially lacking an ability to synthesize ketohexose other than L-fuculose from L-fucitol, the microorganism being transformed to be capable of expressing a first microorganism-derived protein having a dehydrogenase activity to synthesize L-fuculose from L-fucitol; a culture of the microorganism; and a treated microbial cell product of the microorganism, and a step synthesizing L-fucose wherein L-fuculose is contacted with a protein having an activity to synthesize L-fucose from L-fuculose.
(18) A method for producing L-fucose comprising contacting L-fucitol with one or more selected from the group consisting of: a microorganism substantially lacking an ability to synthesize ketohexose other than L-fuculose from L-fucitol, the microorganism being transformed to be capable of expressing a first microorganism-derived protein having a dehydrogenase activity to synthesize L-fuculose from L-fucitol and another protein having an activity to synthesize L-fucose from L-fuculose, a culture of the microorganism, and a treated microbial cell product of the microorganism.
(19) A protein of the following (A) or (B): (A) a protein having an amino acid sequence according to SEQ ID NO:16, or (B) a protein having an amino acid sequence including one or several amino acid mutations selected from the group consisting of substitution, deletion, insertion, addition and inversion in the amino acid sequence according to SEQ ID NO:16, and having a dehydrogenase activity to synthesize L-fuculose from L-fucitol.
(20) A polynucleotide encoding the protein according to (19).
(21) A polynucleotide of the following (a) or (b): (a) a polynucleotide having a nucleotide sequence according to SEQ ID NO:15, or (b) a polynucleotide which hybridizes with a polynucleotide having a nucleotide sequence complementary to the nucleotide sequence according to SEQ ID NO:15 under a stringent condition and encodes a protein having a dehydrogenase activity to synthesize L-fuculose from L-fucitol.
(22) A protein of the following (C) or (D): (C) a protein having an amino acid sequence according to SEQ ID NO:18, or (D) a protein having an amino acid sequence including one or several amino acid mutations selected from the group consisting of substitution, deletion, insertion, addition and inversion in the amino acid sequence according to SEQ ID NO:18, and having an NADH oxidase activity.
(23) A polynucleotide encoding the protein according to (22) .
(24) A polynucleotide of the following (c) or (d): (c) a polynucleotide having a nucleotide sequence according to SEQ ID NO:17, or (d) a polynucleotide which hybridizes with a polynucleotide having a nucleotide sequence complementary to the nucleotide sequence according to SEQ ID NO:17 under a stringent condition and encodes a protein having an activity to produce NAD from NADH.
(25) A recombinant polynucleotide comprising the polynucleotide according to (20) incorporated therein.
(26) A recombinant polynucleotide comprising the polynucleotide according to (23) incorporated therein.
(27) A recombinant polynucleotide comprising the polynucleotide according to (20) and the polynucleotide according to (23) incorporated therein.
(28) A transformant comprising the recombinant polynucleotide according to (25), the transformant being capable of expressing a protein having a dehydrogenase activity to synthesize L-fuculose from L-fucitol.
(29) A method for producing a protein comprising culturing the transformant according to (28), wherein the transformant is a microorganism, in a medium to accumulate the protein having a dehydrogenase activity to synthesize L-fuculose from L-fucitol in the medium and/or in the microorganism.
(30) A method for producing L-fuculose comprising adding one or more selected from the group consisting the transformant according to (28) wherein the transformant is a microorganism, a culture of the microorganism and a treated microbial cell product of the microorganism, to a reaction system containing L-fucitol to synthesize L-fuculose from L-fucitol.
(31) A method for producing L-fucose comprising: a step of synthesizing L-fuculose wherein one or more selected from the group consisting of the transformant according to (28) wherein the transformant is a microorganism, a culture of the microorganism and a treated microbial cell product of the microorganism are added to a reaction system containing L-fucitol to synthesize L-fuculose from L-fucitol, and a step synthesizing L-fucose wherein L-fuculose is contacted with a protein having an activity to synthesize L-fucose from L-fuculose.
(32) A transformant comprising the recombinant polynucleotide according to (25) and the recombinant polynucleotide according to (26) incorporated therein, the microorganism being capable of expressing a protein having a dehydrogenase activity to synthesize L-fuculose from L-fucitol and another protein having an activity to produce NAD from NADH.
(33) A method for producing a protein comprising culturing a transformed microorganism in which the recombinant polynucleotide according to (26) has been introduced, the microorganism being capable of expressing an protein having an activity to produce NAD from NADH, in a medium to accumulate the protein having the activity to produce NAD from NADH in the medium and/or in the microorganism.
(34) A method for producing L-fuculose comprising adding one or more selected from the group consisting of the transformant according to (32) wherein the transformant is a microorganism, a culture of the microorganism and a treated microbial cell product of the microorganism, to a reaction system containing L-fucitol to synthesize L-fuculose from L-fucitol.
(35) A method for producing L-fucose comprising: a step of synthesizing L-fuculose wherein one or more selected from the group consisting of the transformant according to (32) wherein the transformant is a microorganism, a culture of the microorganism and a treated microbial cell product of the microorganism are added to a reaction system containing L-fucitol to synthesize L-fuculose from L-fucitol, and a step synthesizing L-fucose wherein L-fuculose is contacted with a protein having an activity to synthesize L-fucose from L-fuculose.
(36) A transformant comprising the recombinant polynucleotide according to (27) introduced therein, the transformant being capable of expressing a protein having a dehydrogenase activity to synthesize L-fuculose from L-fucitol and another protein having an activity to produce NAD from NADH.
(37) A method for producing a protein comprising culturing the transformant according to (36), wherein the transformant is a microorganism, in a medium to accumulate the protein having a dehydrogenase activity to synthesize L-fuculose from L-fucitol and the protein having an activity to produce NAD from NADH in the medium and/or in the microorganism.
(38) A method for producing L-fuculose comprising adding one or more selected from the group consisting of the transformant according to (36) wherein the transformant is a microorganism, a culture of the microorganism and a treated microbial cell product of the microorganism, to a reaction system containing L-fucitol to synthesize L-fuculose from L-fucitol.
(39) A method for producing L-fucose comprising: a step of synthesizing L-fuculose wherein one or more selected from the group consisting of the transformant according to (36) wherein the transformant is a microorganism, a culture of the microorganism and a treated microbial cell product of the microorganism are added to a reaction system containing L-fucitol to synthesize L-fuculose from L-fucitol, and a step synthesizing L-fucose wherein L-fuculose is contacted with a protein having an activity to synthesize L-fucose from L-fuculose.

Sequences represented in SEQ ID NOS herein indicate the sequences described in Sequence Listing unless otherwise specified.

### EFFECT OF THE INVENTION

According to the present invention, the method for producing L-fuculose and the method for producing L-fucose, which are industrially suitable are provided. The production method of the present invention is easy and simple as the industrial production. The production method of the present invention can reduce or eliminate the production of a byproduct, and is efficient. Since the production method of the present invention can utilize D-galactose which is an inexpensive material as the starting material, the production cost thereof can be reduced. The present invention is therefore extremely advantageous in terms of the industrial production.

### BRIEF DESCRIPTION OF DRAWINGS

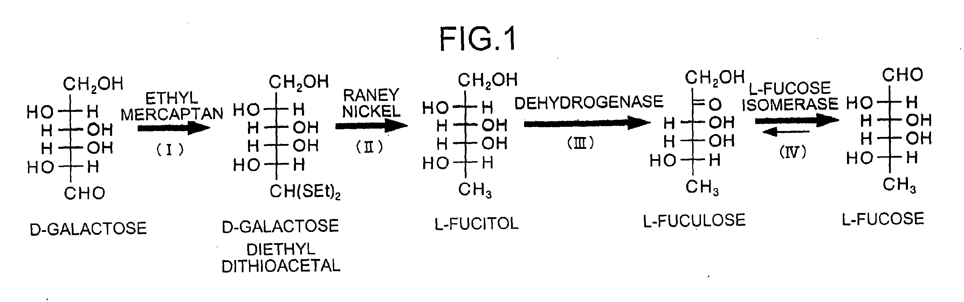

FIG. 1 is a view showing a reaction process from D-galactose to L-fucose.
FIG. 2 is a view showing amounts of L-fucitol converted by FucI-expressing acetobacterium.
FIG. 3 is a view showing the amounts of produced byproducts (BP) in the production of L-fuculose from L-fucitol under different pH conditions.
FIG. 4 is a view showing the amounts of produced byproducts (BP) when L-fuculose was produced from L-fucitol in the presence of EDTA.
FIG. 5 is a view showing the amounts of produced byproducts (BP) when L-fuculose was produced from L-fucitol with knocked out sldA gene.
FIG. 6 is a view showing an SDS-PAGE profile of purified FcDH.
FIG. 7 is a view showing an SDS-PAGE profile of purified NOX.
FIG. 8 is a view showing a result of measuring optimal pH of purified FcDH for the reaction.
FIG. 9 is a view showing a result of measuring pH stability of purified FcDH.
FIG. 10 is a view showing a result of measuring optimal temperature of purified FcDH for the reaction.
FIG. 11 is a view showing a result of measuring temperature stability of purified FcDH.
FIG. 12 is a view showing a result of measuring specific activity of purified FcDH at various concentrations of D-arabitol (A) and L-fucitol (B).
FIG. 13 is a view showing a result of examining FcDH-oxidized products of D-arabitol, D-mannitol and D-sorbitol by HPLC analysis.
FIG. 14 is a view showing a result of measuring specific activity of purified FcDH at various NAD concentrations.
FIG. 15 is a view showing a result of measuring optimal pH of purified NOX for the reaction.
FIG. 16 is a view showing a result of measuring pH stability of purified NOX.
FIG. 17 is a view showing a result of measuring optimal temperature of purified NOX for the reaction.
FIG. 18 is a view showing a result of measuring temperature stability of purified NOX.
FIG. 19 is a view showing a result of measuring specific activity of purified NOX at various concentrations of flavin coenzymes (FAD, riboflavin and FMN).
FIG. 20 is a view showing a result of measuring specific activity of purified NOX at various concentrations of NADH (FIG. 20 (A)). FIG. 20 (B) shows Lineweaver-Burk plot of (A) .
FIG. 21 is a view showing SDS-PAGE profiles of cell free extracts prepared from *E. coli*/pUC18, *E. coli*/pIEX11, *E. coli*/pFEX3052, *E. coli*/pFNEX4105 and *E. coli*/pFNIEX5706.
FIG. 22 is a view showing an SDS-PAGE profile of rFcDH purified from *E. coli*/pFEX3052.
FIG. 23 is a view showing an SDS-PAGE profile of rNOX purified from *E. coli*/pFNEX4105.
FIG. 24 is a view showing time courses of L-fucitol conversion using FcDH and NOX purified from *G. oxydans.*
FIG. 25 is a view showing time courses of L-fucitol conversion using rFcDH and rNOX prepared from recombinant *E. coli .*
FIG. 26 is a view showing time courses of L-fucitol conversion by recombinant *E. coli* intact cells.
FIG. 27 is a view showing time courses of L-fucitol conversion in the presence of catalase.

### BEST MODE FOR CARRYING OUT THE INVENTION

Embodiments of the present invention will be described below including their best mode. As to the below-mentioned various gene engineering techniques, many standard experimental manuals such as Molecular Cloning, 2nd edition, Cold Spring Harbor press (1989), Saibo Kogaku Handbook (Cell Engineering Handbook) edited by Toshio Kuroki et al., Yodosha (1992) and Shin Idenshi Kogaku Handbook (New Genetic Engineering Handbook), revised 3rd edition edited by Muramatsu et al., Yodosha (1999) are available for those skilled in the art. Those skilled in the art can utilize these techniques with reference to these manuals and this specification.

### 1. Method for producing L-fuculose

### 1-1. Method using protein derived from microorganism

In the method for producing L-fuculose of the present invention, L-fuculose is synthesized from L-fucitol using a protein derived from a microorganism. The protein used therein has a dehydrogenase activity to synthesize L-fuculose from L-fucitol. As described in detail in the following Examples, the present inventors have found that a protein having an activity to synthesize L-fuculose from L-fucitol is present independently from another protein having an activity to synthesize a ketohexose other than L-fuculose from 1-fucitol in the microorganism. Therefore, by obtaining the protein having the activity to synthesize L-fuculose from L-fucitol from a given microorganism, it becomes possible to reduce or eliminate the production of byproducts other than L-fuculose. Since the protein is derived from a microorganism, its handlings such as isolation and purification are easier than proteins derived from plants and animals, and the protein is thus advantageous in terms of compatibility with a host when xenogeneic expression of the protein on a large scale in the microorganism is performed for mass production of the enzyme. Cultivation of a microorganism is easier than cultivation of plant or animal cells, and therefore easily provides a sufficient amount for industrial production.
As described separately below, based on the protein isolated from *Gluconobacter oxydans,* the present inventors determined its amino acid sequence and nucleotide sequence.

The method for producing L-fuculose of the present invention may reduce the cost and is thus advantageous for the industrial production because L-fucitol is easily obtainable from L-galactose which is an inexpensive raw material. The method for obtaining L-fucitol from D-galactose is described in, for example, Non-patent Document 1.

The protein to be used may be those derived from the microorganism and having the dehydrogenase activity to synthesize L-fuculose from L-fucitol. The microorganisms herein include prokaryotic microorganisms, eukaryotic microorganisms and viruses, but exclude plant cells including algae. Examples of the microorganism from which such a protein is obtainable may include those belonging to an acetobacterium. Those belonging to acetobacterium may specifically include bacteria belonging to genera *Gluconobacter* and *Acetobacter*, and more specifically bacteria belonging to *Gluconobacter oxydans, Gluconobacter frateurii, Acetobacter turbidans, Gluconobacter roseus*, *Gluconobacter cerinus, Gluconobacter oxydans subsp. suboxydans, Acetobacter melanogenus, Gluconobacter xylinus subsp. xylinus, Acetobacter aurantius, Gluconobacter melanogenus, Gluconobacter scleroideus* and *Gluconobacter suboxydans* are exemplified. More specifically, the aforementioned protein may be obtained from various bacterial strains described in the following Table 1.

In order to obtain a sufficient amount of such a microorganism, the microorganism may be cultured in an appropriate medium depending on its type. The medium therefor is not particularly limited as long as the microorganism can grow in the medium, and may be any of the standard media typically containing carbon sources, nitrogen sources, phosphorous sources, sulfur sources, inorganic ions, and further optionally containing organic nutrient sources.

As the carbon sources, any carbon sources which the aforementioned microorganism can utilize may be used. Specifically, saccharides such as glucose, fructose, maltose and amylose, alcohols such as sorbitol, ethanol and glycerol, organic acids such as fumaric acid, citric acid, acetic acid and propionic acid and salts thereof, hydrocarbons such as paraffin, and mixtures thereof may be used.

As the nitrogen sources, ammonium salts of inorganic acids such as ammonium sulfate and ammonium chloride, ammonium salts of organic acids such as ammonium fumarate and ammonium citrate, nitrate salts such as sodium nitrate and potassium nitrate, organic nitrogen compounds such as peptone, yeast extract, meat extract and corn steep liquor, or mixture thereof may be used.

Additionally, the nutrient sources such as inorganic salts, trace metal salts and vitamins used for the standard media may be used by appropriately mixing them.

Conditions for cultivation are not particularly limited. The cultivation may be performed under an aerobic condition with appropriately controlling pH and temperature ranging from pH 5 to 8 and from 15 to 40°C for about 12 to 70 hours.

For purification of the protein derived from the microorganism, it is possible to employ any of standard methods used for ordinary purification of the protein, e.g., an ammonium sulfate salting out method, a gel filtration method, an ion exchange chromatography method and a hydrophobic chromatography method. Upon purification, a microbial cell extract is subjected to purification as the starting material. If non-disrupted or non-lysed cells remains therein, re-centrifugation of the solubilized solution may be advantageously performed in the purification process for removing the precipitated residues.

To synthesize L-fuculose from L-fucitol using the protein derived from the microorganism and having the dehydrogenase activity to synthesize L-fuculose from L-fucitol, L-fucitol may be contacted with the protein derived from the aforementioned microorganism in a medium. For example, L-fucitol and the protein derived from the aforementioned microorganism may be added to buffer and then reacted. The ranges of pH, temperature, reaction time and amount of the enzyme to be added may be appropriately altered depending on the type of the protein derived from the microorganism. For instance, pH is preferably 5 to 11, and more preferably 7 to 10. The temperature is preferably 20 to 50°C and more preferably 25 to 40°C.

### 1-2. Method using microorganism, culture thereof or treated microbial cell product thereof

Subsequently, the method using a microorganism, a culture of the microorganism or a treated microbial cell product of the microorganism will be described. As another embodiment for synthesizing L-fuculose using the aforementioned protein derived from the microorganism, it is also possible to use the microorganism containing the aforementioned microorganism-derived protein, the culture thereof or the treated microbial cell product thereof. However, when the microorganism itself is used, the microorganism also has sometimes an ability to synthesize ketohexose other than L-fuculose (hereinbelow referred to as a byproduct). Thus, when the microorganism itself is used, it is preferable to use the microorganism having weak ability to produce the byproduct. That is, it is preferable to use the microorganism which suffices at least two criteria that are (i) having the ability to synthesize L-fuculose from L-fucitol, and (ii) having the ability to synthesize L-fuculose to an extent so that the amount of L-fuculose thus produced accounts for preferably 50% or more, more preferably 70% or more and still more preferably 90% or more of total L-fucitol oxide generated by oxidizing L-fucitol.

The microorganism which suffices both the aforementioned criteria (i) and (ii) may include *Gluconobacter xylinus subsp. xylinus* and *Gluconobacter oxydans,* more preferably *Gluconobacter xylinus subsp. xylinus* ATCC 53582 strain and ATCC 23767 strain and *Gluconobacter oxydans* IFO 3189 strain, and still more preferably *Gluconobacter xylinus subsp. xylinus* ATCC 53582 strain. These microorganisms do not substantially synthesize ketohexose other than L-fuculose or synthesize ketohexose in a small amount without below-mentioned genetic engineering.

That is, as another preferable embodiment of the present invention, there is provided a method using non-genetically engineered microorganism having the protein having the dehydrogenase activity to synthesize 1-fuculose from L-fucitol, wherein the microorganism does not substantially have the ability to synthesize ketohexose other than L-fuculose from L-fucitol. The term "not substantially have" refers to that the amount of the produced byproduct is not more than the detection limit in the conditions shown in the following Example 1. When specifically exemplified by a numerical value, "not substantially have" may be the state in which the amount of the byproduct is about 1 mM or less.

A "genetically engineered microorganism" refers to a microorganism in which a gene has been artificially engineered using a gene engineering technique, and does not include naturally occurring mutants. The "non-genetically engineered microorganism" refers to those which are not the genetically engineered microorganisms.

The "culture of the microorganism" is a matter obtained by culturing the microorganism. The culture of the microorganism may specifically contains microbial cells of the microorganism, the medium used for the cultivation of the microorganism and a mixture of substances produced by the microorganism, and further a supernatant thereof.

The "treated microbial cell product" refers to a matter obtained by giving some artificial manipulation to the microbial cell of the microorganism, i.e., the cell of the microorganism. The treated microbial cell product may contain, for example, disrupted microbial cells, lysed cells and lyophilized cells. The treated microbial cell product may also be a crude protein collected by treating the microbial cell, and a purified protein. The purified protein may be a partially purified protein obtained by various purification methods, and an immobilized protein obtained by immobilizing the protein by a covalent bond method, an absorption method or an inclusion method.

As described above, the treated microbial cell product may contain a matter that was a content in the microorganism cells. Therefore, when the treated microbial cell product is used, an unintended protein which synthesizes the byproduct may be present. Thus, it is preferable to use the microorganism having the ability to synthesize L-fuculose to the extent so that the amount of L-fuculose thus produced accounts for 50% by weight or more of total L-fucitol oxide. When the protein having the activity to synthesize L-fuculose from L-fucitol is sufficiently purified from the microorganism and this purified protein is used, the microorganism is only a source of the protein. Thus, as already described above, the microorganism is not limited by whether the microorganism produces the byproduct or not.

Synthesis of L-fuculose using the microorganism may be performed by carrying out the reaction so that the protein having the activity to synthesize L-fuculose derived from the microorganism may act upon the substrate L-fucitol. For example, the microorganism may be cultured and L-fucitol may be added to the culture of the microorganism. Synthesis with the treated microbial cell product may be performed by mixing the treated microbial cell product with L-fucitol so that the protein having the activity to synthesize L-fuculose derived from the microorganism may act upon the substrate L-fucitol. That is, the reaction system may be constructed in the same manner as the reaction system that employs the enzyme or a bioactive substance.

### 1-3. method using genetically engineered strain

As another embodiment of the present invention, there is provided a method using a microorganism having a first protein having the activity to synthesize L-fuculose, but in which a gene encoding a second protein having the activity to produce the byproduct has been knocked out. Even though the original microorganism produces the byproduct, knocking out of the enzyme gene which causes the production of the byproduct may give a microorganism suitable for the production of L-fuculose. Such a microorganism can efficiently produce L-fuculose with no substantial production of the byproduct.

Gene knockout refers to that the gene encoding the target protein is modified to reduce or lose the function of the intact protein. The "gene" refers to a medium which encodes gene information, and the medium includes DNA, RNA, and polynucleotides such as hybrid molecules thereof or chimera molecules thereof. The gene knockout includes techniques of introducing a mutation by deletion, substitution, insertion and inversion so that the protein itself is modified or techniques of inhibiting the expression of the protein by transcription inhibition or translation inhibition.

The method for knocking out the gene is not particularly limited. To knock out the gene, for example, the gene may be mutated or the gene may be deleted. Inactivation of the gene may be performed by general methods of the gene knockout such as mutagenesis by UV irradiation or treatment with N-methyl-N'-nitro-N-nitrosoguanidine, site-directed mutagenesis, homologous recombination or insertion-deletion mutagenesis referred to as homologous recombination or "Red-driven integration" (Datsenk K.A. and Wanner B.L., Proc. Natl. Acad. Sci. USA, 97(12:6640-45, 2000).

For example, when the gene is knocked out by the homologous recombination, a DNA (recombinant) containing the deleted type gene modified may be prepared by deleting a part of the target gene so as not to produce peptidase which normally functions. A microorganism in which a peptidase gene on a chromosome has been deleted may be obtained by transforming the microorganism with the DNA containing the deleted type gene to cause the recombination between the deleted type peptidase gene and the chromosomal peptidase gene.

As the second protein subjected to the gene knockout, the protein having the high activity to synthesize ketohexose other than L-fuculose from L-fucitol is suitable.

As another embodiment using the genetically engineered microorganism, there is provided a method using the microorganism not substantially having the ability to synthesize ketohexose other than L-fuculose from L-fucitol and transformed to become capable of expressing the first microorganism-derived protein having the dehydrogenase activity to synthesize L-fuculose from L-fucitol. Even though the original microorganism does not have the protein having the activity to synthesize L-fuculose, it is possible to obtain a microorganism suitable for the production of L-fuculose by modifying the microorganism to obtain a transformant in which the gene has been introduced to express such a protein. When producing the transformant, the microorganism which originally does not have the ability to produce the byproduct may be selected so as to easily obtain the microorganism which efficiently produces L-fuculose without producing the byproduct.

As the microorganism not substantially having the ability to synthesize ketohexose other than L-fuculose from L-fucitol, it is possible to use the microorganism in which the gene encoding the protein having the ability to synthesize ketohexose other than L-fuculose from L-fucitol has been knocked out, in addition to the microorganisms which originally do not have such an ability. Examples of the protein to be subjected to the gene knockout may include the protein having the activity to synthesize D-xylulose from D-arabitol.

The transformant may be produced in accordance with the standard methods. For example, the transformant may be obtained as follows. An objective protein such as a protein having the activity to synthesize L-fucose is obtained from the aforementioned microorganism, and an amino acid sequence of the purified objective protein is determined. The amino acid sequence may be determined using Edman method (Edman, P., Acta Chem. Scand. 4, 227 (1950)). The amino acid sequence may also be determined using a sequencer supplied from Applied Biosystems. As to the purified protein, the amino acid sequence of 30 amino acid residues from the N terminus is determined, and a nucleotide sequence of the DNA encoding this sequence may be deduced based on the determined amino acid sequence. Universal codons are employed for deducing the nucleotide sequence of the DNA.

Based on the deduced nucleotide sequence, a DNA molecule of about 30 base pairs is synthesized. The method for synthesizing the DNA molecule is disclosed in Tetrahedron Letters, 22, 1859 (1981). The DNA molecule may also be synthesized using a synthesizer supplied from Applied Biosystems. The DNA molecule synthesized in this way may be utilized as a probe for isolating the DNA in full length encoding the objective protein from a chromosomal gene library of the microorganism. The DNA molecule may also be utilized as a primer when the DNA encoding the objective protein is amplified by PCR method.

Manipulation in the PCR method is described in White, T.J. et al., Trends Genet. 5, 185 (1989). The method for preparing the chromosomal DNA and the method for isolating the objective DNA molecule from the gene library using the DNA molecule as the probe are described in Molecular Cloning, 2nd edition, Cold Spring Harbor press (1989) .

The method for determining the nucleotide sequence of the isolated DNA encoding the objective protein is described in A Practical Guide to Molecular Cloning, John Wiley & Sons, Inc. (1985). The nucleotide sequence may also be determined using the DNA sequencer supplied from Applied Biosystems.

Subsequently, the preparation of the transformant which expresses the objective protein will be described. There has been reported numerous examples for producing useful proteins such as enzymes and physiologically active substances by taking advantage of recombinant DNA technology. By the use of the recombinant DNA technology, it is possible to perform mass production of the useful protein which naturally present in a trace amount.

To prepare the transformant which expresses the objective protein, the DNA molecule isolated by the aforementioned method may be introduced into a host. That is, the isolated DNA molecule is incorporated into an expression vector in a expressible manner, and this is introduced into the host cell.

When the protein is produced on a large scale using the recombinant gene technology, as the transformed host cells, bacterial cells, actinomycetal cells, yeast cells, fungal cells, plant cells and animal cells may be used. Generally, enteric bacteria, preferably *Escherichia coli* is suitable, because there are many findings for the technology of producing the protein on a large scale using the enteric bacteria such as *Escherichia coli*. The method for producing the introduced protein using transformed *Escherichia coli* will be described below.

As a promoter to express the DNA encoding the objective protein, the promoter conventionally used for the production of a xenogeneic protein in *Escherichia coli* may be used. Example thereof may include potent promoters such as T7 promoter, lac promoter, trp promoter, trc promoter, and tac promoter, as well as P_{R} promoter and P_{L} promoter of lambda phage.

This is ligated to the gene encoding the objective protein so as to match reading frames of the codons. The ligation may be performed at appropriate restriction enzyme sites. Alternatively, a synthetic DNA of an appropriate sequence may be utilized.

In order to increase the amount of production, it is preferable in some cases that a terminator which is a transcription termination sequence is ligated to downstream of the protein gene. The terminator may include T7 terminator, fd phage terminator, T4 terminator, a terminator of a tetracycline resistant gene and a terminator of *Escherichia coli* trpA gene.

As the vector for introducing the gene encoding the objective protein into *Escherichia coli*, so-called multicopy type vectors are preferable. Examples thereof may include plasmids having a replication origin derived from ColE1, e.g., pUC based plasmids, pBR322 based plasmids or derivative thereof. As used herein, the "derivative" means the plasmid modified by substitution, deletion, insertion, addition and/or inversion. The modification referred to herein may be the modification by mutagenesis by a mutagen or UV irradiation or natural mutation. More specifically, as the vector, for example, pUC19, pUC18, pBR322, pHSG299, pHSG298, pHSG399, pHSG398, RSF1010, pMW119, pMW118, pMW219, and pMW218 may be used. Additionally, the vectors of phage DNA or transposon DNA may be used.

In order to select the transformant, it is preferable that the vector has a marker such as an ampicillin resistant gene. As such a plasmid, the expression vectors with the potent promoter are commercially available [pUC series (Takara Shuzo Co., Ltd.), pPROK series (Clonetech), pKK233-2 (Clonetech)].

A DNA fragment obtained by ligating the promoter, the gene encoding a peptide synthesizing enzyme or a fusion protein of the peptide synthesizing enzyme and another protein and the terminator in this sequence is ligated to the vector DNA to yield a recombinant DNA.

*Escherichia coli* is transformed with the resulting recombinant DNA, and this *Escherichia coli* is cultured to express and produce the objective protein. As the host to be transformed, the strain usually used for the expression of the xenogeneic gene may be used. It is preferable to use *Escherichia coli* JM109 strain which is one of *Escherichia coli* K12 subspecies. The method for transforming and the method for selecting the transformant are described in Molecular Cloning, 2nd edition, Cold Spring Harbor press (1989).

As the medium for the cultivation, the media such as M9-casamino acid medium and LB medium usually used for culturing *Escherichia coli* may be used. A cultivation condition and a production induction condition are appropriately selected depending on the types of the marker and the promoter of the vector used, and the type of the host microorganism.

The objective protein may be collected by the same techniques as in the aforementioned separation and purification of the protein.

### 1-4. Method of production under reaction condition where production of byproduct is inhibited

Even though the microorganism synthesizes the byproduct other than L-fuculose from L-fucitol, by using this microorganism and by oxidizing L-fucitol under the condition where the production of the byproduct is inhibited, it is possible to produce L-fuculose in an industrially available manner. In order to inhibit the production of the byproduct, for example, a pH condition may be adjusted, or an inhibitor which selectively inhibits the enzyme which controls the production of the byproduct may be added.

The pH condition which lead to inhibiting the production of the byproduct varies depending on the type of the microorganism. Thus, a preliminary experiment is performed depending on the type of the microorganism. The preliminary experiment may be performed under the condition shown in the following Example, and those skilled in the art may set the pH condition to inhibit the byproduct based on the following example.

In order to inhibit the production of the byproduct, the inhibitor for the production of the byproduct may be added to the reaction system. It is desirable that the inhibitor dose not inhibit the production of L-fuculose from L-fucitol at all, but any inhibitor can be used as long as L-fuculose is sufficiently produced relatively compared with the production of the byproduct. Examples of the inhibitor for the production of the byproduct may include a bivalent ion chelator and preferably EDTA (ethylenediamine tetraacetate).

### 1-5. NAD supply system

As shown in the following Example, it has been shown that the protein having the activity to synthesize L-fuculose from L-fucitol is NAD-dependent. In this case, NAD is required in a molar amount equivalent to the amount of L-fuculose to be produced. However, NAD is expensive, and considering the industrial production scale, the addition of NAD in the equivalent amount is disadvantageous in terms of production cost. Based on these findings, there is provided a method for producing L-fuculose, in which L-fuculose is synthesized from L-fucitol in the presence of the protein having the activity to reproduce NAD from NADH that has been converted from NAD upon synthesis of L-fuculose. By constituting the reaction system in which NAD is supplied, it is possible to produce L-fuculose continuously and sufficiently, and the method is extremely advantageous in terms of industrial production.

The microorganism having the ability to synthesize L-fuculose from L-fucitol may have the protein having the activity to produce NAD from NADH in some cases. In such a case, the microorganism or the treated microbial cell product of the microorganism may be added to the reaction system. When the protein having the activity to produce NAD from NADH is not present in the reaction system, the protein having this activity may be added thereto. Many dehydrogenases have the activity to produce NAD from NADH, and a reduction activity of this dehydrogenase may be utilized. In this case, NADH is utilized as a coenzyme, and thus, it is necessary to add the additional substrate of this reaction. Other than these dehydrogenases, oxidase and peroxidase which oxidize NADH to NAD may be used. The substrate required for the reaction is oxygen for NADH oxidase or hydrogen peroxide for peroxidase. In the former case, the substrate is easily supplied into a reaction solution by stirring the reaction solution. In the latter case, hydrogen peroxide is very inexpensive and advantageous in terms of cost. These oxidase and peroxidase known publicly may be used and are also commercially available.

### 2. Method for producing L-fucose

Subsequently, the method for producing L-fucose of the present invention will be described. The method for producing L-fucose of the present invention includes a step of synthesizing L-fuculose in which L-fuculose is synthesized in accordance with the method for producing L-fuculose of the present invention, and a step of synthesizing L-fucose in which L-fucose is synthesized from L-fuculose. The aforementioned steps may be performed in the separated reaction systems or the combined reaction system. L-Fucitol which is the raw material of L-fuculose is easily obtained from D-galactose which is the inexpensive material, and the method of the present invention is easy and simple. Therefore, the method for producing L-fucose of the present invention is the industrially excellent method which is easy and simple and can reduce the cost.

The step of synthesizing L-fuculose is as described in the above section "1. Method for producing L-fuculose". In the method for producing L-fuculose of the present invention, the various embodiments as mentioned above may be employed.

Subsequently, the step of synthesizing L-fucose will be described. The method for synthesizing L-fucose from L-fuculose is shown in, for example Patent Document 5. Preferably, L-fucose isomerase is utilized for obtaining L-fucose. Examples L-fucose isomerase may include those whose sequences have been registered in the database in, for example, National Center for Biotechnology Information.

In the case of using L-fucose isomerase, the microorganism transformed to express L-fucose isomerase may be produce to produce L-fucose isomerase, and the produced L-fucose isomerase may be then isolated for use. Alternatively, the microorganism transformed to express L-fucose isomerase or the treated microbial cell product of the microorganism may be used. The transformant may be made in the same way as the technique described in the aforementioned section "1-3. Method using genetically engineered strain".

As a preferable embodiment of the method for producing L-fucose of the present invention, there is a method in which a co-expressing transformant is made and this is used to synthesize L-fucose from L-fucitol in one reaction system. In this embodiment, L-fucose is synthesized from L-fucitol in the presence of at least one of the following: a microorganism transformed to be capable of expressing a microorganism-derived protein not substantially having the ability to synthesize ketohexose other than L-fuculose from L-fucitol and having the dehydrogenase activity to synthesize L-fuculose from L-fucitol, and another protein having the activity to synthesize L-fucose from L-fuculose; and a treated microbial cell product of the microorganism. By preparing such a co-expressing transformant and using this as described above, it is possible to simplify the process, which is highly advantageous as the industrial production.

### 3. Protein having dehydrogenase activity to synthesize L-fuculose from L-fucitol and polynucleotide encoding the same

Example of the protein not substantially having the ability to synthesize ketohexose other than L-fuculose from L-fucitol and having the dehydrogenase activity to synthesize L-fuculose from L-fucitol may specifically include the following protein (A) or (B):
(A) the protein having an amino acid sequence described in SEQ ID NO:16; or
(B) the protein having an amino acid sequence including one or several amino acid mutations selected from the group consisting of substitution, deletion, insertion, addition and inversion in the amino acid sequence described in SEQ ID NO:16, and having the dehydrogenase activity to synthesize L-fuculose from L-fucitol.

The aforementioned protein (A) may be isolated from *Gluconobacter oxydans* IFO 3255. The protein of the present invention includes the protein which is substantially identical to (A). Specifically, the protein (B) is one of the protein which is substantially identical to (A). The substantially identical protein may also be isolated from each bacterial strain described in Table 1. However, the protein of the present invention specified by the aforementioned sequence is not limited by its source from which the protein has been isolated. As described in detail below, the aforementioned protein may be produced by preparing the transformed microorganism based on the aforementioned sequence using the gene recombinant technology.

As used herein, "several" varies depending on a position and a type in a three dimensional structure of the protein with amino acid residues, and are in the range where the three dimensional structure and the activity of the protein with amino acid residues are not significantly impaired. Specifically, "several" ranges from 2 to 100, preferably from 2 to 50, more preferably from 2 to 30 and still more preferably from 2 to 10. However, in the case of the amino acid sequence (B) having one or several amino acid mutations selected from the group consisting of one or several substitutions, deletions, insertions, additions and inversions in the amino acid sequence of the protein, it is desirable that the enzyme derived from the sequence retains the enzyme activity at about a half or more, preferably 80% or more, more preferably 90% or more and particularly preferably 95% or more of the protein (A) under the conditions of 30°C and pH 9.5.

The amino acid mutation described in (B) may be obtained by modifying the nucleotide sequence of the gene encoding the protein of the present invention using, e.g., site-directed mutagenesis so that the amino acid at specific position is substituted, deleted, inserted, or added. The polypeptide having the modified nucleotide sequence in accordance with the aforementioned sequence may be obtained by conventionally known mutagenesis treatments. The mutagenesis treatments may include the method of *in vitro* treatment of the DNA encoding (A) with hydroxylamine and the method of treating *Escherichia* coli having the DNA encoding (A) with ultraviolet ray irradiation or the mutagen such as N-methyl-N'-nitro-N-nitrosoguanidine (NTG) or nitrous acid that are used for an ordinary artificial mutagenesis.

The aforementioned mutations by amino acid substitutions, deletions, insertions, additions and inversions owing to the nucleotide modifications may also include naturally occurring mutations such as differences depending on species and bacterial strains of the microorganisms. The DNA molecule encoding the protein substantially identical to the protein described in SEQ ID NO:16 may be obtained by expressing the DNA molecule having the aforementioned mutations in the appropriate cell and examining the present enzyme activity of an expressed product.

A polynucleotide encoding the protein for the method for producing L-fuculose of the present invention may include a polynucleotide encoding the amino acid sequence described in SEQ ID NO:16. There can be a plurality of nucleotide sequences which define one amino acid sequence because of codon degeneracy. That is, the polynucleotide of the present invention includes the polynucleotides having the nucleotide sequences encoding the following proteins:
(A) the protein having an amino acid sequence described in SEQ ID NO:16; or
(B) the protein having an amino acid sequence including one or several amino acid mutations selected from the group consisting of substitution, deletion, insertion, addition and inversion in the amino acid sequence described in SEQ ID NO:16, and having the dehydrogenase activity to synthesize L-fuculose from L-fucitol.

As the nucleotide sequence encoding the amino acid sequence described in SEQ ID NO:16, the nucleotide sequence described in SEQ ID NO:15 is exemplified. In addition, the polynucleotide substantially identical to the DNA having the nucleotide sequence described in SEQ ID NO:15 may include the followings. The polynucleotide substantially identical to the polynucleotide having the nucleotide sequence described in SEQ ID NO:15 may be obtained by isolating the polynucleotide which hybridizes with the polynucleotide consisting of the nucleotide sequence complementary to the nucleotide sequence described in SEQ ID NO:15 or a probe prepared from the nucleotide sequence under a stringent condition and encodes the protein having the dehydrogenase activity to synthesize L-fuculose from L-fucitol.

That is, the preferable polynucleotides of the present invention may include the polynucleotides shown in the following (a) and (b) as specific examples.
(a) The polynucleotide having the nucleotide sequence described in SEQ ID NO:15; and
(b) the polynucleotide which hybridizes with the polynucleotide having the nucleotide sequence complementary to the nucleotide sequence described in SEQ ID NO:15 under the stringent condition and encodes the protein having the dehydrogenase activity to synthesize L-fuculose from L-fucitol.

The probe may be prepared based on the nucleotide sequence described in SEQ ID NO:15 by a conventional method. Using the probe, the polynucleotide which hybridizes with this probe may be captured in accordance with a conventional method to isolate the objective polynucleotide. For example, the DNA probe may be prepared by amplifying the nucleotide sequence cloned into the plasmid or the phage vector, cutting out the desired nucleotide sequence as the probe using restriction enzymes and extracting it. Sites to be cut out may be controlled depending on the objective DNA.

As used herein, the "stringent condition" refers to the condition where a so-called specific hybrid is formed whereas non-specific hybrid is not formed. Although it is difficult to clearly quantify this condition, examples thereof may include the condition where a pair of DNA sequences with high homology, e.g., DNA sequences having the homology of 50% or more, more preferably 80% or more, still more preferably 90% or more and particularly preferably 95% or more are hybridized whereas DNA with lower homology than that are not hybridized, and a washing condition of an ordinary Southern hybridization, i.e., hybridization at salt concentrations equivalent to 1 x SSC and 0.1% SDS at 60°C, and preferably 0.1 x SSC and 0.1% SDS at 60°C. The genes which hybridize under such a condition may include those in which a stop codon has occurred or an active center has been lost by the mutation of the active center. However, those may be easily removed by ligating the gene to the commercially available vector, expressing it in the appropriate host, and measuring the enzyme activity of the expressed product by the methods described in the following Example.

In the polynucleotide having the nucleotide sequence encoding the aforementioned protein (B) and the polynucleotide (b), it is desirable that the polynucleotide encodes the protein which retains the dehydrogenase activity to synthesize L-fuculose from L-fucitol at about a half or more, more preferably 80% or more and still more preferably 90% of the protein having the amino acid sequence encoded by the nucleotide sequence of SEQ ID NO:1 under the conditions of 30°C and pH 9.5.

The DNA having the nucleotide sequence of SEQ ID NO:15 may be obtained from the chromosomal DNA or the DNA library of *Gluconobacter oxydans* IFO 3255 or IFO 3171 by PCR (see Polymerase chain reaction, White, T. J. et al; Trends Genet., 5, 185(1989)) or hybridization. Primers used for PCR may be designed based on an internal amino acid sequence determined based on the purified protein having the peptide-synthesizing activity. The full length coding region of the present protein may be amplified by using the primers corresponding to 5' non-translation region and 3' non-translation region as the PCR primers.

The primer may be synthesized using a DNA synthesizer model 380B supplied from Applied Biosystems and a phosphoamidite method (see Tetrahedron Letters (1981), 22, 1859) in accordance with conventional methods. The PCR reaction may be performed by using Gene Amp PCR System 9600 (PERKIN ELMER) and TaKaRa LA PCR in vitro Cloning Kit (Takara Bio Inc.) in accordance with the methods instructed by suppliers such as manufacturers.

The methods for producing the recombinant polynucleotide such as an expression vector, and the transformant using the aforementioned polynucleotide is as described in the section "1-3. Method using genetically engineered strain".

### 4. Protein having NADH oxidase activity and polynucleotide encoding the same

As described above, one preferable embodiment of the method for producing L-fuculose of the present invention may be an embodiment in which NADH oxidase is added into the reaction system. As the specific examples of NADH oxidase used herein, the following proteins (C) and (D) are exemplified.
(C) The protein having an amino acid sequence described in SEQ ID NO:18; and
(D) the protein having an amino acid sequence including one or several amino acid mutations selected from the group consisting of substitutions, deletions, insertions, additions and inversions in the amino acid sequence described in SEQ ID NO:18, and having the NADH oxidase activity.

The aforementioned protein (C) may be isolated from *Gluconobacter oxydans* IFO 3255. The protein of the present invention includes the protein substantially identical to (C). Specifically the protein (D) is one of the protein substantially identical to (C). Furthermore, it is likely that the substantially identical protein may be isolated from each bacterial strain described in Table 1.

What is meant by "several" herein, the method for introducing the mutation and the types of the mutations are the same as described in "3. Protein having dehydrogenase activity to synthesize L-fuculose from L-fucitol and polynucleotide encoding the same". However, the proteins of the present invention specified by the aforementioned sequences are not limited by their source from which they have been isolated.

As the polynucleotide encoding the protein for the method for producing L-fuculose of the present invention, the polynucleotide encoding the amino acid sequence described in SEQ ID NO:18. There can be a plurality of nucleotide sequences which define one amino acid sequence because of codon degeneracy. That is, the polynucleotide of the present invention includes the polynucleotides having the nucleotide sequences encoding the following proteins:
(C) the protein having an amino acid sequence described in SEQ ID NO:18; or
(D) the protein having an amino acid sequence including one or several amino acid mutations selected from the group consisting of substitution, deletion, insertion, addition and inversion in the amino acid sequence described in SEQ ID NO:18, and having the NADH dehydrogenase activity.

As the nucleotide sequence encoding the amino acid sequence described in SEQ ID NO:18, the nucleotide sequence descried in SEQ ID NO:17 is exemplified. That is, the preferable polynucleotides of the present invention may include the polynucleotides shown in the following (c) and (d) as specific examples.
(c) The polynucleotide having the nucleotide sequence described in SEQ ID NO:17; and
(d) the polynucleotide which hybridizes with the polynucleotide having the nucleotide sequence complementary to the nucleotide sequence described in SEQ ID NO:17 under the stringent condition and encodes the protein having the NADH dehydrogenase activity.

The "stringent condition", the method for producing the probes and the primers for the isolation, and the method for isolating DNA using the same are identical with those described in "3. Protein having dehydrogenase activity to synthesize L-fuculose from L-fucitol and polynucleotide encoding the same".

When the polynucleotide encoding NADH oxidase is incorporated into a recombinant polynucleotide such as an expression vector, the aforementioned polynucleotide alone may be incorporated, or the aforementioned polynucleotide may be incorporated together with the polynucleotide encoding "the protein having the dehydrogenase activity to synthesize L-fuculose from L-fucitol" in the same vector. Furthermore, upon producing the transformant, the transformant which expresses the protein having the NADH oxidase activity and the transformant which expresses the protein having the dehydrogenase activity to synthesize L-fuculose from L-fucitol may be produced separately. Alternatively, the transformant may be designed so that they are co-expressed in the same microorganism. However, when NADH oxidase is used together with another dehydrogenase as the NAD reproduction system as in the present invention, the dehydrogenase, the NADH oxidase and NAD are necessary to be present in the same reaction system. In the case of the enzymatic reaction using the produced enzyme or the case of using the treated microbial cell product obtained by disrupting the cell membrane, the dehydrogenase and the NADH oxidase may be produced in separate hosts. However, when the untreated microbial cells are used as a catalyst, it is desirable to co-express the both enzyme in the same host.

### EXAMPLES

The present invention will be described in more detail hereinbelow with reference to Examples, but the invention is not limited thereto.

### Example 1: Search for microorganism which oxidizes L-fucitol to synthesize L-fuculose (1)

Each of acetobacterium strains was cultured on YPG agar medium (10 g/l glycerol, 0.3 g/l yeast extract, 0.3 g/l peptone, 20 g/l agar, pH 6.5) at 30°C for 18 to 66 hours for refreshment, and one platinum loopful thereof was then seeded in 0.5 mL of a liquid medium containing 10 g/l (61 mM) L-fucitol, 10 g/l glycerol, 3g/l yeast extract, 3 g/l peptone and 20 g/l CaCO₃ pH 6.5 that had been sterilized by autoclaving at 120°C for 20 minutes. Cultivation was then performed with shaking at 30°C for 42 hours. A 96-well microplate having 2 ml volume per well was used for the cultivation. Microbial cells were removed from the cultured medium by centrifugation. The supernatant obtained by the centrifugation was analyzed by high performance liquid chromatography (HPLC) to quantify the concentration of produced L-fuculose. Analysis conditions for HPLC were as follows:
column: Sugar SC1011 supplied from Shodex, diameter: 10 mm, length: 300 mm
column temperature: 75°C
mobile phase: 50 ppm Ca-EDTA in H₂O
flow rate: 1.2 ml/minute
detection: differential refractive index (RI) detector

In this analysis, L-fucose, L-fucitol and L-fuculose were eluted at about 7.6, 11.9 and 13.6 minutes of analytical times, respectively. By using commercial available preparations of L-fucitol and L-fucose supplied from Sigma as standard compounds, their eluted positions were identified and their concentrations in analyzed preparations were calculated. For L-fuculose, no commercial available product was available as the standard preparation. Thus, a substance produced from L-fucose by L-fucose isomerase which will be described below in detail was regarded as L-fuculose. In this case, the sum of the integral value of the peak (peak area value) of remaining fucose and the integral value of the peak of produced L-fuculose on a chart was scarcely changed and was constant regardless of before and after reacting with L-fucose isomerase. Thus, the peak area value of L-fuculose per unit concentration was regarded to be the same as that of L-fucose, and the concentration of L-fuculose was calculated accordingly in the analyzed preparation. The detection limit is about 1 mM in any cases in the analyses in the following Examples. In many analyses, an unidentified peak which seemed to be a transformed product derived from L-fucitol because of its peak area size was observed at about 8.0 minutes of the analytical time. The concentration of this unidentified substance (referred to hereinbelow as BP) eluted at about 8 minutes of the analytical time was expediently calculated using the peak area value of L-fucose per unit concentration, i.e., in the same manner as calculation of L-fuculose concentration. Analysis results are shown in Table 1. Hereinbelow, "*Gluconobacter*" and "*Acetobacter*" are sometimes abbreviated as "G." and "A.", respectively.

**Table 1. MICROORGANISMS WHICH PRODUCE L-FUCULOSE FROM L-FUCITOL (1)**

| Strain | L- FUCULOSE (mM) | BP (mM) | L-FUCITOL (mM) | L-FUCULOSE/ (L-FUCULOSE + BP) (wt%) |
|---|---|---|---|---|
| *Gluconobacter oxydans* IFO 3171 | 14.4 | 25.5 | 6.9 | 36.1 |
| *G. frateurii* IFO3264 | 14.1 | 27.3 | 16.2 | 34.1 |
| *G. oxydans* ATCC621 | 12.3 | 17.3 | 20.0 | 41.6 |
| *Acetobacter melanogenus* IFO3190 | 12.2 | 17. 3 | 20.5 | 41.4 |
| *G. frateurii* IFO3268 | 11.6 | 18.7 | 29.0 | 38.3 |
| *G. roseus* AJ2840 | 10.3 | 13.1 | 22.9 | 44.0 |
| *G. cerinus* IFO3262 | 10.2 | 14.5 | 26.2 | 41.3 |
| *G. oxydans* IFO3255 | 9.1 | 14.5 | 36.4 | 38.6 |
| *G. roseus* AJ2845 | 9.0 | 17.0 | 23.9 | 34.6 |
| *G. oxydans subsp. suboxydans* AJ2866 | 7.8 | 21.9 | 23.9 | 26.3 |
| *G. roseus* AJ2843 | 7.7 | 13.8 | 28.1 | 35.8 |
| *G. oxydans I*FO3294 | 7.6 | 15.5 | 28.0 | 32.9 |
| *G. cerinus* IFO3276 | 6.9 | 11.6 | 37.3 | 37.3 |
| *G. suboxydans* AJ2849 | 6.3 | 15.4 | 26.5 | 29.0 |
| *A. melanogenus* AJ2867 | 5.8 | 10.5 | 36.0 | 35.6 |
| *G. cerinus* IFO3267 | 5.5 | 10.2 | 39.4 | 35.0 |
| *G. frateurii* IFO3254 | 4.9 | 8.9 | 46.2 | 35.5 |
| *A. turbidans* AJ2908 | 4.0 | 7.3 | 42.9 | 35.4 |
| *G. suboxydans* AJ2846 | 3.9 | 10.2 | 31.4 | 27.7 |
| *G. xylinus subsp. xylinus* ATCC53582 | 3.6 | 0.0 | 54.8 | 100.0 |
| *A. aurantius* IFO3246 | 3.1 | 7.5 | 40.1 | 29.2 |
| *G. melanogenus* AJ2876 | 2.9 | 7.9 | 39.9 | 26.9 |
| *G. oxydans* IFO14819 | 2.4 | 8.9 | 49.0 | 21.2 |
| *G. scleroideus* AJ2838 | 1.4 | 2.5 | 45.3 | 35.9 |
| *G. suboxydans* AJ2841 | 1.3 | 3.6 | 46.2 | 26.5 |
| *G. xylinus subsp. xylinus* ATCC23767 | 1.1 | 1.0 | 52.2 | 52.4 |
| *G. oxydans* IFO 3189 | 1.0 | 0.1 | 54.1 | 90.9 |

| | | | | |
|---|---|---|---|---|
| ^{*} The microorganisms which produced 1 mM or more of L-fuculose under the conditions in Example 1 are shown. | | | | |

Among the aforementioned microorganisms, those having ATCC number have been deposited to American Type Culture Collection (P.O. Box 1549 Manassas, VA 20110, the United States of America), and are available with reference to each number. Among the aforementioned microorganisms, those having IFO number have been deposited to Institute for Fermentation Osaka (17-85 Juso-honmachi 2-chome, Yodogawa-ku, Osaka, Japan). However, its business has been transferred to NITE Biological Resource Center (NBRC) in Department of Biotechnology, National Institute of Technology and Evaluation (NITE) since June 30, 2002, and the microorganisms are available from NBRC with reference to the aforementioned IFO number.

### Example 2: Search for microorganism which oxidizes L-fucitol to synthesize L-fuculose (2)

In order to induce the expression of an oxidation enzyme of L-fucitol by dulcitol whose structure is similar to that of L-fucitol, each of acetobacterium strains was cultured on YP-dulcitol agar medium (10 g/l dulcitol, 0.3 g/l yeast extract, 0.3 g/l peptone, 20 g/l agar, pH 6.5) at 30°C for 18 to 66 hours for refreshment, and one loopful thereof was then seeded in 0.5 mL of the liquid medium containing 10 g/l L-fucitol, 3g/l yeast extract and 3 g/l peptone pH 6.5 that had been sterilized by autoclaving at 120°C for 20 minutes. Cultivation was then performed with shaking at 30°C for 42 hours. This cultured medium was analyzed in the same way as in Example 1. The analysis results are shown in Table 2.

**TABLE 2. MICROORGANISMS WHICH PRODUCE L-FUCULOSE FROM L-FUCITOL (2)**

| Strain | L-FUCULOSE (mM) | BP (mM) | L-FUCITOL (mM) | L-FUCULOSE/ (L-FUCULOSE + BP) (wt%) |
|---|---|---|---|---|
| *G. xylinus subsp.xylinus* ATCC53582 | 7.8 | 0.0 | 48.5 | 100.0 |
| *Glucanobacter roseus* AJ2840 | 3.8 | 6.9 | 49.9 | 35.5 |
| *G. oxydans subsup suboxydans* AJ2866 | 3.6 | 10.4 | 34. 5 | 25.7 |
| *G. oxydans* IFO3255 | 1.6 | 6.2 | 41. 7 | 20.5 |
| *G. oxydans* IFO3189 | 1.6 | 0.1 | 57.6 | 94.1 |

| | | | | |
|---|---|---|---|---|
| * The microorganisms which produced 1 mM or more of L-fuculose under the conditions in Example 2 are shown. | | | | |

In both Examples 1 and 2, a plurality of acetobacterium species belonging to genus *Gluconobacter* or *Acetobacter* which produced L-fuculose using L-fucitol as the raw material have been found, although many of them produced BP other than L-fuculose simultaneously. The microorganism which produced only L-fuculose is only one strain, *Gluconobacter xylinus subsp. xylinus* ATCC 53582. *Gluconobacter xylinus subsp. xylinus* ATCC 23767 and *Gluconobacter oxydans* IFO 3189 in addition to *Gluconobacter xylinus subsp. xylinus* ATCC 53582 were found to produce L-fuculose at 50% or more based on the total amount of produced L-fuculose and BP.

### Example 3: Identification of substance assumed to be L-fuculose by HPLC analysis

In order to identify the substance assumed to be L-fuculose by HPLC analysis, bioassay using L-fucose isomerase was conducted.

L-Fucose isomerase (EC 5.3.1.3., referred to hereinbelow as "FucI") is an enzyme which catalyzes isomerization between L-fuculose and L-fucose. If the compound assumed to be L-fuculose in Examples 1 and 2 is L-fuculose, it was thought that the compound would be converted into L-fucose by the addition of FucI. Commercially available L-fucose (e.g., supplied from Sigma) is available. Utilizing this product as the standard compound, it is possible to perform HPLC analysis or a colorimetric quantification using L-fucose dehydrogenase described in Patent No. 3132913 Publication.

First, FucI was prepared as follows. FucI derived from *E. coli* K12 strain was used. This enzyme has been registered with accession number AAC75844 in the database of National Center for Biotechnology Information. For the purpose of expressing this enzyme in a large amount in *E. coli*, and for the purpose of inserting a histidine tag at N terminus of FucI for facilitating purification, pQE30 available from QIAGEN was used as the expression vector of FucI. In order to insert a FucI gene (fucI) between SphI and HindIII sites in multicloning sites of pQE30, the following PCR primer 1 (SEQ ID NO:1) and PCR primer 2 (SEQ ID NO:2) were prepared. PCR using as a template the genomic DNA derived from *E. coli* W3100 strain that had been obtained by the standard method was performed to yield a fragment with a length of about 1.8 kbp. This fragment and pQE30 vector were digested with SphI and HindIII and purified, and then ligated to produce a plasmid pQE30FucIH6 for the expression of FucI protein (hereinbelow referred to as FucIH6) having the His-tag sequence at the N terminus. The entire nucleotide sequence and the deduced amino acid sequence of fucIH6 formed by the gene encoded on this plasmid are shown in SEQ ID NOS:3 and 4, respectively. *E. coli* JM109 strain was transformed with this plasmid to produce FucIH6-expressing strain *E. coli* JM109/pQE30FucIH6.

This bacterial strain was cultured at 37°C in LB medium, and the expression of fucIH6 was induced by adding IPTG at a final concentration of 1 mM when absorbance at 600 nm of the medium was about 0.4 during the cultivation. The cultivation was continued for 2 hours after the induction. Then microbial cells were collected by centrifuging the resulting medium, and the obtained microbial cells were washed with 50 mM Tris-HCl (pH 8.0). The washed microbial cells were disrupted by sonication at 200W for 10 minutes. The disrupted product was then centrifuged to obtain a supernatant which was used as a crude enzyme solution. Imidazole at a final concentration of 10 mM and NaCl at a final concentration of 0.3 M were added to this crude enzyme solution, which was then mixed with 1 ml of Ni-NTA resin (supplied from QIAGEN) which had been equilibrated with 50 mM Tris-HCl (pH 8.0), 10 mM imidazole and 0.3 M NaCl (this is referred to as buffer W). The mixture was shaken overnight, whereby protein having the His-tag sequence was bound to the resin. After shaking, the resin was collected by centrifugation. Resin was further washed several times with the buffer W, and transferred to a column. Subsequently, the absorbed protein was eluted by adding 0.2 M imidazole. The eluted fraction thus obtained was dialyzed against 50 mM Tris-HCl (pH 8.0), 1 mM 2-mercaptoethanol and 0.1 mM MnCl₂, and further concentrated through a membrane as needed, for subsequent experiment. The resulting enzyme solution was subjected to SDS-PAGE. As a result, an almost single band with a molecular weight of about 67 kDa which seemed to be FucIH6 was observed.

As the bacterial strains for the conversion experiment of L-fucitol by the acetobacterium, the bacterial strains listed in the following Table 3 were used. One platinum loopful of each acetobacterium strain that had been refreshed was seeded in 0.5 mL of the liquid medium containing 10 g/l L-fucitol, 10 g/l glycerol, 3g/l yeast extract and 3 g/l peptone pH 6.5, and cultured with shaking at 30°C for 42 hours. At that time, the medium from which glycerol had been removed was used for only the cultivation of *Gluconobacter xylinus subsp. xylinus* ATCC 53582 strain. The 96-well microplate with 2 ml per well was used for the cultivation.

The resulting cultured liquid was centrifuged for removing the microbial cells, and an equivalent volume of 0.2 M potassium phosphate buffer (pH 7.0) containing 2 mM 2-mercaptoethanol, 0.2 mM MnCl₂ and 0.4 mg/ml FucIH6 (each final concentration is a half thereof) were added to react at 37°C for 2 hours. At that time, the experimental group in which FucIH6 had not been added was made simultaneously and used as the control.

After completion of the reaction, the reaction solution was analyzed by HPLC. When L-fucose and BP were simultaneously produced, it was difficult to strictly quantify them by HPLC because their elution positions were close. In such a case, L-fucose was also quantified by the colorimetric method using L-fucose dehydrogenase. The quantification was performed by adding L-fucose dehydrogenase (supplied from Kikkoman Corporation, NADP-dependent) at the final concentration of 0.2 mg/ml, NADP at the final concentration of 1 mM in 0.1 M glycine-NaOH buffer (pH 9.5) to the appropriately diluted FucIH6 reaction solution to react at 37°C for 30 minutes and measuring changes of the absorbance at 340 nm induced by NADPH production. The measurement results are shown in Table 3.

**Table 3. Conversion by FuclH6 of compound produced by conversion of L-fucitol**

| (A) With Fucl | | | | | |
|---|---|---|---|---|---|
| | COLORIMETRIC METHOD | HPLC | | | |
| Strain | L-FUCOSE (mM) | L-FUCOSE (mM) | L-FUCULOSE (mM) | BP (mM) | L-FUCITOL (mM) |
| *G. xylinus subsp. xylinus* ATCC53582 | 7.86 | 6.5 | 0 | 17.7 | 34.1 |
| *Gluconobacter roseus* AJ2840 | 7.52 | 7 | 1.6 | 0 | 51.4 |
| *G. oxydans subsup suboxydans* AJ2866 | 7.51 | 6.4 | 0 | 18.5 | 32.8 |
| *G. oxydans* IFO3255 | 7.44 | 6.2 | 0 | 19.6 | 33.5 |
| *G oxydans* IFO3189 | 7.06 | 6 | 0 | 17.3 | 35.1 |
| *G. xylinus subsp. xylinus* ATCC53582 | 6.94 | 5.8 | 0 | 25.6 | 29.1 |
| *Gluconobacter roseus* AJ2840 | 6.72 | 5.2 | 0 | 17.3 | 38.3 |
| *G. oxydans subsup suboxydans* AJ2866 | 5.41 | 3.8 | 0 | 9.6 | 44.5 |
| *G. oxydans* IFO3255 | 5.32 | 3.8 | 0 | 18.6 | 37.2 |
| *G oxydans* IFO 3189 | 5.27 | 3.5 | 0 | 15.6 | 41.7 |
| *G oxydans* IFO 3189 | 2.45 | 1.3 | 0 | 9 | 51.8 |

| (B) Without Fucl | | | | | |
|---|---|---|---|---|---|
| | COLORIMETRIC METHOD | HPLC | | | |
| Strain | L-FUCOSE (mM) | L-FUCOSE (mM) | L-FUCULOSE (mM) | BP (mM) | L-FUCITOL (mM) |
| *Glucanobacter oxydans* IFO 3171 | 0.56 | 0 | 8 | 15.1 | 33.4 |
| *G. xylinus subsp. xylinus* ATCC53582 | 0.59 | 0.1 | 8.2 | 0 | 51.6 |
| *G. oxydans* IFO3255 | 0.75 | 0 | 7.3 | 16 | 35.5 |
| *G roseus* AJ2845 | 0.76 | 0 | 7.2 | 16.1 | 34.2 |
| *G roseus* AJ2843 | 0.41 | 0 | 7.4 | 15.8 | 37.5 |
| *G. oxydans* ATCC621 | 0.58 | 0 | 7 | 23.7 | 29.1 |
| *G. nateurii* IFO3268 | 0.41 | 0 | 6.6 | 15.3 | 38.9 |
| *Acetobacter turbidans* AJ2908 | 0.02 | 0 | 4.4 | 7.8 | 45.8 |
| *G. oxydans subsp. suboxydans* AJ2866 | 0.49 | 0 | 4.8 | 17.6 | 38.1 |
| *G cerinus* IFO3267 | 0.07 | 0 | 4.3 | 12.9 | 45 |
| *G. melanogenus* AJ2876 | 0.67 | 0 | 1.5 | 6.5 | 52.7 |

As a result, the substance assumed to be L-fuculose by the HPLC analysis was converted into L-fucose by the reaction with FucIH6. Thus, by this result, the substance was identified to be L-fuculose as predicted. At the same time, the present experimental results indicate that it is possible to synthesize L-fucose by contacting L-fucose isomerase with L-fuculose synthesized from L-fucitol by the acetobacterium. The quantitative capability of L-fucose had been appeared to be precarious because of poor separation of BP from L-fucose on HPLC. However, a good correlation with the quantitative result by the colorimetric method using L-fucose dehydrogenase was obtained, and it was thus confirmed that L-fucose was quantified by HPLC with almost no problem. Meanwhile, BP which had been believed to be the byproduct was not converted by the addition of FucIH6, and was identified to be the compound different from L-fuculose or L-fucose which was the substrate of FucIH6.

SEQ ID NO:1: Nucleotide sequence of 5' primer for obtaining fucI gene
GAAGCATGCATGAAAAAAATCAGCTTACCG

SEQ ID NO:2: Nucleotide sequence of 3' primer for obtaining fucI gene
TGTTCAGGCCCGGAAGCTTGAGCGACCGGG

SEQ ID NO:3: Nucleotide sequence of fucIH6 gene
atgagaggatcgcatcaccatcaccatcacggatccgcatgcatgaaaaaaatcagctt
accgaaaattggtatccgcccggttattgacggtcgtcgcatgggtgttcgtgagtcgc
ttgaagaacaaacaatgaatatggcgaaagctacggccgcactgctgaccgagaaactg
cgccatgcctgcggagctgccgtcgagtgtgtcatttccgatacctgtatcgcgggtat
ggctgaagccgctgcttgcgaagaaaaattcagcagtcagaatgtaggcctcaccatta
cggtaacgccttgctggtgctatggcagtgaaaccatcgacatggatccaacccgcccg
aaggccatttggggctttaacggcactgaacgccccggcgctgtttacctggcagcggc
tctggcagctcacagccagaaaggcatcccagcattctccatttacggtcatgacgttc

aggatgccgatgacacatcgattcctgccgatgttgaagaaaaactgctgcgctttgcc
cgcgccggtttggccgtcgccagcatgaaaggtaaaagctatctgtcgctgggcggcgt
ttcgatgggtatcgccggttccattgttgatcacaacttctttgaatcctggctgggaa
tgaaagtccaggcggtggatatgaccgaactgcgtcgccgtatcgatcagaagatttac
gacgaagccgaattggaaatggcactggcctgggctgataaaaacttccgctatggcga
agatgaaaataacaaacagtatcaacgtaatgccgagcaaagccgcgcagttctgcgcg
aaagtttactgatggcgatgtgtatccgcgacatgatgcaaggcaacagcaaactggcc
gatattggtcgcgtggaagaatcacttggctacaacgccatcgctgcgggcttccaggg
gcaacgtcactggaccgatcaatatcccaatggtgacaccgccgaagcgatcctcaaca
gttcatttgactggaatggcgtgcgcgaaccctttgtcgtggcgaccgaaaacgacagt
cttaacggcgtggcaatgctaatgggtcaccagctcaccggcaccgctcaggtatttgc
cgatgtgcgtacctactggtcaccagaagcaattgagcgtgtaacggggcataaactgg
atggactggcagaacacggcatcatccatttgatcaactccggttctgctgcgctggac
ggttcctgtaaacaacgcgacagcgaaggtaacccgacgatgaagccacactgggaaat
ctctcagcaagaggctgacgcttgcctcgccgctaccgaatggtgcccggcgatccacg
aatacttccgtggcggcggttactcttcccgcttccttaccgaaggcggcgtcccgttc
accatgactcgtgtcaacatcatcaaaggcctgggaccggtactgcaaatcgcggaagg
ctggagcgtggaattgccgaaggatgtgcatgacatcctcaacaaacgcaccaactcaa
cctggccaaccacctggtttgcaccgcgcctcaccggtaaagggccgtttacggatgtg
tactcggtaatggcgaactggggcgctaaccatggggttctgaccatcggccacgttgg
cgcagactttatcactctcgcctccatgctgcgtatcccggtatgtatgcacaacgttg
aagagaccaaagtgtatcgtccttctgcctgggctgcgcacggcatggatattgaaggc
caggattaccgcgcttgccagaactacggtccgttgtacaagcgttaa

SEQ ID NO:4: Amino acid sequence of FucIH6
MRGSHHHHHHGSACMKKISLPKIGIRPVIDGRRMGVRESLEEQTMNMAKATAALLTEKL
RHACGAAVECVISDTCIAGMAEAAACEEKFSSQNVGLTITVTPCWCYGSETIDMDPTRP
KAIWGFNGTERPGAVYLAAALAAHSQKGIPAFSIYGHDVQDADDTSIPADVEEKLLRFA
RAGLAVASMKGKSYLSLGGVSMGIAGSIVDHNFFESWLGMKVQAVDMTELRRRIDQKIY
DEAELEMALAWADKNFRYGEDENNKQYQRNAEQSRAVLRESLLMAMCIRDMMQGNSKLA
DIGRVEESLGYNAIAAGFQGQRHWTDQYPNGDTAEAILNSSFDWNGVREPFVVATENDS
LNGVAMLMGHQLTGTAQVFADVRTYWSPEAIERVTGHKLDGLAEHGIIHLINSGSAALD
GSCKQRDSEGNPTMKPHWEISQQEADACLAATEWCPAIHEYFRGGGYSSRFLTEGGVPF
TMTRVNIIKGLGPVLQIAEGWSVELPKDVHDILNKRTNSTWPTTWFAPRLTGKGPFTDV

YSVMANWGANHGVLTIGHVGADFITLASMLRIPVCMHNVEETKVYRPSAWAAHGMDIEG
QDYRACQNYGPLYKR*

### Example 4: Conversion of L-fucitol by FucI expressing acetobacterium

In Example 3, it was demonstrated that L-fuculose can be produced from L-fucitol by the acetobacterium, and L-fuculose can be converted into L-fucose using FucI. As to production of L-fucose using L-fucitol as material, it is possible to separately perform the production of L-fuculose from L-fucitol and the production of L-fucose from L-fuculose as in Example 3. However, when the actual production is considered, it is advantageous to simultaneously perform these reactions in terms of simplifying the process. In particular, if the ability to convert L-fuculose to L-fucose was given to the acetobacterium itself, it would become possible to advantagously produce L-fucose from L-fucitol with the single bacterial strain.

For this purpose, *G. oxydans* IFO 3171 strain, a typical strain having the ability to synthesize L-fuculose from L-fucitol, was transformed to construct a strain which expresses FucI derived from *E. coli*. As an example of the expression system of the specific protein in the acetobacterium, it is possible to utilize the method of using the plasmid pSA19 described in Biosci. Biotechnol. Biochem., 67: 584-591 (2003). A gene fragment encoding full length FucI derived from *E. coli* was obtained by PCR in the same way as in Example 3, and this was inserted into the multicloning sites of pSA19 to yield FucI expression plasmid for the acetobacterium, pSA19FucI. However, unlike Example 3, no His-tag was inserted into FucI, and a wild type FucI was expressed. Primers having the sequences of SEQ ID NOS:5 and 6 were used upon PCR cloning from *E. coli.* This makes the predicted amino acid sequence of the expressed FucI and the fuel gene inserted on pSA19FucI identical to those of the wild type enzyme. *G. oxydans* IFO 3171 strain was transformed with pSA19FucI with reference to the aforementioned reference.

SEQ ID NO:5: Nucleotide sequence of 5'-primer for obtaining fucI gene
AACTGAATTCATTTTCCGAATAAAGTGAGG

SEQ ID NO:5: Nucleotide sequence of 3'-primer for obtaining fucI gene
GTTCAGGCCCTGCAGCCGGAGCGACCGGGC

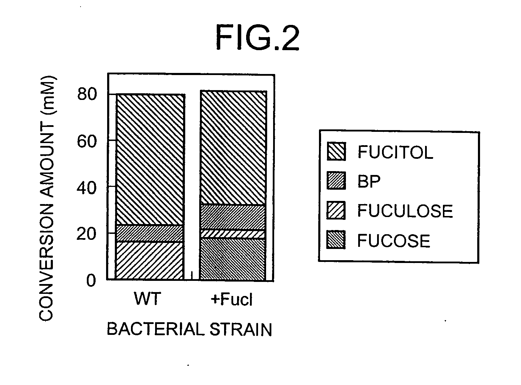

After refreshing the resulting transformant *G. oxydans* IFO3171/pSA19FucI on YPG agar medium, one platinum loopful thereof was seeded in 3 ml of the liquid medium containing 10 g/l L-fucitol, 10 g/l glycerol, 3g/l yeast extract and 3 g/l peptone pH 6.5, and cultured with shaking at 30°C for 18 hours. Microbial cells were collected by centrifuging 1 ml of the resulting medium, and then washed with 0.1 M Tris-HCl buffer (pH 8.0). The washed microbial cells were resuspended in 0.5 ml of the same buffer. L-fucitol at a final concentration of 10 g/l was added thereto, and the mixture was reacted at 30°C for 90 hours in the 96-well microplate having 2 ml volume per well. After the reaction, the microbial cells were removed from the resulting medium by centrifugation, and the supernatant was subjected to HPLC analysis. The total sum of the concentrations of the product and the remaining substrate exceeded the substrate concentration at the onset of the conversion reaction. This appeared to be attributed to the evaporation of the reaction solution during the conversion reaction. The analysis results are shown in FIG. 2. In FIG. 2, WT and +FucI represent the wild type strain and the FucI-expressing strain, respectively.

As a result, the effect as anticipated was observed. That is, L-fuculose which was synthesized by the conversion with the wild type strain was further converted into L-fucose in the FucI-expressing strain. This enabled production of L-fucose from L-fucitol by the conversion with the acetobacterium strain alone.

### Example 5: Attempt to reduce byproduct (BP) in production of L-fuculose from L-fucitol (1); Control of reaction pH

For the purpose of reducing BP which was problematic in the production of L-fuculose from L-fucitol, reaction pH was examined.

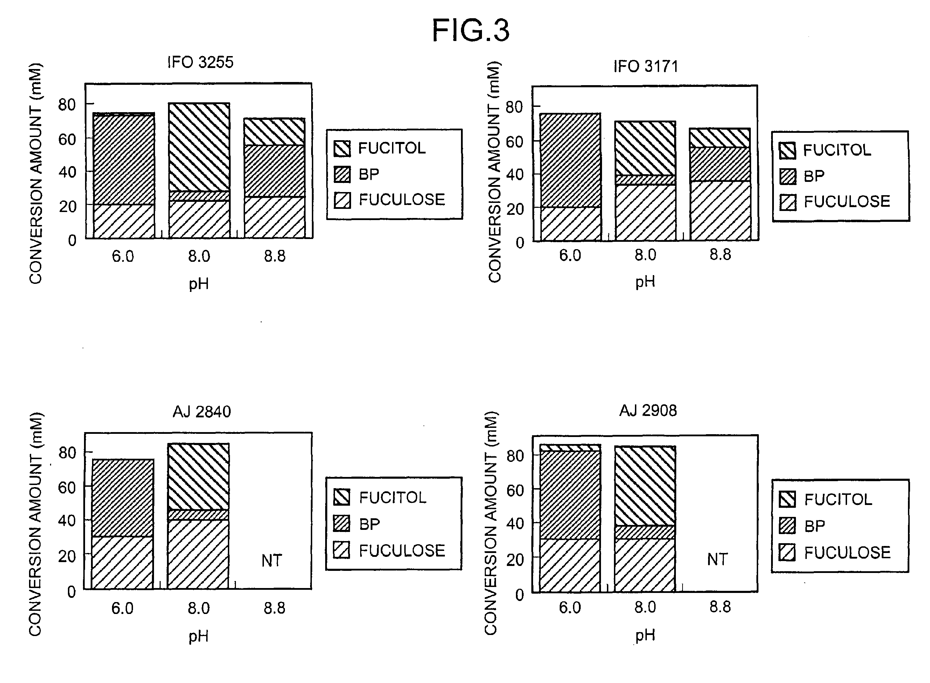

*G. oxydans* IFO 3255 strain, *G. oxydans* IFO 3171 strain, *G. roseus* AJ 2840 strain and *A. turbidans* AJ 2908 strain were used in the experiments. One platinum loopful of the bacterial strain that had been refreshed on YPG agar medium was seeded in the sterilized liquid medium containing 10 g/l glycerol, 10 g/l L-fucitol, 0.3g/l yeast extract, 0.3 g/l peptone and 20 g/l CaCO₃ pH 6.5, and cultured in a test tube at 30°C for 42 hours. Subsequently, 1 ml of the resulting cultured medium was dispensed. The microbial cells were collected by centrifugation, and then washed with 0.1M potassium phosphate buffer (pH 6.0) or 0.1 M Tris-HCl buffer (pH 8.0) or 0.1 M glycine NaOH buffer (pH 8.8). The washed microbial cells were resuspended in 0.5 ml of each buffer. L-fucitol at a final concentration of 10 g/l was added thereto, and the mixture was reacted at 30°C for 66 hours in the 96-well microplate having 2 ml volume per well. After the reaction, the microbial cells were removed by centrifugation, and the supernatant was subjected to HPLC analysis. The analysis results are shown in FIG. 3. In the figure, NT represents that the experiment was not performed.

As a result, in the cases using any of the bacterial strains, the amount of produced L-fuculose was increased as the reaction pH was elevated, whereas the amount of produced BP was minimized at pH 8.

### Example 6: Attempt to reduce byproduct (BP) in production of L-fuculose from L-fucitol (2); Addition of EDTA

For the purpose of reducing BP which was problematic in the production of L-fuculose from L-fucitol, EDTA was added to the reaction solution, and its effect was examined.

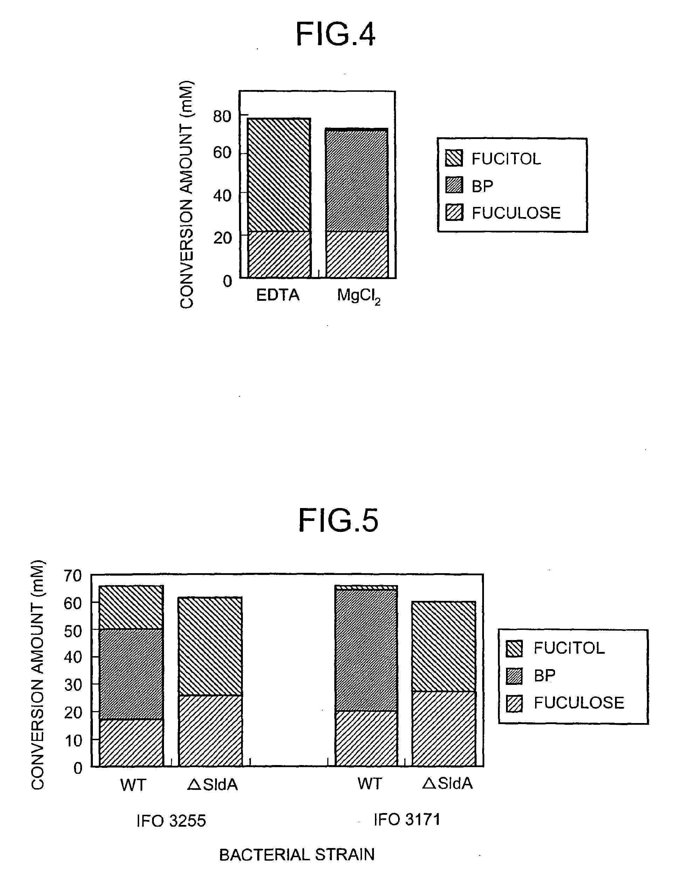

*G. oxydans* IFO 3255 strain was used in the experiments. The cultured liquid was obtained in the same way as in Example 5 (however, CaCO₃ was not added to the cultivation medium), and 1 ml of the cultured liquid was dispensed. The microbial cells were collected by centrifugation and washed with 0.1 M potassium phosphate buffer (pH 6.0). The washed microbial cells were resuspended in 0.5 ml of the same buffer. L-fucitol at a final concentration of 10 g/l and EDTA or MgCl₂ at a final concentration of 10 mM were added thereto, and the mixture was reacted at 30°C for 90 hours in the 96-well microplate having 2 ml volume per well. After the reaction, the microbial cells were removed by centrifugation, and the supernatant was subjected to HPLC analysis. The analysis results are shown in FIG. 4.

As a result, the addition of EDTA did not affect the amount of produced L-fuculose, whereas the addition of EDTA remarkably reduced the amount of produced BP to almost zero.

### Example 7: Attempt to reduce byproduct (BP) in production of L-fuculose from L-fucitol (3); Knockout of sldA gene

It was demonstrated that the methods shown in Examples 5 and 6 are effective for controlling the amount of BP production when L-fuculose is produced using L-fucitol as the raw material.

From the results of Examples 5 and 6, it was demonstrated that it is likely that the enzyme to synthesize L-fuculose from L-fucitol and the enzyme to synthesize BP are independently present. Therefore, it was thought that it would be effective to delete the enzyme which controls the synthesis of BP as the technique for fundamentally solving the BP generation.

Examples of prior art references concerning the conversion of L-fucitol using the acetobacterium strains may include Journal of American Chemical Society 72: 4934-4937 (1950) (Non-patent Document 2) and Canadian Journal of Chemistry 45: 741-744 (1967) (Non-patent Document 3). These references disclose that L-fucitol is oxidized by the acetobacterium strain to consequently produce 1-deoxy-D-glycero-3-hexulose. In the results of our work, L-fucitol and the unidentified substance (BP) were detected as oxides of L-fucitol that have been produced by the acetobacterium strain. Thus it is strongly suggested that the unidentified substance (BP) is likely to be 1-deoxy-D-glycero-3-hexulose described in the prior art references. In the prior art references, the possibility that the enzymes producing these substances are independently present has been discussed. However, their independent existence has not been proved and the enzymes which control the respective reactions have not been identified.

Another prior art reference, Biosci. Biotechnol. Biochem. 65: 2755-2762 (2001), reports that D-arabitol dehydrogenase present on the membrane of the acetobacterium controls the oxidation of many sugar compounds. In this reference, the oxidation of L-fucitol is not described. However, because of its broad substrate specificity, it is considered that the enzyme possibly catalyzes the oxidation of L-fucitol to produce either L-fuculose or BP which seemed to be 1-deoxy-D-glycero-3-hexulose. In the latter case, it was predicted that the production of BP would be fundamentally eliminated by knocking out this D-arabitol dehydrogenase. Based on this hypothesis, an attempt was made for producing a bacterial strain in which the aforementioned D-arabitol dehydrogenase was defected, as described below.

D-Arabitol dehydrogenase present on the cell membrane was also referred to by alternative names such as glycerol dehydrogenase or D-sorbitol dehydrogenase because of the broadness of its substrate specificity. Among the prior art references, for example in JP H8-242850 A (Patent Document 6), this enzyme has been referred to as D-sorbitol dehydrogenase, and its gene has been cloned and its nucleotide sequence has been determined. In accordance with information in this reference, the gene encoding a D-arabitol dehydrogenase catalytic subunit is hereinbelow designated as sldA, and the protein encoded by sldA is hereinbelow designated as SldA. For producing SldA-defected strain, a DNA fragment which lacks an internal partial sequence of sldA was prepared. This fragment was introduced into an acetobacterium, to induce homologous recombination of sldA on chromosomal DNA. For detecting the resulting mutant strain having the homologous recombination, a kanamycin resistant gene Kmr was introduced together with creating the defect upon preparing the fragment having internal defect in sldA. Thus, the mutant strain was easily detected by growing on the medium containing kanamycin.

First, to prepare the fragment containing a homologous recombination region, the fragment containing the internal partial sequence of sldA was obtained by PCR using the primers shown in SEQ ID NOS:7 and 8 (having KpnI and PstI recognition sites, respectively). As the template, genomic DNA prepared from *Gluconobacter oxydans* IFO 3255 strain whose sldA nucleotide sequence had been reported was used. In addition, when genomic DNA prepared from *Gluconobacter oxydans* IFO 3171 strain was used, the fragment having the anticipated length was also amplified. This result would probably be because of high homology of the sequence. With each fragment, analysis by the treatment with restriction enzymes revealed that one recognition site of BamHI and one site of BalII were present. Thus, the sldA partial sequence between these sites was removed, and instead, the Kmr gene was inserted in this site. The Kmr gene was obtained by PCR with the template of plasmid pHSG298 (supplied from Takara Bio Inc.) bearing Kmr using the primers shown in SEQ ID NOS:9 and 10.

The fragment obtained by PCR with the template of the acetobacterium genomic DNA was digested with KpnI and PstI and purified, and then subcloned into pUC18 (supplied from Takara Bio Inc.) that had been digested with KpnI and PstI and purified. *E. coli* JM 109 strain was transformed with this plasmid, and cultured. Subsequently, the plasmid was extracted, purified, and digested with BamHI and BglII and purified. The fragment prepared by PCR with pHSG298 containing Kmr was ligated thereto to yield a novel plasmid. *E. coli* JM109 strain was transformed with this plasmid, and cultured. Subsequently, the plasmid was extracted, purified, further digested with KpnI and PstI, and purified to yield a Kmr gene-inserted sldA partial sequence fragment. Each of the Kmr gene-inserted sldA partial sequence fragments derived from IFO 3255 strain and IFO 3171 strain was introduced into IFO 3255 strain and IFO 3171 strain, respectively, by electroporation (electrode interval: 0.5 mm, 14.0 kV/cm). The strains capable of growing on a kanamycin-containing plate were selected to yield *G. oxydans* IFO 3255 sldA:Kmr strain and *G. oxydans* IFO 3171 sldA:Kmr strain.

Using these mutant strains, *G. oxydans* IFO 3255 sldA:Kmr strain and *G. oxydans* IFO 3171 sldA:Kmr strain, the L-fucitol conversion reaction was performed. 1 mL of the cultured medium obtained in the same way as in Example 6 was dispensed. The microbial cells were collected by centrifugation and washed with 0.1 M glycine NaOH buffer (pH 8.8). The washed microbial cells were resuspended in 0.5 ml of the same buffer. L-fucitol at a final concentration of 10 g/l and MgCl₂ at a final concentration of 10 mM were added thereto, and the mixture was reacted at 30°C for 66 hours in the 96-well microplate having 2 ml volume per well. After the reaction, the microbial cells were removed by centrifugation, and the supernatant was subjected to HPLC analysis. The analysis results are shown in FIG. 5. In FIG. 5, WT and ΔSldA represent the wild type strain and the sldA gene-knockout strain, respectively.

As a result, it was observed that the mutant strains produced L-fuculose but no BP at all. This result demonstrates that the synthesis of BP from L-fucitol is catalyzed by SldA, and that the enzyme to synthesize L-fuculose from L-fucitol is present independently from SldA. It was demonstrated that the use of the SldA-defect strain does not require control of pH and addition of EDTA, and is effective for producing 1-fuculose from L-fucitol without producing the byproduct from L-fucitol.

SEQ ID NO:7: Nucleotide sequence of 5'-primer for obtaining sldA internal partial sequence gccggcggtaccttctagcaacagccgcaggactc

SEQ ID NO:8: Nucleotide sequence of 3'-primer for obtaining sldA internal partial sequence acaccttgtctgcagcatcactacgcagggcgctg

SEQ ID NO:9: Nucleotide sequence of 5'-primer for obtaining Kmr gene
taaaactggatccttacataaacagtaatacaagg

SEQ ID NO:10: Nucleotide sequence of 3'-primer for obtaining Kmr gene
atgctctgccagatctacaaccaattaaccaattc

### Example 8: Search for enzyme activity to synthesize L-fuculose from L-fucitol (1)

Since it was demonstrated in Example 7 that the enzyme other than SldA to synthesize L-fuculose from L-fucitol was present, the enzyme activity to catalyze this activity was searched.

A candidate of the enzyme of interest seemed to be NAD(P)-dependent dehydrogenase because the reaction to synthesize L-fuculose from L-fucitol was the oxidation reaction. As the example of catalysis of the oxidation of L-fucitol by NAD(P)-dependent dehydrogenase, it has been reported that D-arabitol dehydrogenase derived from red algae *Galdieria sulphuraria* catalyzes the oxidation of L-fucitol in an NAD-dependent manner (Planta 202: 487-493 (2003), Non-patent Document 5). However, the activity thereof is weak, and no product has been identified. Thus, it has not been examined whether L-fuculose is formed or the product other than L-fuculose is produced as in the case of SldA.

First, it was examined whether the acetobacterium strain has the activity to oxidize L-fucitol in an NAD(P)-dependent manner. After refreshing sldA:Kmr mutant strain derived from *G. oxydans* on YPG agar medium, one platinum loopful thereof was seeded in the liquid medium containing 10 g/l glycerol, 0.3g/l yeast extract and 0.3 g/l peptone pH 6.5, and cultured in the test tube at 30°C for 24 hours. Subsequently, 1 ml of the resulting cultured medium was added to 50 ml of the same liquid medium, and cultured at 30°C for 18 hours using a 500 ml Sakaguchi flask. The microbial cells were collected by centrifuging the resulting cultured medium, washed with 9 g/l of NaCl, and sonicated with 200 W for 10 minutes to yield the disrupted microbial cells. The microbial cells were further centrifuged at 15,000 g for 15 minutes to yield the supernatant, which was then used as a cell free extract. Using this cell free extract as an enzyme source, L-fucitol and NAD(P) at final concentrations of 10 mM and 1 mM, respectively, were reacted in the presence of 0.1 M glycine NaOH buffer (pH 8.8) or 0.1 M potassium phosphate buffer (pH 6.0) at 30°C, for measuring L-fucitol oxdation activity. The experiment without adding L-fucitol was also performed as the control. The oxidation activity was quantified by the increase of absorbance at 340 nm caused by the production of NAD(P)H. The activity units were calculated defining the activity to oxidize 1 µmol of the substrate in one minute as 1 unit (U). The measurement results are shown in Table 4.

As a result, L-fucitol dehydrogenase using NAD as the coenzyme was detected. This activity was higher at pH 8.8 than at pH 6.0. Although it has not been identified that the product is L-fuculose, the result corresponded to the fact observed in Example 5, i.e., the amount of produced L-fuculose was increased at higher pH. Thus, this activity might be involved in the synthesis of L-fuculose.

**Table 4. NAD(P)-dependent L-fucitol oxidation activity in cell free extract of G. oxydans IFO 3255 sldA:Kmr mutant strain**

| | Coenzyme | Dehydrogenase activity (×10⁻³ U/mg) | | | |
|---|---|---|---|---|---|
| | | NAD | | NADP | |
| SUBSTRATE | pH | 6.0 | 8.8 | 6.0 | 8.8 |
| H₂O | | 0.0 | 0.0 | 0.0 | 0.0 |
| L-FUCITOL | | 3.1 | 18.4 | 0.0 | 1.9 |

### Example 9: L-fucitol conversion reaction using acetobacterium cell free extract

Since the L-fucitol oxidation activity was present in the acetobacterium cell free extract, the L-fucitol conversion reaction using this activity was attempted.

As the enzyme source, the cell free extract obtained in Example 8 was added at 0, 0.8, 1.6 and 4.0 as total protein final concentrations to the reaction solution, and left stand with 10 g/l L-fucitol, 0.1M glycine NaOH (pH 8.8), 0.03 mM FAD, 1 mg/ml BSA and 2 mM NAD at 30°C for 90 hours. Subsequently, the product was subjected to HPLC analysis. In order to identify NAD dependency of the L-fucitol conversion reaction, the group in which NAD had not been added, and the group in which NADH oxidase had been added for the purpose of supplying NAD which would be consumed during the reaction were made as controls. NADH oxidase used was the enzyme to synthesize NAD and hydrogen peroxide from NADH and oxygen (supplied from Nacalai Tesque, derived from *Bacillus liqueniformis*). It was anticipated that NADH produced during the reaction would be reconverted into NAD by addition of this enzyme, and resulting NAD would be reused for the oxidation of L-fucitol. NADH oxidase was added at a final concentration of 0.1 U/ml. Catalase at a final concentration of 10 U/ml was also added for the purpose of consuming produced hydrogen peroxide. The results are shown in Table 5.

The conversion of L-fucitol by the acetobacterium cell free extract was observed in any groups in which NAD had been added depending on the amount of added NAD. However, the conversion was not observed at all in the group in which NAD had not been added. Thus, it was confirmed that this conversion proceeds in a NAD-dependent manner, which supports the result in Example 8. Therefore, it was suggested that the activity which is involved in this conversion was attributed to dehydrogenase that had been identified in Example 8. The products in this conversion were L-fuculose and L-fucose, and BP was not observed at all. Therefore, it was confirmed that the L-fucitol oxidation enzyme present in this cell free extract was an L-fuculose-forming enzyme. A trace amount of L-fucose was observed in this conversion. Thus, it was suggested that the enzyme such as L-fucose isomerase to synthesize L-fucose from L-fuculose or L-fucitol oxidase to directly synthesize L-fucose from L-fucitol was present in the cell free extract. Furthermore, the amount of produced L-fuculose exceeded the amount of added NAD regardless of the addition of NAD oxidase. This result implies that the activity to reconvert NADH that had been produced from NAD by the oxidation reaction into NAD was also present in the cell free extract. This activity appears to be, for example, NADH oxidase activity.

**Table 5. L-fucitol conversion experiments using cell free extract of G. oxydans IFO 3255 sldA:Kmr mutant strain**

| Extract (mg/ml) | NAD (mM) | NADH oxidase, Catalase | L-FUCULOSE (mM) | L-FUCOSE (mM) | BP (mM) | L-FUCITOL (mM) |
|---|---|---|---|---|---|---|
| 0.0 | 2 | + | 0.0 | 0.0 | 0.0 | 61.6 |
| 0.8 | 2 | + | 6.1 | 0.4 | 0.0 | 35.9 |
| 1.6 | 2 | + | 8.0 | 2.2 | 0.0 | 29.4 |
| 4.0 | 2 | + | 13.3 | 2.6 | 0.0 | 21.9 |
| 0.0 | 2 | - | 0.0 | 0.0 | 0.0 | 55.8 |
| 0.8 | 2 | - | 2.8 | 0.0 | 0.0 | 36.2 |
| 1.6 | 2 | - | 6.6 | 1.7 | 0.0 | 29.3 |
| 4.0 | 2 | - | 12.6 | 1.9 | 0.0 | 22.5 |
| 0.8 | 0 | + | 0.0 | 0.0 | 0.0 | 53.0 |

### Example 10: NADH oxidase activity in acetobacterium cell free extract

Since it was suggested in Example 9 that the NADH oxidase activity (NADH oxidation activity) was present in the acetobacterium cell free extract, the detection of this enzyme activity was attempted.

As the enzyme source, the cell free extract obtained in Example 8 was reacted with NADH and FAD at final concentrations of 0.2 mM and ± 0.03 mM, respectively, in the presence of 0.1 M Tris-HCl buffer (pH 8.0) or 0.1 M glycine NaOH buffer (pH 8.8) at 30°C, for measuring the NADH oxidation activity. The group in which the extract had not been added was made as a control. The oxidation activity was quantified by the decrease of absorbance at 340 nm caused by the conversion of NADH to NAD. The activity units were calculated defining the activity to oxidize 1 µmol of NADH in one minute at 30°C as one unit (U). The measurement results are shown in Table 6.

As a result, the NADH oxidation activity was detected in both buffers at pH 8.0 and 8.8. In the measurement at pH 8.0, it was also shown that the addition of FAD facilitated this activity. The activity of many known NADH oxidases has been reported to be FAD-dependent. Thus, it was suggested that the NADH oxidation activity present in the extract used in this experiment might be exerted by similar NADH oxidase.

**Table 6. NADH oxidase activity in cell free extract of G. oxydans IFO 3255 sldA:Kmr mutant strain**

| Extract | pH | FAD (mM) | ACTIVITY (×10⁻³ U/mg) |
|---|---|---|---|
| + | 8.0 | 0 | 3.6 |
| + | 8.0 | 0.03 | 26.3 |
| - | 8.0 | 0.03 | 0.0 |
| + | 8.0 | 0.03 | 22.5 |
| - | 8.0 | 0.03 | 0.0 |

### Example 11: Determination of microbial cell as enzyme source and cultivation conditions for producing enzyme

For isolating and purifying L-fuculose-producing type L-fucitol dehydrogenase (FcDH), the bacterial strain as the enzyme source and its cultivation conditions were studied.

Bacterial strain: *Gluconobacter oxydans* IFO 3255 or IFO 3171 strain
Medium: 10 g/l carbon source, 5 g/l yeast extract, 5 g/l peptone (pH 6.5)
carbon source: glycerol, D-mannitol, D-arabitol, xylitol
Cultivation temperature: 30°C
Cultivation time period: 20 hours or 66 hours

### Methods:

The stored bacterial strain was refreshed by culturing on YPG medium (3 g/l peptone, 3 g/l yeast extract, 1 g/l glycerol, pH 6.5) containing 20 g/l agar at 30°C for two nights. These refreshed microbial cells were inoculated to the test tube containing 3 ml each of autoclaved medium, and cultured with shaking at 30°C for 24 or 66 hours. After the cultivation, the microbial cells were collected by centrifuging 3 ml of the cultured liquid, washed with 25 mM Tris-HCl buffer (pH 8.0), and resuspended in 0.3 ml of the same buffer. This was subjected to the sonication to disrupt the microbial cells, and then centrifuged at 200,000 g for 30 minutes to yield the supernatant as a sample for measuring the enzyme activity.

The enzyme activity of FcDH was measured by appropriately adding the enzyme solution to the standard condition of 0.2 M Glycine NaOH (pH 9.5), 1 mM MgCl₂, 10 mM L-fucitol and 1 mM NAD and reacting the mixture at 30°C. The activity was quantified by monitoring the change of absorbance at 340 nm to detect the production of NADH from NAD caused by the oxidation. The measurement was started at the addition of NAD. The change of absorbance in the first one minute was taken as an initial rate, and the activity was calculated therefrom. The group in which L-fucitol had not been added was also made as a blank value. As to the FcDH activity, the activity to oxidize 1 µmol of L-fucitol in one minute at 30°C was defined as one unit (U). Calculation was performed with the absorbance coefficient of NADH e₃₄₀ = 0.63 mM⁻¹cm⁻¹. The measurement was principally performed consecutively three times. Their mean value was taken as the measurement results and shown in the figure with their standard deviation.

Results: The measurement results are shown in Table 7. Using both the FcDH specific activity per unit protein weight and the activity quantity obtained from the unit amount of the cultured liquid as indicators, *G. oxydans* IFO 3255 was employed as the FcDH-producing bacterial strain; the medium (10 g/l D-mannitol, 5 g/l yeast extract, 5 g/l peptone (pH 6.5)) was employed as its cultivation condition; and 24 hours was employed as the cultivation time period.

**Table 7**

| BACTERIAL STRAIN | CARBON SOURCE | CULTIVATION TIME PERIOD (h) | PROTEIN (mg/ml) | FcDH SPECIFIC ACTIVITY | |
|---|---|---|---|---|---|
| | | | | (mU/ml) | (mU/ml) |
| *G. oxydans* IFO 3255 | GLYCEROL | 24 | 7.8 | 6.1 | 46.9 |
| *G. oxydans* IFO 3255 | GLYCEROL | 66 | 6.6 | 4.2 | 28.0 |
| *G. oxydans* IFO 3255 | D-MANNITOL | 24 | 4.1 | 18.7 | 77.5 |
| *G. oxydans* IFO 3255 | D-MANNITOL | 66 | 2.8 | 16.7 | 46.6 |
| *G. oxydans* IFO 3255 | D-MANNITOL | 24 | 5.1 | 17.6 | 89.6 |
| *G. oxydans* IFO 3255 | XYLITOL | 66 | 5.0 | 10.5 | 52.4 |
| *G. oxydans* IFO 3255 | XYLITOL | 24 | 7.2 | 13.9 | 100.2 |
| *G. oxydans* IFO 3255 | GLYCEROL | 66 | 3.8 | 9.4 | 35.9 |
| *G. oxydans* IFO 3171 | GLYCEROL | 24 | 5.4 | 5.7 | 31.1 |
| *G. oxydans* IFO 3171 | D-MANNITOL | 66 | 5.0 | 0.4 | 2.0 |
| *G. oxydans* IFO 3171 | D-MANNITOL | 24 | 7.6 | 2.7 | 20.4 |
| *G. oxydans* IFO 3171 | D-MANNITOL | 66 | 7.5 | 1.9 | 14.0 |
| *G. oxydans* IFO 3171 | D-ARABITOL | 24 | 9.2 | 1.8 | 16.0 |
| *G. oxydans* IFO 3171 | XYLITOL | 66 | 6.6 | 0.9 | 5.8 |
| *G. oxydans* IFO 3171 | XYLITOL | 24 | 7.3 | 4.7 | 34.3 |
| *G. oxydans* IFO 3171 | XYLITOL | 66 | 8.4 | 0.5 | 4.2 |

### Example 12: Purification of FcDH

The stored *G. oxydans* IFO 3255 strain was refreshed by culturing in the YPG agar medium at 30°C for two nights. These refreshed microbial cells were inoculated to the test tube containing 3 ml each of autoclaved medium, and cultured with shaking at 30°C for 18 hours. This was seeded at 1% (v/v) in the 500 ml Sakaguchi flask containing 100 ml of the same autoclaved medium, and cultured at 30°C for 24 hours. After the cultivation, the microbial cells were collected by centrifugation, washed with 25 mM Tris-HCl buffer (pH 8.0), and suspended in the same buffer at one 25th volume of the cultured medium. This was subjected to the sonication to disrupt the microbial cells. Subsequently, the supernatant (crude extract) was obtained by centrifuging at 200,000 g for 30 minutes. From approximately 3 1L of the cultured medium, 934 mg of the protein was yielded in the crude extract.

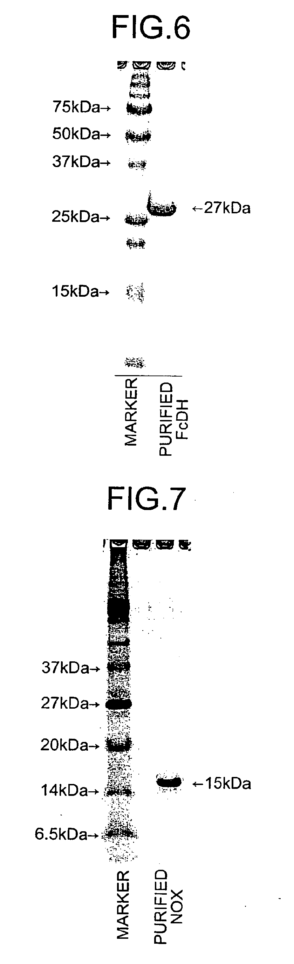

Subsequently, a fraction of this crude extract was obtained by fractionation with 30% to 60% saturated ammonium sulfate. The fraction was dialyzed against the buffer 1 (50 mM Tris-HCl, pH 8.0, 1.2 M (NH₄)₂SO₄, 1 mM MnCl₂) overnight. The sample thus obtained was applied onto Phenyl Sepharose HP 26/10 (Amersham Biosciences) that had been equilibrated with the buffer 1. The protein absorbed in the carrier was eluted by decreasing the (NH₄)₂SO₄ concentration in the buffer from 1.2 M to 0 M. In this operation, the FcDH activity was detected in the fraction having the (NH₄)₂SO₄ concentration in the vicinity of about 0.2 M. The fraction containing the FcDH activity was collected, concentrated, dialyzed against 50 mM potassium phosphate buffer (pH 6.0), and applied onto Q-Sepharose 16/10 (Amersham Biosciences) that had been equilibrated with the same buffer. The protein absorbed in the carrier was eluted by increasing the NaCl concentration in the buffer from 0 M to 0.5 M. In this operation, the FcDH activity was detected in the fraction having the NaCl concentration in the vicinity of about 0.4 M. The fraction containing the FcDH activity was collected, concentrated, and then applied onto Superdex 200 16/60 (Amersham Biosciences) that had been equilibrated with 50 mM potassium phosphate buffer (pH 6.0). As a result of this operation, FcDH was eluted at an elution position at which the molecular weight was estimated as 102 kDa. The fraction containing the FcDH activity was collected, and concentrated. Its purity was then examined on SDS-PAGE. As a result, FcDH was observed as a single band whose molecular weight was estimated as about 27 kDa (FIG. 6). By this series of operation of the purification, the specific activity of FcDH was increased 520 times from 0.015 U/mg in the crude extract to 7.8 U/mg. The amount of purified FcDH thus obtained was 0.60 mg, and the recovery rate of the activity was 34% (Table 8).

**Table 8**

| STEP | PROTEIN (mg/ml) | ACTIVITY (U) | RECOVERY RATE (%) | SPECIFIC ACTIVITY (U/mg) | PURIFICATION (-fold) |
|---|---|---|---|---|---|
| CRUDE EXTRACT | 934 | 14 | 100 | 0.015 | 1.0 |
| AMMONIUM SULFATE DIALYSIS | 535 | 11 | 79 | 0.020 | 1.4 |
| CHROMATOGRAPHY (Phenyl Sepharose) | 64 | 7.1 | 51 | 0.11 | 7.4 |
| CHROMATOGRAPHY (Q-Sepharose) | 1.1 | 7.0 | 50 | 6.4 | 427 |
| CHROMATOGRAPHY (Superdex) | 0.60 | 4.7 | 34 | 70 | 520 |

### Example 13: Purification of NOX

The stored *G. oxydans* IFO 3255 strain was refreshed by culturing in the YPG agar medium at 30°C for two nights. These refreshed microbial cells were inoculated to the test tube containing 3 ml each of autoclaved medium, and cultured with shaking at 30°C for 18 hours. This was seeded at 1% (v/v) in the 500 ml Sakaguchi flask containing 100 ml of the same autoclaved medium, and cultured at 30°C for 24 hours. After the cultivation, the microbial cells were collected by centrifugation, washed with 25 mM Tris-HCl (pH 8.0), and suspended in the same buffer at one 25th volume of the cultured medium. This was subjected to the sonication to disrupt the microbial cells. Subsequently, the supernatant (crude extract) was obtained by centrifuging at 200,000 g for 30 minutes. From approximately 4L of the cultured medium, 1253 mg of the protein was yielded in the crude extract.

Subsequently, a fraction of this crude extract was obtained by fractionation with 30% to 60% saturated ammonium sulfate. The fraction was dialyzed against the buffer 2 (50 mM potassium phosphate, pH 6.0, 1 mM DTT, 0.5 mM EDTA) overnight. The sample thus obtained was applied onto Q-Sepharose 26/10 that had been equilibrated with the buffer 2. The protein absorbed in the carrier was eluted by increasing the NaCl concentration in the buffer from 0 M to 0.5 M. In this operation, the NOX activity was detected in the fraction having the NaCl concentration in the vicinity of about 0.25 M. The fraction containing the NOX activity was collected, concentrated, dialyzed against the buffer 3 (50 mM Tris-HCl, pH 8.0, 0.6 M (NH₄)₂SO₄), and applied onto Phenyl Sepharose 16/10 that had been equilibrated with the same buffer. The protein absorbed in the carrier was eluted by decreasing the (NH₄)₂SO₄ concentration in the buffer from 0.6 M to 0 M. In this operation, the NOX activity was detected in the fraction having the (NH₄)₂SO₄ (NH₄)₂SO₄ concentration in the vicinity of about 0.2 M. The fraction containing NOX activity was collected, concentrated, dialyzed against 50 mM Tris-HCl (pH 8.0), then concentrated, and subsequently applied onto 1.5 ml FAD-agarose resin (Sigma) that had been equilibrated with the same buffer. The unabsorbed proteins were thoroughly washed with the same buffer. The absorbed protein was then eluted with the buffer containing 50 mM Tris-HCl and 1 mM FAD. NOX was absorbed specifically to the resin, and subsequently eluted by the addition of the buffer containing FAD. This eluted fraction was collected, and concentrated. Its purity was then examined on SDS-PAGE. As a result, NOX was observed as a single band whose molecular weight was estimated as about 15 kDa (FIG. 7).

By a series of the aforementioned operation of the purification, the specific activity of NOX was increased 6763 times from 0.029 U/mg in the crude extract to 196 U/mg. The amount of purified NOX thus obtained was 0.014 mg, and the recovery rate was 7.3% (Table 9).

**Table 9**

| STEP | PROTEIN (mg/ml) | ACTIVITY (U) | RECOVERY RATE (%) | SPECIFIC ACTIVITY (U/mg) | PURIFICATION (-fold) |
|---|---|---|---|---|---|
| CRUDE EXTRACT | 1253 | 14 | 100 | 0.029 | 1.0 |
| AMMONIUM SULFATE DIALYSIS | 486 | 11 | 54 | 0.041 | 1.4 |
| CHROMATOGRAPHY (Phenyl Sepharose) | 87 | 7.1 | 26 | 0.11 | 3.8 |
| CHROMATOGRAPHY (Q-Sepharose) | 4.2 | 7.0 | 8.9 | 0.79 | 27 |
| CHROMATOGRAPHY (FAD agarose) | 0.014 | 4.7 | 7.3 | 196 | 6763 |

The purified NOX solution was applied onto Superdex 200 16/60 that had been equilibrated with 50 mM Tris-HCl (pH 8.0). Consequently, NOX was eluted at the elution position at which molecular weight was estimated as 34 kDa. In the subsequent experiments, the sample obtained by purifying NOX as the single band on SDS-PAGE and eluting through Superdex 200 16/60 was used as a purified enzyme preparation.

The NOX activity was measured in accordance with the following standard procedure. The enzyme was appropriately added to 0.1 M Tris-HCl (pH 8.0), 30 µM FAD, 5 mM EDTA, 2 mM DTT, 0.2 mM NADH, and reacted at 30°C. The activity was quantified by monitoring reduction of absorbance at 340 nm caused by the production of NAD from NADH. The measurement was started at the addition of NADH. The change of absorbance in the first one minute was taken as an initial rate, and the activity was calculated therefrom. The experimental group in which the enzyme source had not been added was als made as a blank. As to the NOX activity, the activity to oxidize 1 µmol NADH in one minute at 30°C was defined as one unit (U). The measurement was principally performed consecutively three times. Their mean value was taken as the measurement results and shown in the figure with their standard deviation.

### Example 14: Various natures of FcDH

Using the FcDH preparation purified in Example 12 as the enzyme source, its various natures were examined.

### 4-1) Optimal reaction pH

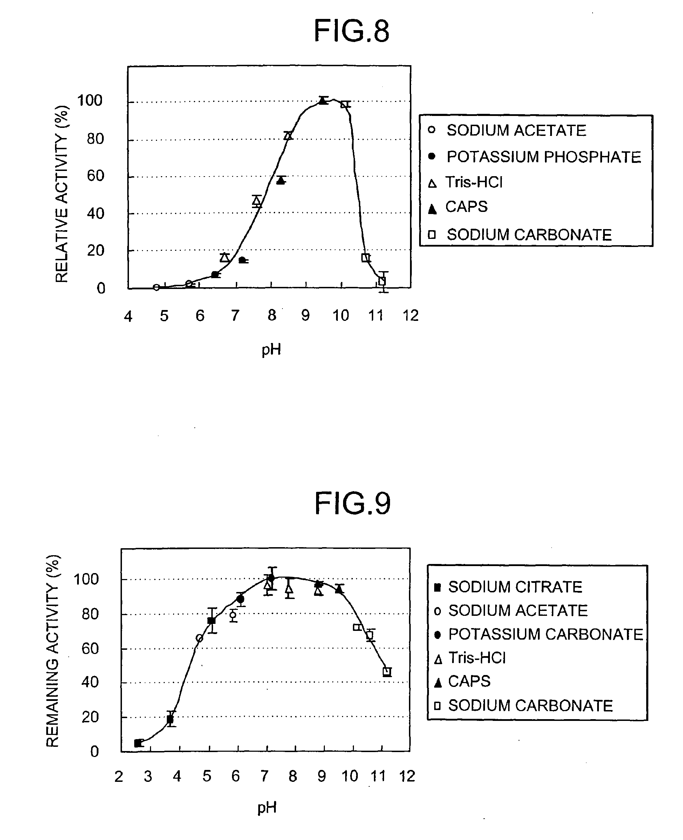

The reaction was performed in the solution containing 0.1 M pH buffer, 1 mM MgCl₂, 10 mM L-fucitol, 1 mM NAD and 1.1 µg/ml purified FcDH at 30°C, and the production of NADH was estimated from the measurement value of A₃₄₀. As pH buffers, sodium acetate buffer (pH 4.8, 5.7), potassium phosphate buffer (pH 6.4, 7.2), Tris-HCl buffer (pH 6.7, 7.6, 8.5), CAPS-NaOH buffer (Sodium cyclohexylaminopropanesulfonate) (pH 8.3, 9.5) and sodium carbonate buffer (pH 10.1, 10.7, 11.2) were used. The specific activity of FcDH measured at each pH was shown in FIG. 8 as the percentage relative to the specific activity at pH 9.5 at which the maximum specific activity was obtained.

### 4-2) pH stability

The solution containing 0.1 M pH buffer and 80 µg/ml purified FcDH was left stand on ice for 30 minutes, and subsequently the solution was diluted to a final FcDH concentration of 1.6 µg/ml. The reaction was performed under the standard condition, and the remaining FcDH activity was measured. As the pH buffers, sodium citrate buffer (pH 2.6, 3.7, 5.1), sodium acetate buffer (pH 4.7, 5.8), potassium phosphate buffer (pH 6.1, 7.2), Tris-HCl buffer (pH 7.0, 7.8, 8.8), CAPS-NaOH buffer (pH 8.8, 9.5) and sodium carbonate buffer (pH 10.1, 10.6, 11.2) were used. The remaining activity of FcDH measured at each pH was shown in FIG. 9 as the percentage relative to the remaining activity at pH 7.2 at which the maximum stability was observed. As a result, 80% or more remaining activity was observed at pH of about 5 to 10.

### 4-3) Optimal reaction temperature

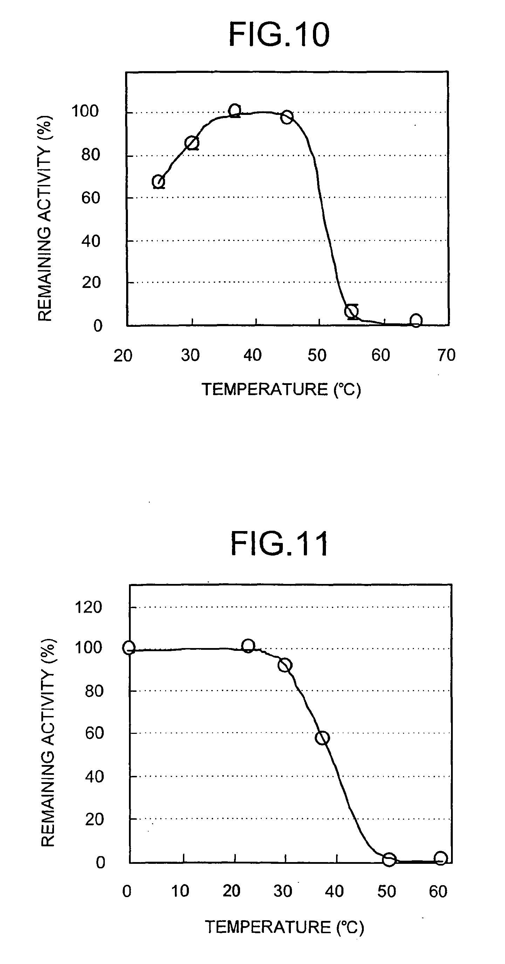

The solution containing Gly-NaOH (pH 9.5), MgCl₂, fucitol and purified FcDH was preincubated at each temperature (25, 30, 37, 45, 55 or 65°C) for 3 minutes, and then NAD that had separately been preincubated at each temperature was added thereto to start the reaction. The final concentrations in the reaction solution were adjusted to the standard condition for the activity measurement, and the final concentration of FcDH was 1.6 µg/ml. The specific activity of FcDH measured at each temperature was shown in FIG. 10 as the percentage relative to the specific activity at 37°C at which the maximum specific activity was observed.

### 4-4) Temperature stability

The solution containing Gly-NaOH (pH 9.5) and 80 µg/ml purified FcDH was left stand at 0, 25, 30, 37, 50 or 60°C for 30 minutes. The solution was then diluted so that the final concentration of FcDH was 1.6 µg/ml. The reaction was performed under the standard condition, and the remaining FcDH activity was measured. The remaining activity of FcDH measured at each temperature was shown in FIG. 11 as the percentage relative to the remaining activity when left stand at 0°C . Up to 30°C, 80% or more remaining activity was observed.

### 4-5) Substrate specificity

Reactivity of FcDH to various sugar alcohol including L-fucitol was measured. Values of k_{cat} and Kₘ for each substrate were obtained by measuring the reaction initial rate under various substrate concentrations and drawing Lineweaver-Burk plot of the results. The results are shown in Table 10. As a result, FcDH had the oxidation activity on various sugar alcohols including L-fucitol, and the k_{cat} and Kₘ values were large in the order of D-arabitol> L-fucitol> xylitol> D-sorbitol> D-mannitol> ribitol> dulcitol> glycerol.

**Table 10**

| | Kcat (U·mg-1) | Km (mM) | kcat/Km (U·mg⁻¹·mM⁻¹) |
|---|---|---|---|
| D-ARABITOL | 32.9 | 1.3 | 25.19 |
| L-FUCITOL | 33.8 | 31.9 | 1.06 |
| XYLITOL | 9.3 | 31.9 | 0.29 |
| D-SORBITOL | 4.2 | 22.4 | 0.19 |
| D-MANNITOL | 4.7 | 30.2 | 0.16 |
| RIBITOL | 2.9 | 20.4 | 0.14 |
| DULCITOL | 3.1 | 30.5 | 0.10 |
| GLYCEROL | 0.9 | 10.1 | 0.09 |

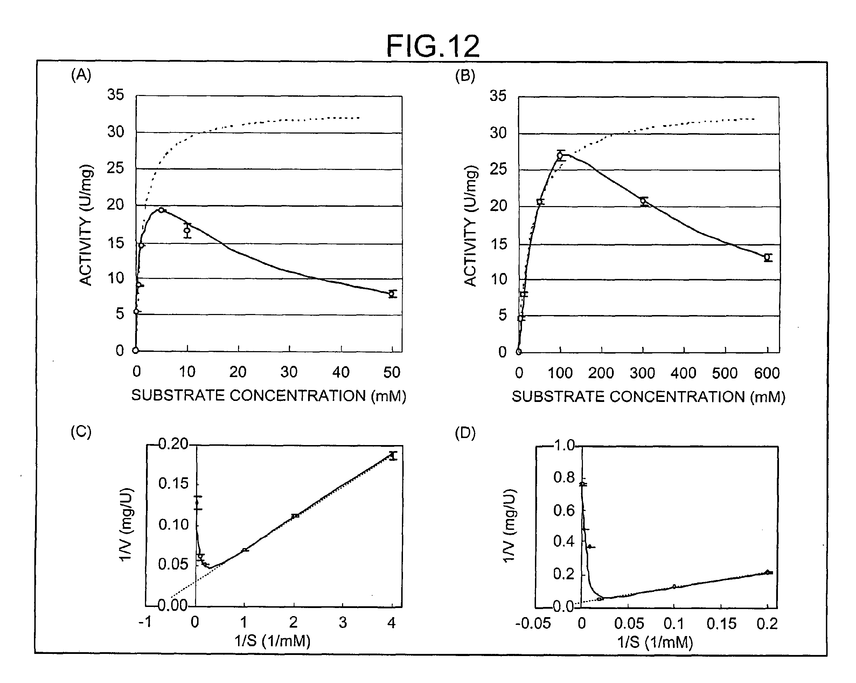

The results of measuring the specific activity of the purified FcDH are shown in FIG. 12. For D-arabitol and L-fucitol, reaction inhibition by the substrate was observed at high concentration of the substrate. The reduction of the specific activity was observed at 5 mM or more of D-arabitol (A) and at 100 mM or more of L-fucitol (B). Lineweaver-Burk plots of the results in (A) and (B) were shown in FIG. 12 (C) and (D), respectively. The Kₘ and k_{cat} values were calculated from the range in which the substrate inhibition was not observed, i.e., from a straight line represented by a broken line in FIG. 12 (C) and (D). Thus, in the cases of these substrates, the k_{cat} and Kₘ values were calculated within the range of substrate concentrations at which no inhibitory effect was observed. For other sugar alcohols, no inhibitory effect by the substrate was observed up to the substrate concentration of 100 mM.

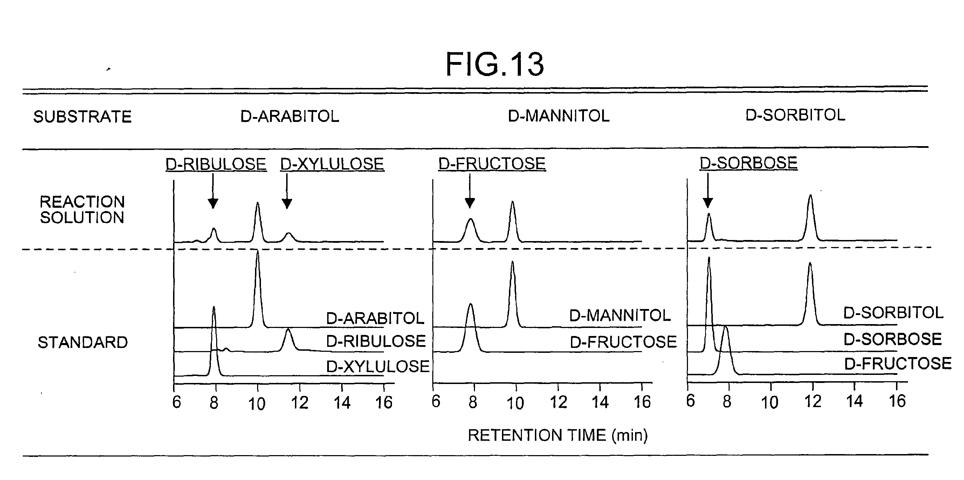

Among the substrates recognized by FcDH, the oxidation product of L-fucitol is L-fuculose, and the oxidation products of the other substrates were examined. The substrates used were D-arabitol, D-mannitol and D-sorbitol. The reaction solution containing 0.5 g/dl of the substrate, 50 mM Gly-NaOH (pH 9.5), 1 mM NAD, 30 mM FAD, 5 mM MgCl₂, 0.6 U/ml purified FcDH, 0.2 U/ml NOX and 0.1 mg/ml catalase was reacted at 30°C for 16 hours, and analyzed by HPLC (FIG. 13). As a result, the products which seemed to be D-ribulose and D-xylulose from D-arabitol were identified, the product which seemed to be D-fructose from D-mannitol was identified, and the product which seemed to be L-sorbose from D-sorbitol was identified.

### Bivalent metal ion specificity

Since it was found that the induction of FcDH activity required the addition of bivalent ion, availability of various bivalent ions by FcDH was measured. The solution containing Gly-NaOH (pH 9.5), purified FcDH and various bivalent metal ions (or EDTA) was left stand on ice for 30 minutes, and then L-fucitol and NAD were added thereto to start the reaction. In the reaction solution, the final concentrations of the bivalent metal ion or EDTA were adjusted to be 5 mM and the concentration of FcDH was adjusted to be 1.6 µg/ml. The others were adjusted to the standard condition. Each specific activity measured is shown in Table 11 as the percentage relative to the specific activity when MnCl₂ was added, where the maximum specific activity was observed. As a result, the activation effect on FcDH was almost equally observed for Mn²⁺, Mg²⁺ and Ca²⁺ ions. However, Ni²⁺ and Co²⁺ ions had no effect. Conversely, Zn²⁺ and Cu²⁺ ions had the inhibitory effect. When the bivalent metal ion was chelated by EDTA, the activity of FcDH nearly disappeared.

**Table 11**

| ADDED REAGENT | RELATIVE |
|---|---|
| none | 35.1±0.5 |
| MnCl₂ | 100.0±6.9 |
| MgCl₂ | 93.7±2.9 |
| CaCl₂ | 89.9±3.4 |
| NiCl₂ | 42.4±0.7 |
| CoCl₂ | 34.8±1.1 |
| ZnSO₄ | 11.7±0.5 |
| CuCl₂ | 5.0±0.5 |
| EDTA | 3.2±0.7 |

### 4-7) NAD and NADP specificity

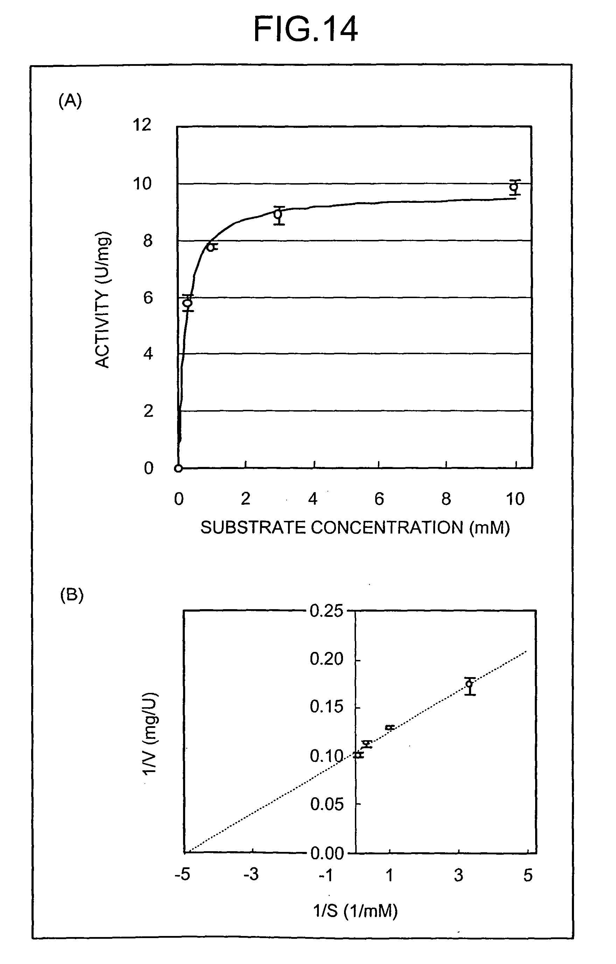

since it was found that FcDH used NAD as the coenzyme, the availability of NAD and NADP was measured. The activity was measured by adding NAD or NADP at various concentrations under the standard condition. The Kₘ value for each coenzyme was calculated by drawing Lineweaver-Burk plot of the results (FIG. 14 (B)). As a result, as shown in FIG. 14, the Kₘ value for NAD was estimated as 0.20 mM. Meanwhile, when NADP was used as the coenzyme, no significant activity was observed (data not shown).

### Example 15: Various natures of NOX

Using the NOX preparation purified in Example 13 as the enzyme source, its various natures were examined.

### 15-1) Optimal reaction pH

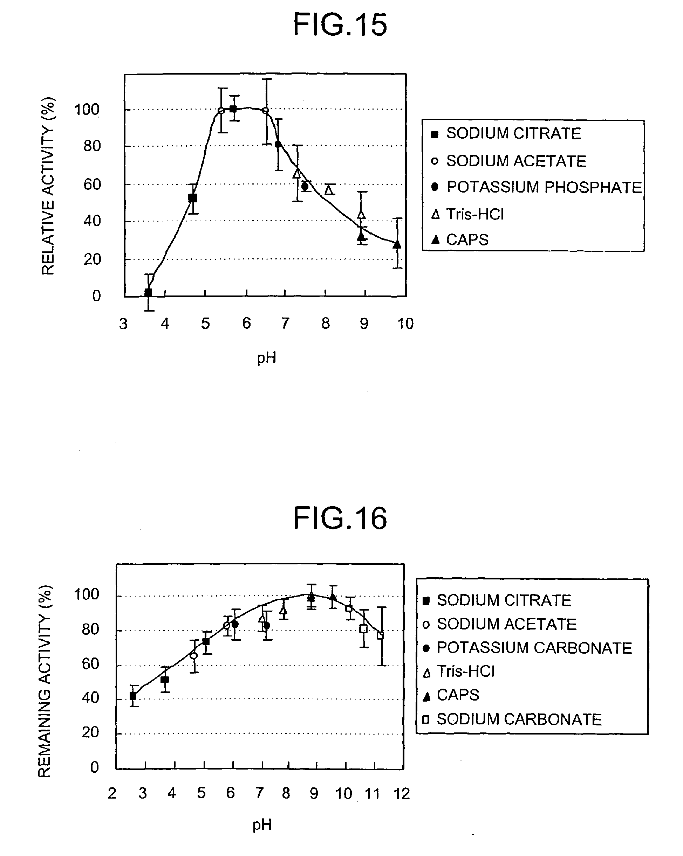

The reaction was performed in the solution containing 0.1 M pH buffer, 30 µM FAD, 5 mM EDTA, 2 mM DTT, 0.2 mM NADH and 0.055 µg/ml purified NOX at 30°C, and the reduction of NADH was estimated from the measurement value of A₃₄₀. As pH buffers, sodium citrate buffer (pH 3.6, 4.7, 5.7), sodium acetate buffer (pH 4.8, 5.7), potassium phosphate buffer (pH 6.4, 7.2), Tris-HCl buffer (pH 6.7, 7.6, 8.5), and CAPS-NaOH buffer (pH 8.3, 9.5) were used. The specific activity of NOX measured at each pH was shown in FIG. 15 as the percentage relative to the specific activity at pH 5.7 at which the maximum specific activity was obtained.

### pH stability

The solution containing 0.1 M pH buffer, 30 µM FAD, 5 mM EDTA, 2 mM DTT, and 100 µg/ml purified NOX was left stand on ice for 30 minutes, and subsequently the solution was diluted so that the final concentration of FcDH was 0.055 µg/ml. The reaction was performed under the standard condition, and the remaining NOX activity was measured. As the pH buffers, sodium citrate buffer (pH 2.6, 3.7, 5.1), sodium acetate buffer (pH 4.7, 5.8), potassium phosphate buffer (pH 6.1, 7.2), Tris-HCl buffer (pH 7.0, 7.8, 8.8), CAPS-NaOH buffer (pH 8.8, 9.5) and sodium carbonate buffer (pH 10.1, 10.6, 11.2) were used. The remaining activity of NOX measured at each pH was shown in FIG. 16 as the percentage relative to the remaining activity at pH 8.8 at which the maximum stability was observed. As a result, 80% or more remaining activity was observed at pH of about 6 to 11.

### 15-3) Optimal reaction temperature

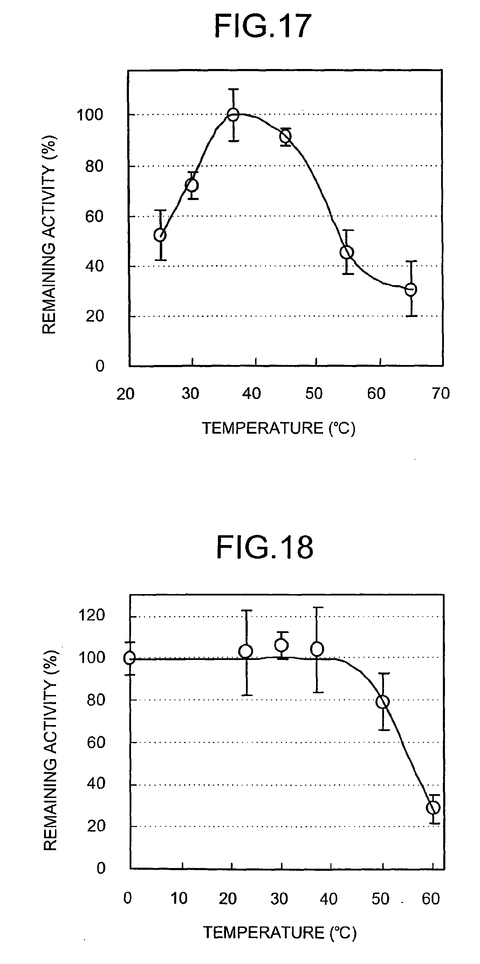

The solution containing Tris-HCl (pH 8.0), FAD, EDTA, DTT and purified NOX was preincubated at each temperature (25, 30, 37, 45, 55 or 65°C) for 3 minutes, and then NADH that had separately been preincubated at each temperature was added thereto to start the reaction. The final concentrations in the reaction solution were adjusted to the standard condition of the activity measurement, and the final concentration of NOX was 0.055 µg/ml. The specific activity of NOX measured at each temperature was shown in FIG. 17 as the percentage (remaining activity) relative to the specific activity at 37°C at which the maximum specific activity was observed.

### 15-4) Temperature stability

The solution containing 0.1 M Tris-HCl (pH 8.0), 30 µM FAD, 5 mM EDTA, 2 mM DTT, and 100 µg/ml purified NOX was left stand at 0, 25, 30, 37, 50 or 60°C for 30 minutes. The solution was then diluted so that the final concentration of NOX was 0.055 µg/ml. The reaction was performed under the standard condition, and the remaining NOX activity was measured. The remaining activity of NOX measured at each temperature was shown in FIG. 18 as the percentage relative to the remaining activity when left stand at 0°C. Up to 50°C, 80% or more remaining activity was observed.

### 15-5) Flavin coenzyme specificity

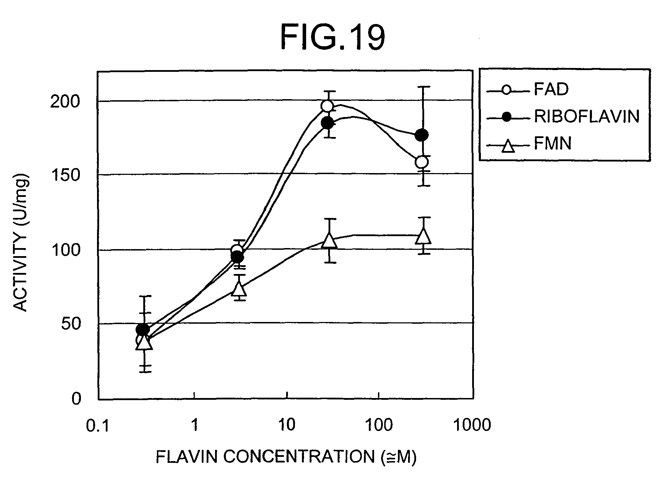

Since it was found that the induction of NOX activity required the addition of flavin coenzyme, the availability of various flavin coenzyme by NOX was measured. The solution containing Tris-HCl (pH 8.0), FAD, EDTA, DTT, purified NOX and any of various flavin coenzymes was left stand on ice for 30 minutes, and then NADH was added thereto to start the reaction. The concentration of flavin in the reaction solution was 0 to 300 µM, and the others were adjusted to the standard condition. The results are shown in FIG. 19. As a result, the NOX activation effect was observed for any of FAD, riboflavin and FMN used, and the effect was almost equal in FAD and riboflavin and relatively weaker in FMN.

### 15-6) NADH and NADPH specificity

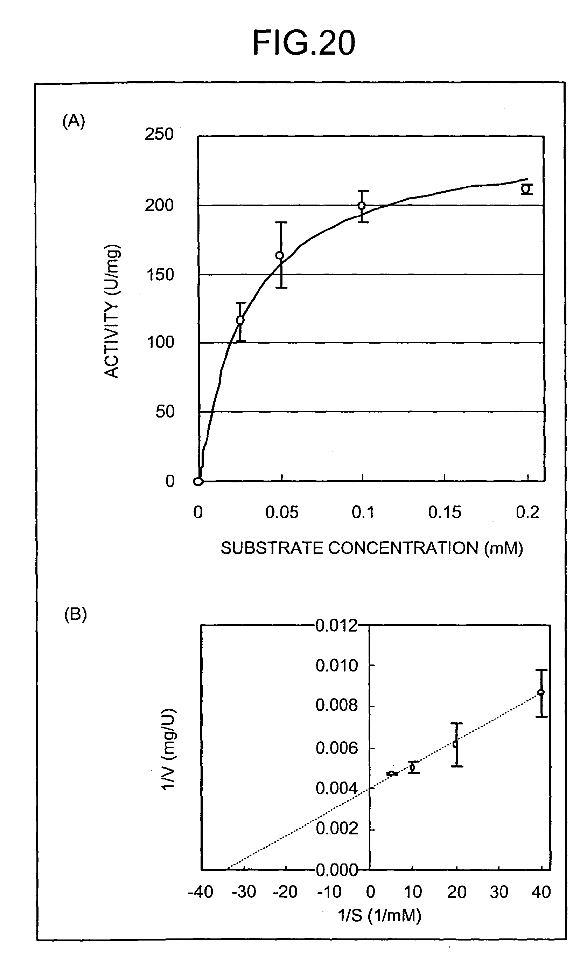

Since it was found that NOX had NADH oxidation activity, the substrate specificity for NADH and NADPH was measured. The Kₘ value for the substrate was calculated by measuring the activity with a variety of the substrate concentrations and drawing Lineweaver-Burk plot of the results. As a result, the Kₘ value for NADH was estimated as 29 µM (FIG. 20). Meanwhile, when NADPH was used as the substrate, no significant activity was observed (data not shown) .

### Example 16: Determination of partial amino acid sequences of FcDH and NOX

Using the purified FcDH and NOX as the materials, their partial amino acid sequences were determined. Both enzymes were subjected to SDS-PAGE, a part of the gel where the band was observed was cut out, and the protein contained therein was used as the sample. In accordance with the standard methods, the protein was fragmented by treatment with trypsin, and separated by reverse phase HPLC to yield fractions. The resulting several fractions were subjected to the protein sequencer. The resulting sequences are shown in SEQ ID NOS:11 to 14.

### Example 17: Cloning of genes encoding FcDH and NOX

In order to clone the gene encoding FcDH or NOX, DNA mix primers corresponding thereto were synthesized from the peptide sequence obtained in Example 16. The PCR was performed using genomic DNA prepared from *G. oxydans* IFO 3255 as the template in accordance with the standard method. The resulting fragment was purified and its nucleotide sequence was analyzed by the DNA sequencer. As a result, the DNA sequences encoding the peptide sequences obtained in Example 16 were found in the resulting PCR products.

Subsequently, Southern hybridization and colony hybridization were performed using this DNA fragment as the probe in accordance with the standard methods to yield positive clones. The plasmids were prepared from the yielded clones, and their DNA sequences were analyzed to obtain a FcDH-encoding gene (fcdh) consisting of 774 bases (including the termination codon) shown in SEQ ID NO:15 and a NOX-encoding gene (nox) consisting of 474 bases (including the termination codon) shown in SEQ ID NO:17. The entire amino acid sequence of FcDH consisting of 257 amino acid residues shown in SEQ ID NO:16 and the entire amino acid sequence of NOX consisting of 157 amino acid residues shown in SEQ ID NO:18 were determined from these nucleotide sequences. These included the peptide sequences determined in Example 6. The molecular weights estimated from the amino acid sequences were 27,522 Da for FcDH and 16,777 Da for NOX, which were almost identical to the molecular weights estimated from SDS-PAGE.

### Example 18: Preparation of strain expressing FcDH, FcDH+NOX or FcDH+NOX+FucI and cultivation thereof

For the expression of FcDH, the PCR was performed with the genomic DNA from *G. oxydans* IFO 3255 strain as the template and the synthetic primers shown in SEQ ID NOS:19 and 20, to yield a fragment of 858 bp including the fcdh gene. The resulting PCR product was digested with EcoRI and PstI and inserted into the corresponding position in the plasmid pUC18 (Takara Bio Inc.) to prepare the plasmid pFEX3052 for the expression of FcDH.

Subsequently in order to obtain the nox gene, the PCR was performed with the genomic DNA from *G. oxydans* IFO 3255 strain as the template and the synthetic primers shown in SEQ ID NOS:21 and 22, to yield a fragment of 605 bp including the nox gene. The resulting PCR product was digested with PstI and HindIII, and inserted into the corresponding position in the plasmid pFEX3052 to prepare the plasmid pFNEX4105 for the co-expression of FcDH and NOX.

The PCR was further performed with this plasmid pFNEX4105 as the template and the synthetic primers shown in SEQ ID NOS: 19 and 23, to yield a fragment of 1390 bp including a gene fragment in which the fcdh gene and the nox gene had been ligated in a tandem manner. The resulting PCR product was digested with EcoRI and KpnI, and subsequently inserted into the corresponding position in the plasmid pUC18 to prepare pFNEX4502.

Subsequently, in order to obtain the fucI gene encoding FucI, the PCR was performed with the genomic DNA obtained from *E. coli* W3110 strain by the standard method and the primers shown in SEQ ID NOS:24 and 25 synthesized based on the fucI gene sequence described in Accession No. NC_000913, to yield a fragment of 1873 bp including the fuel gene. The resulting PCR product was digested with KpnI and SalI, and subsequently inserted into the corresponding position in the plasmid pFNEX4502 to prepare the plasmid pFNIEX5706 for the co-expression of FcDH, NOX and FucI. Because of need to provide in the subsequent experiments, the plasmid pIEX11 for the expression of FucI alone was prepared. This was prepared by performing the PCR similar to the above using the synthetic primers shown in SEQ ID NOS:26 and 27 to yield the fucI gene, digesting the resulting product with EcoRI and PstI, and inserting it into the corresponding position in the plasmid pUC18.

*Escherichia coli* JM 109 strain (supplied from Takara Bio Inc.) was transformed with the constructed plasmid pFEX3052, pFNEX4105, pFNIEX570 or pIEX11 to produce FcDH-expressing strain *E. coli*/pFEX3052, FcDH and NOX co-expressing strain *E. coli*/pFNEX4105, FcDH, NOX and FucI co-expressing strain *E. coli*/pFNIEX5706 and FucI-expressing strain *E. coli*/pIEX11.

The expression strains thus obtained and *E. coli*/pUC18 transformed with pUC18 containing no inserted gene as a control were refreshed by culturing on LB/Amp plates at 37°C. LB/Amp liquid medium was used for the liquid cultivation, and the cultivation was performed at 37°C. After inoculating the refreshed microbial cells, IPTG at a final concentration of 1 mM was added thereto. The microbial cells were cultured for 6 to 18 hours and then collected by centrifugation. The collected microbial cells were washed with 25 mM Tris-HCl buffer (pH 8.0) and used in the subsequent experiments. TB medium (12 g/l trypton, 24 g/l yeast extract, 4 g/l glycerol, 2.3 g/l KH₂PO₄, 12.5 g/l K₂HPO₄) was used instead of the LB medium in some cases, and they were not essentially different except that the larger amount of the microbial cells was obtained.

### Example 19: Preparation of cell free extract and purification of expressed protein

### (19-1) Preparation of cell free extract

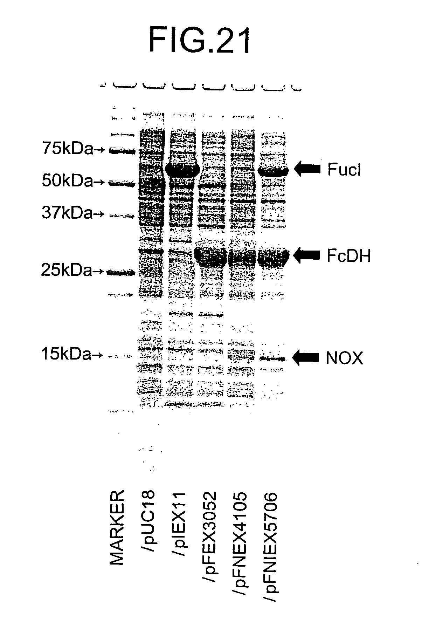

The washed microbial cells were resuspended in 25 mM Tris-HCl (pH 8.0), and disrupted by sonication (200 W, 10 minutes). Then the supernatant was obtained by centrifugation at 14,000 g for 15 minutes, and this was used as the cell free extract in the various experiments. The result of analysis of the cell free extracts obtained from *E. coli*/pUC18, *E. coli*/pIEX11, *E. coli*/pFEX3052, *E. coli*/pFNEX4105 and *E. coli*/pFNIEX5706 on SDS-PAGE is shown in FIG. 21. FucI was produced with *E. coli*/pIEX11. FcDH was produced with *E. coli*/pFEX3052. FcDH and NOX were produced with *E. coli*/pFNEX4105. FucI, FcDH and NOX were produced with *E. coli*/pFNIEX5706. Each band was observed on the position of the corresponding molecular weight. When the expressed protein was purified, this cell free extract was further subjected to ultracentrifugation at 200,000 g for 30 minutes, and the resulting supernatant was used as the material.

### (19-2) Purification of expressed recombinant FcDH (rFcDH)

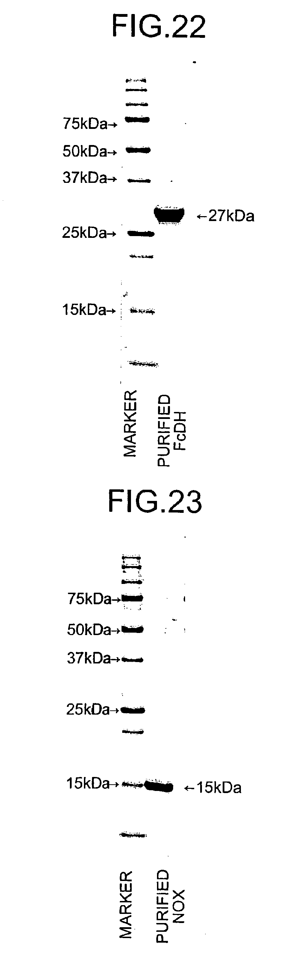

The supernatant obtained by ultracentrifugation of the cell free extract obtained from *E. coli*/pFEX3052 was purified. The crude enzyme solution was dialyzed against 50 mM potassium phosphate buffer (pH 6.0), and applied onto Q-Sepharose 16/10 that had been equilibrated with the same buffer. The protein absorbed in the carrier was eluted by increasing the NaCl concentration from 0 M to 0.5 M in the buffer. The fraction containing the FcDH activity was collected, concentrated and then applied onto Superdex 200 16/60 that had been equilibrated with 50 mM potassium phosphate buffer (pH 6.0). The fraction containing the FcDH activity was collected, concentrated, and its purity was then examined on SDS-PAGE. As a result, rFcDH was observed as the single band whose molecular weight was estimated as about 27 kDa (FIG. 22).

### (19-3) Purification of expressed recombinant NOX (rNOX)

The supernatant obtained by ultracentrifugation of the cell free extract of *E. coli*/pFNEX4105 was purified. The crude enzyme solution was applied onto FAD-agarose resin that had been equilibrated with 50 mM Tris-HCl (pH 8.0), and unabsorbed proteins were thoroughly washed with the same buffer. The absorbed protein was then eluted with the buffer containing 50 mM Tris-HCl (pH 8.0) and 1 mM FAD. By this operation, NOX was specifically absorbed in the resin, and eluted by the addition of the buffer containing FAD. The fraction containing the NOX activity was collected, concentrated, and then applied onto Superdex 200 16/60 that had been equilibrated with 50 mM Tris-HCl (pH 8.0), to collect the fraction containing the NOX activity. This was concentrated, and its purity was then examined on SDS-PAGE. As a result, rNOX was observed as the single band whose molecular weight was estimated as about 15 kDa (FIG. 23).

### (19-4) Measurement of enzyme activity

The activity of FcDH, NOX and FucI in the cell free extracts obtained from *E. coli*/pUC18, *E. coli*/pIEX11, *E. coli*/pFEX3052, *E. coli*/pFNEX4105 and *E. coli*/pFNIEX5706 was measured (Table 12). The activity of the purified preparations rFcDH and rNOX that had been purified as described above was also measured. The specific activity of rFcDH was 11.1 U/mg, and that of rNOX was 175 U/mg. These values can be regarded to be the same degrees as in the specific activities 7.8 U/mg of FcDH and 196 U/mg of NOX purified from *G. oxydans.*

**Table 12**

| Plasmids | ENZYME ACTIVITY (U/mg) | | |
|---|---|---|---|
| | FcDH | NOX | Fucl |
| pUC18 | ND* | 0.03 | ND* |
| pIEX11 | NT** | NT** | 0.84 |
| pFEX3052 | 3.08 | 0.04 | ND* |
| pFNEX4105 | 2.95 | 5.90 | ND* |
| pFNIEX5706 | 3.55 | 6.08 | 0.27 |

| | | | |
|---|---|---|---|
| *ND, Not detected **NT, Not tested | | | |

The FucI activity was measured as follows. The enzyme solution was appropriately added to 0.1 M L-fucose, 0.1 M Tris-HCl (pH 8.0) and 5 mM MgCl₂, and reacted at 30°C. The activity was quantified by measuring the amount of L-fuculose produced by the isomerization of L-fucose by HPLC. The activity was calculated from the initial rate in the range in which the amount of produced L-fuculose did not exceed 3 mM. As to the FucI activity, the activity to produce L-fuculose by isomerizing 1 µmol of L-fucose in one minute at 30°C was defined as 1 U.

The relationship between the expressed gene and the measured enzyme activity was reasonable, and it was believed that the fcdh gene and the nox gene encoded FcDH and NOX, respectively. The nox activity at a subtle level was observed in the experimental group in which nox had not been expressed, which was thought to be derived from the host *E. coli.*

### Example 20: Conversion of L-fucitol by enzyme reaction

### (20-1) Conversion by purified enzyme prepared from G. oxydans IFO 3255

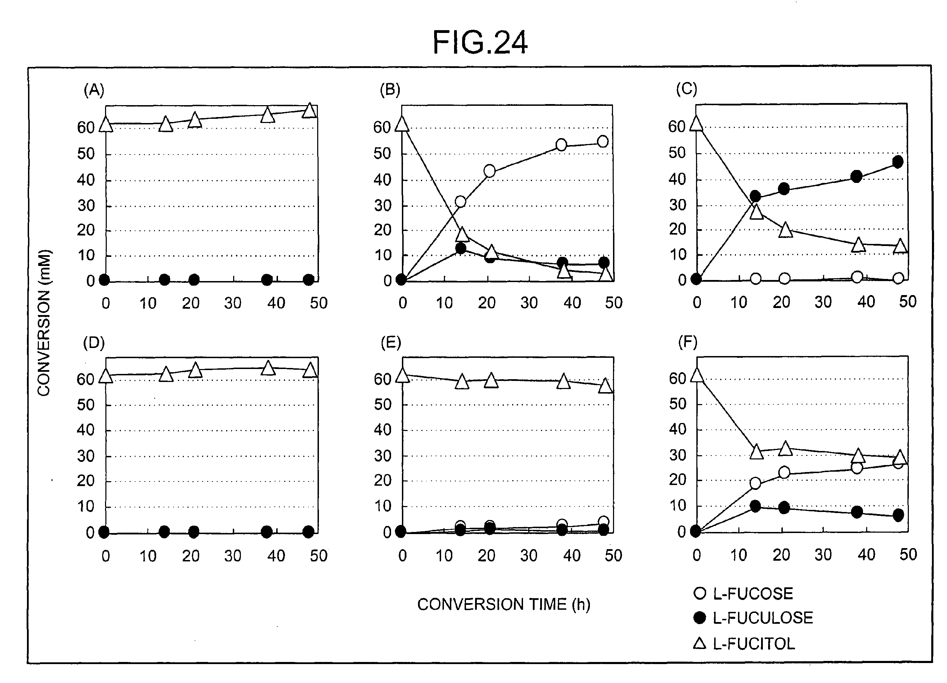

Using purified FcDH and NOX prepared from *G. oxydans,* the conversion from L-fucitol to L-fuculose or to L-fucose was attempted. The enzymatic conversion from L-fuculose to L-fucose requires L-fucose isomerase. FucIH6 prepared from *E. coli*/pQE30FucIH6 was used therefor. As the enzymes having the NOX activity, an H₂O producing type and an H₂O₂ producing type have been reported. In the case of the H₂O₂ producing type, as NADH is oxidized, H₂O₂ is produced. Generally, H₂O₂ has the nature to inhibit the activity of many enzymes. Thus, for the purpose of avoiding this, catalase was added as the enzyme which converts H₂O₂ into H₂O. As catalase, the commercially available preparation (supplied from Nacalai Tesque, derived from bovine liver) was used. As the standard reaction condition, the reaction solution containing 62 mM L-fucitol, 0.1 M Gly-NaOH (pH 9.5), 2 mM NAD, 30 µM FAD, 1 mM MgCl₂, 0. 1 U/ml purified FcDH, 0.1 U/ml purified NOX, 0.2 mg/ml purified FucIH6 and 0.1 mg/ml catalase was reacted at 30°C with shaking while sampling was appropriately performed. L-fucitol, L-fuculose and L-fucose therein were quantified by HPLC. The experimental groups and the analysis results after the reaction for 48 hours are shown in the following Table 13. The conversion time course in each experimental group is shown in FIG. 24.

**Table 13**

| EXPERIMENTAL GROUP | CONDITION | AFTER CONVERSION FOR 48 HOURS | | |
|---|---|---|---|---|
| | | L-FUCOSE (mM) | L-FUCULOSE (mM) | L-FUCITOL (mM) |
| A | NO ADDITION OF ENZYME | 0.0 | 0.0 | 67.1 |
| B | STANDARD CONDITION | 54.2 | 6.4 | 3.3 |
| C | NO ADDITION OF FucIH6 | 0.0 | 45.7 | 13.4 |
| D | NO ADDITION OF FcDH | 0.2 | 0.0 | 64.2 |
| E | NO ADDITION OF NOX | 3.9 | 0.7 | 57.4 |
| F | NO ADDITION OF CATALASE | 26.1 | 6.0 | 29.0 |

In each plot in FIG. 24, open circles, solid circles and open triangles represent the concentrations of L-fucose, L-fuculose and L-fucitol, respectively. (B) shows the result in the standard condition (62 mM L-fucitol, 0.1 M Gly-NaOH (pH 9.5), 2 mM NAD, 30 µM FAD, 1 mM MgCl₂, 0.1 U/ml purified FcDH, 0.1 U/ml purified NOX, 0.2 mg/ml purified FucIH6 and 0.1 mg/ml catalase). (A) shows the result without any addition of the enzymes, (C) shows the result without addition of FucIH6, (D) shows the result without addition of FcDH, (E) shows the result without addition of NOX and (F) shows the result without addition of catalase.

From the results shown in Table 13 and FIG. 24, the presence of both enzymes FcDH and NOX enabled the recycle of NAD(H), and the addition of 2 mM NAD led to production of 50 mM or more L-fuculose from L-fucitol. By the additional presence of FucI enzyme, produced L-fuculose was converted into L-fucose, and consequently it was possible to produce L-fucose from L-fucitol. The inhibition of the conversion was observed in the group in which catalase was not added. Thus, it was suggested that NOX used here was the H₂O₂ producing type, H₂O₂ inhibited the oxidation of L-fucitol and produced H₂O₂ could be removed by the addition of catalase.

### (20-2) Conversion by expressed recombinant enzyme prepared from E. coli

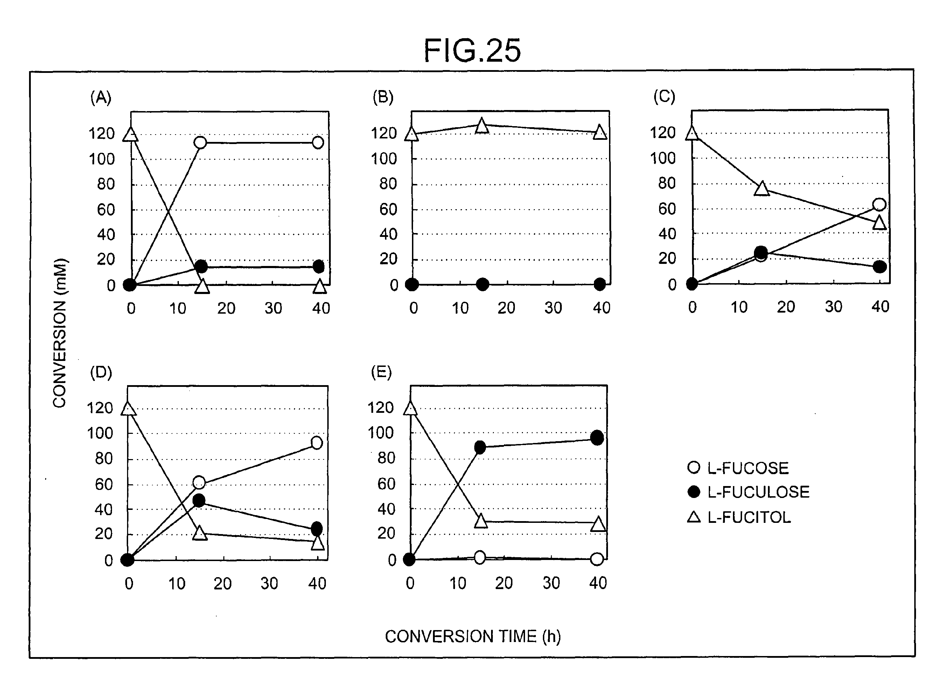

Using rFcDH and rNOX purified after the recombinant expression in *E. coli*, the enzymatic conversion of L-fucitol was attempted in the same way as in (20-1). As the standard reaction condition, the reaction solution containing 120 mM L-fucitol, 0.1 M Gly-NaOH (pH 9.5), 2 mM NAD, 30 µM FAD, 1 mM MgCl₂, 1 U/ml purified rFcDH, 1 U/ml purified rNOX, 0.2 mg/ml purified FucIH6 and 0.1 mg/ml catalase was reacted at 30°C with shaking, while sampling was appropriately performed. L-fucitol, L-fuculose and L-fucose were quantified by HPLC. The experimental groups and the analysis results after the reaction for 40 hours are shown in the following Table 14. The conversion time course in each experimental group is shown in FIG. 25.

**Table 14**

| EXPERIMENTAL GROUP | CONDITION | AFTER CONVERSION FOR 40 HOURS | | |
|---|---|---|---|---|
| | | L-FUCOSE (mM) | L-FUCULOSE (mM) | L-FUCITOL (mM) |
| A | STANDARD CONDITION | 113.0 | 14.8 | 0.0 |
| B | NO ADDITION OF NAD | 0.2 | 0.0 | 121.2 |
| C | NO ADDITION OF FAD | 62.3 | 13.0 | 48.7 |
| D | NO ADDITION OF CATALASE | 91.5 | 23.6 | 14.2 |
| E | NO ADDITION OF FuclH6 | 0.0 | 95.6 | 28.0 |

In each plot in FIG. 25, open circles, solid circles and open triangles represent the concentrations of L-fucose, L-fuculose and L-fucitol, respectively. (A) shows the result in the standard condition (120 mM L-fucitol, 0.1 M Gly-NaOH (pH 9.5), 2 mM NAD, 30 µM FAD, 1 mM MgCl₂, 1 U/ml purified rFcDH, 1 U/ml purified rNOX, 0.2 mg/ml purified FucIH6 and 0.1 mg/ml catalase). (B) shows the result without addition of NAD, (C) shows the result without addition of FAD, (D) shows the result without addition of catalase, and (E) shows the result without addition of FucIH6.

From the results shown in Table 14 and FIG. 25, the presence of both enzymes rFcDH and rNOX enabled the recycle of NAD(H) as was the case of FcDH and NOX prepared from *G. oxydans,* and the addition of 2 mM NAD led to production of 100 mM or more L-fuculose from L-fucitol. By the coexistence of FucI enzyme, produced L-fuculose was converted into L-fucose, and consequently it was possible to produce L-fucose from L-fucitol. The effect of catalase addition was observed as in the above (20-1). In the present experiment, the necessity of the addition of the coenzymes NAD and FAD was also examined. In the experimental group in which NAD was not added, the conversion of L-fucitol did not proceed at all even though both enzymes FcDH and NOX were present. Thus, it appeared that the addition of NAD at least in a catalytic amount was necessary for the conversion. In the experimental group in which FAD was not added, although the conversion proceeds, the conversion rate was delayed compared with the group in which FAD was added. Thus, as shown in Example 15-5, it was shown that the activation of NOX by the addition of the flavin coenzyme was effective for the conversion.

### Example 21: Conversion of L-fucitol by recombinant E. coli microbial cell reaction

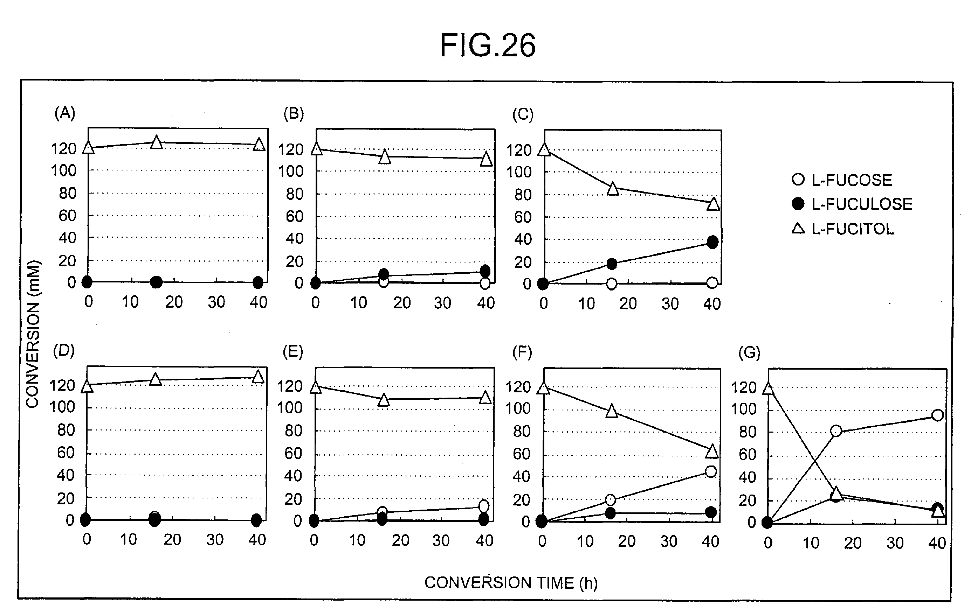

The experiment was performed for the purpose of converting L-fucitol by intact microbial cells without taking the produced enzyme out of the microbial cells. The bacterial strains, *E. coli*/pUC18, *E. coli*/pIEX11, *E. coli*/pFEX3052, *E. coli*/pFNEX4105 and *E. coli*/pFNIEX5706 were used alone or in combination. As the standard reaction condition, the reaction solution containing 120 mM L-fucitol, 0.2 M Gly-NaOH (pH 9.5), 50 uM riboflavin, 1 mM MgC1₂ and the washed microbial cells (adjusted so that the final concentration in the reaction solution was A₆₁₀=5.0) was reacted at 30°C with shaking, while sampling was appropriately performed. L-fucitol, L-fuculose and L-fucose were quantified by HPLC. *E. coli*/pUC18, *E. coli*/pFEX3052, *E. coli*/pFNEX4105 and *E. coli*/pFNIEX5706 were used for the reaction using one type of the microbial cells. *E. coli*/pIEX11 was used as the additional second type of the microbial cells. In the latter case, the final concentration of the first type microbial cells was A₆₁₀=5.0, and the final concentration of *E. coli*/pIEX11 was A₆₁₀=2.5. The experimental groups and the analysis results after the reaction for 40 hours are shown in the following Table 15. The conversion time course in each experimental group is shown in FIG. 26.

**Table 15**

| EXPERIMENTAL GROUP | BACTERIAL STRAIN | AFTER CONVERSION FOR 40 HOURS | | |
|---|---|---|---|---|
| | | L-FUCOSE (mM) | L-FUCULOSE (mM) | L-FUCITOL (mM) |
| A | E. coli/pUC18 | 0.0 | 0.0 | 123.8 |
| B | E. coli/pFEX3052 | 0.1 | 10.3 | 112.5 |
| C | E. coli/pFNEX4105 | 1.7 | 37.6 | 72.9 |
| D | E. coli/pUC18 + /plEX11 | 0.3 | 0.0 | 128.9 |
| E | E. coli/pFEX3052 + /pIEX11 | 12.9 | 0.7 | 110.3 |
| F | E. coli/pFNEX4105 + /pIEX11 | 45.3 | 7.7 | 63.9 |
| G | E. coli/pFNIEX5706 | 95.7 | 13.0 | 12.2 |

In each plot in FIG. 26, open circles, solid circles and open triangles represent the concentrations of L-fucose, L-fuculose and L-fucitol, respectively. The reaction solution was composed of 120 mM L-fucitol, 0.2 M Gly-NaOH (pH 9.5), 50 µM riboflavin, 1 mM MgCl₂ and the washed microbial cells adjusted so that the final concentration in the reaction solution was A₆₁₀=5.0. (A) and (D) show the results using *E. coli*/pUC18, (B) and (E) show the results using *E. coli*/pFEX3052, (C) and (F) show the results using *E. coli*/pFNEX4105, and (G) shows the results using *E. coli*/pFNIEX5706. In (D), (E) and (F), the washed microbial cells of *E. coli*/pIEX11 adjusted to be A₆₁₀=2.5 were added in addition to the aforementioned composition.

As a result, as shown in the experimental group (B), the production of L-fuculose by FcDH-expressing strain *E. coli*/pFEX3052 was observed whereas as shown in the experimental group (A), L-fucitol was not converted at all by the control strain *E. coli*/pUC18. This demonstrates that NAD present in the host microbial cell can work as the coenzyme in the reaction by the intact microbial cell, and that the host has the slight NOX activity. The productivity of L-fuculose was enhanced in the experimental group (C) of the FcDH and NOX expressing strain *E. coli*/pFNEX4105 compared with the group of the FcDH expressing strain, suggesting that the expression of NOX might efficiently recycled NAD(H). As shown in the experimental groups (E) and (F), it was observed that produced L-fuculose was converted into L-fucose by the coexistence of FucI expressing strain *E. coli*/pIEX11 in the conversion by *E. coli*/pFEX3052 or E. coli/pFNEX4105. Furthermore, as shown in the experimental group (G), in the conversion using *E. coli*/pFNIEX5706 which expresses the three enzymes, FcDH, NOX and FucI, it was demonstrated that L-fucitol was converted into L-fucose by a single bacterial strain.

### Example 22: Preparation of FcDH+NOX+FucI+KatE-expressing strain and cultivation thereof

The production of H₂O₂ which seemed to be derived from the NOX activity inhibited the conversion of L-fucitol, and the addition of catalase led to removal of the inhibition. Thus, a catalase gene was further introduced into the FcDH+NOX+FucI co-expressing strain, to obtain a bacterial strain which further co-express catalase. KatE protein derived from E. coli was used as catalase. The gene encoding this enzyme has been registered as accession number M55161 in the database in National Center for Biotechnology Information. For the purpose of expressing this enzyme in a large amount in E. coli, the following PCR primers (SEQ ID NOS:28 and 29) were prepared. The PCR was performed with the genomic DNA derived from *E. coli* W3110 strain obtained by the standard method as the template, to yield a fragment of about 2.5 kbp. The resulting PCR product was digested with SalI and PstI, and introduced into the corresponding position in the plasmid pSTV28 (supplied from Takara Bio Inc.) to yield the plasmid pKEX5804 for KatE expression. *E.coli*/pFNIEX5706 strain was transformed with this plasmid to make the bacterial strain *E.coli*/FNIC7001 having both plasmids pFNIEX5706 and pCEX8401

SEQ ID NO:28: Nucleotide sequence of 5' primer for obtaining KatE
GGCTTCACTAGTCGACTATTAAAAATCAGAAAAAC
SEQ ID NO:29: Nucleotide sequence of 3' primer for obtaining KatE
ATGTAAATCCTGCAGGCGGCGCAATTGCGCGCTCC

This strain was refreshed by culturing on LB/Amp+Cm (chloramphenicol 0.03 mg/ml) plates at 37°C. The liquid cultivation was performed at 37°C using the LB/Amp+Cm liquid medium. After inoculating the refreshed microbial cells, IPTG at a final concentration of 1 mM was added. The microbial cells were cultured for 6 to 18 hours, and then collected by centrifugation. The collected microbial cells were washed with 25 mM Tris-HCl buffer (pH 8.0), and used in the subsequent experiments. The TB medium (12 g/l trypton, 24 g/l yeast extract, 4 g/l glycerol, 2.3 g/l KH₂PO₄, 12.5 g/l K₂HPO₄) was used instead of the LB medium in some cases, and they were not essentially different except that the larger amount of the microbial cells was obtained.

### Example 23: Measurement of enzyme activity in cell free extract of E.coli/FNIC7001 strain

After culturing at 30°C or 37°C using the TB medium, the microbial cells were collected. The washed microbial cells were resuspended in 25 mM Tris-HCl (pH 8.0), disrupted by sonication (200W, 10 minutes), and centrifuged at 14,000 g for 15 minutes to yield the supernatant. This cell free extract was used as the enzyme source in the measurement of the enzyme activity. *E.coli*/pFNIEX5706 strain was also used as a control.

The KatE activity was measured by the following standard condition. That is, 0.5 volume of 59 mM H₂O₂ was added to one volume of the enzyme solution appropriately diluted with 0.1 M Tris-HCl (pH 8.0), and reacted at 30°C. The activity was quantified by monitoring the reduction of absorbance at 240 nm caused by the reduction of H₂O₂. As the absorbance coefficient of H₂O₂, e₂₄₀=43.6M⁻¹cm was used. The measurement was started at the addition of H₂O and the activity was calculated by taking the change of absorbance in the first one minute as the initial rate. The experimental group in which the enzyme source was not added was made as blank. The KatE activity to consume 1 µmol H₂O₂ in one minute at 30°C was defined as 1 U. The enzyme activity measured is shown in Table 16. At both 30°C and 37°C, *E*.*coli*/FNIC7001 strain having pKEX5804 had higher KatE activity than *E*.*coli*/pFNIEX5706 strain.

**Table 16**

| Strain | Plasmid | CULTIVATION TEMPERATURE (°C) | KatE ACTIVITY (U/mg) |
|---|---|---|---|
| E.coli/ | pFNIEX5706 | 30 | 3.2 |
| pFNIEX5706 | NO ADDITION OF NAD | 37 | 2.5 |
| E.coli/ | pFNIEX5706 | 30 | 51.4 |
| FNIC7001 | +pKEX5804 | 37 | 55.0 |

### Example 24: Conversion of L-fucitol by E.coli/FNIC7001 strain

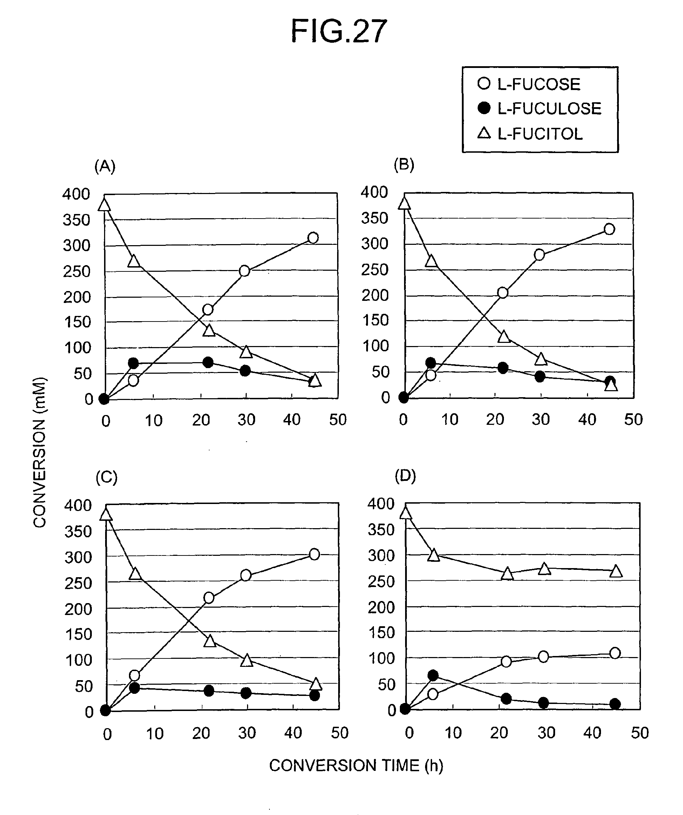

The conversion experiment of L-fucitol by the washed intact microbial cells of *E.coli*/FNIC7001 strain cultured at 27, 30, 33 or 37°C using TB/Amp+Cm was performed. The reaction solution containing 380 mM L-fucitol, 0.2 M Gly-NaOH (pH 9.5), 50 µM riboflavin, 1 mM MnCl₂, 0.1 mg/ml Amp, 0.03 mg/ml Cm, and washed microbial cells (adjusted so that the final concentration in the reaction solution was A₆₁₀=10.0) was reacted at 30°C with shaking, while sampling was appropriately performed. L-fucitol, L-fuculose and L-fucose were quantified by HPLC. The experimental groups and the analysis results after the reaction for 45 hours are shown in the following Table 17, and the conversion time course in each experimental group is shown in FIG. 27. The enzyme activity in the cell free extract prepared from the microbial cells cultured at each temperature is shown in Table 18.

**Table 17**

| EXPERIMENTAL GROUP | CULTIVATION TEMPERATURE OF BACTERIAL STRAIN (°C) | AFTER CONVERSION FOR 45 HOURS | | |
|---|---|---|---|---|
| | | L-FUCOSE (mM) | L-FUCULOSE (mM) | L-FUCITOL (mM) |
| A | 27 | 312.2 | 31.3 | 36.2 |
| B | 30 | 327.1 | 30.3 | 26.9 |
| C | 33 | 299.4 | 27.4 | 50.3 |
| D | 37 | 108.5 | 9.0 | 269.3 |

**Table 18**

| CULTIVATION TEMPERATURE (°C) | ENZYME ACTIVITY (U/mg) | | | |
|---|---|---|---|---|
| | FcDH | NOX | Fucl | KatE |
| 27 | 0.57 | 1.32 | 0.41 | 51.3 |
| 30 | 0.65 | 1.62 | 0.42 | 52.0 |
| 33 | 1.63 | 4.71 | 0.87 | 57.3 |
| 37 | 2.05 | 6.49 | 0.92 | 50.8 |

In each plot in FIG. 27, open circles, solid circles and open triangles represent the concentrations of L-fucose, L-fuculose and L-fucitol, respectively. The reaction solution was composed of 380 mM L-fucitol, 0.2 M Gly-NaOH (pH 9.5), 50 µM riboflavin, 1 mM MgCl₂, 0.1 mg/ml Amp, 0.03 mg/ml Cm, and the washed microbial cells adjusted so that the final concentration in the reaction solution was A₆₁₀=10.0. The microbial cells cultured at 27°C (FIG. A), 30°C (FIG. B), 33°C (FIG. C) or 37°C (FIG. D) were used for the conversion reaction.

As a result, the higher the microbial cell culture temperature was, the higher the enzyme activity per unit protein amount tended to be. However, in the conversion reaction using the intact microbial cells, the conversion rate was faster in the cases of culturing at 27, 30 or 33°C than in the case of 37°C.

### INDUSTRIAL APPLICABILITY

The present invention is suitable for the industrial production of L-fucose. The present invention is expected to contribute to various fields utilizing L-fucose.

### SEQUENCE LISTING FREE TEXT

SEQ ID NO:1 5' Primer for obtaining fucI gene
SEQ ID NO:2 3' Primer for obtaining fucI gene
SEQ ID NO:3 Nucleotide sequence of fucI gene derived from *E. coli*
SEQ ID NO:4 Amino acid sequence of FucI derived from E. coli
SEQ ID NO:5 PCR primer for obtaining fuel gene
SEQ ID NO:6 PCR primer for obtaining fucI gene
SEQ ID NO:7 5' Primer for obtaining sldA gene
SEQ ID NO:8 3' Primer for obtaining sldA gene
SEQ ID NO:9 5' Primer for obtaining Kmr gene
SEQ ID NO:10 3' Primer for obtaining Kmr gene
SEQ ID NO:11 Amino acid sequence of peptide fragment F1 derived from FcDH
SEQ ID NO:12 Amino acid sequence of peptide fragment F2 derived from FcDH
SEQ ID NO:13 Amino acid sequence of peptide fragment N1 derived from NOX
SEQ ID NO:14 Amino acid sequence of peptide fragment N2 derived from NOX
SEQ ID NO:15 Nucleotide sequence of gene fcdh encoding FcDH
SEQ ID NO:16 Amino acid sequence of FcDH
SEQ ID NO:17 Nucleotide sequence of gene nox encoding NOX
SEQ ID NO:18 Amino acid sequence of NOX
SEQ ID NO:19 5' Primer for obtaining fcdh
SEQ ID NO:20 3' Primer for obtaining fcdh
SEQ ID NO:21 5' Primer for obtaining nox
SEQ ID NO:22 3' Primer for obtaining nox
SEQ ID NO:23 3' Primer for obtaining fcdh+nox
SEQ ID NO:24 5' Primer for obtaining fucI
SEQ ID NO:25 3' Primer for obtaining fucI
SEQ ID NO:26 5' Primer for obtaining fucI (single expression)
SEQ ID NO:27 3' Primer for obtaining fucI (single expression)
SEQ ID NO:28 5' Primer for obtaining KatE
SEQ ID NO:29 3' Primer for obtaining KatE

## Claims

1. A method for producing L-fuculose comprising contacting L-fucitol with a protein derived from a microorganism, the protein having a dehydrogenase activity to synthesize L-fuculose from L-fucitol.

2. The method for producing L-fuculose according to claim 1 wherein said microorganism is an acetobacterium.

3. A method for producing L-fuculose comprising contacting L-fucitol with one or more selected from the group consisting of a microorganism having an ability to synthesize L-fuculose from L-fucitol to an extent so that the amount of L-fuculose thus produced accounts for 50% by weight or more of total L-fucitol oxide, a culture of the microorganism and a treated microbial cell product of the microorganism.

4. A method for producing L-fuculose comprising contacting L-fucitol with one or more selected from the group consisting of:
at least one microorganism belonging to *Gluconobacter xylinus subsp. xylinus* and *Gluconobacter oxydans,*
a culture of the microorganism, and
a treated microbial cell product of the microorganism.

5. A method for producing L-fuculose comprising contacting L-fucitol with one or more selected from the group consisting of:
a non-genetically engineered microorganism having a protein having a dehydrogenase activity to synthesize L-fuculose from L-fucitol, the microorganism not substantially having an ability to synthesize ketohexose other than L-fuculose from L-fucitol,
a culture of the non-genetically engineered microorganism, and
a treated microbial cell product of the non-genetically engineered microorganism.

6. A method for producing L-fuculose comprising contacting L-fucitol with one or more selected from the group consisting of:
a microorganism having a first protein derived from a microorganism having a dehydrogenase activity to synthesize L-fuculose from L-fucitol, wherein a gene encoding a second protein having an activity to synthesize ketohexose other than L-fuculose from L-fucitol has been knocked out,
a culture of the microorganism, and
a treated microbial cell product of the microorganism.

7. A method for producing L-fuculose comprising contacting L-fucitol with one or more selected from the group consisting of:
a microorganism substantially lacking an ability to synthesize ketohexose other than L-fuculose from fucitol, the microorganism being transformed to be capable of expressing a first microorganism-derived protein having a dehydrogenase activity to synthesize L-fuculose from L-fucitol,
a culture of the microorganism, and
a treated microbial cell product of the microorganism.

8. The method for producing L-fuculose according to claim 7 wherein said microorganism is a microorganism wherein a gene encoding a second protein having an activity to synthesize ketohexose other than L-fuculose from L-fucitol has been knocked out.

9. The method for producing L-fuculose according to any one of claims 1 to 4 wherein L-fuculose is synthesized from L-fucitol under a condition where synthesis of ketohexose other than L-fuculose from L-fucitol is inhibited.

10. The method for producing L-fuculose according to any one of claims 1 to 4 wherein L-fuculose is synthesized from L-fucitol under a pH condition where synthesis of ketohexose other than L-fuculose from L-fucitol is inhibited.

11. The method for producing L-fuculose according to any one of claims 1 to 4 wherein L-fuculose is synthesized from L-fucitol in the coexistence of a bivalent ion chelator under the condition where synthesis of ketohexose other than L-fuculose from L-fucitol is inhibited.

12. The method for producing L-fuculose according to any one of [claims 1 to 8 wherein L-fuculose is synthesized from L-fucitol in the presence of a protein having an activity to produce NAD from NADH.

13. A method for producing L-fucose comprising:
a step of synthesizing L-fuculose wherein L-fucitol is contacted with a protein derived from a microorganism having a dehydrogenase activity to synthesize L-fuculose from L-fucitol, and
a step synthesizing L-fucose wherein L-fuculose is contacted with a protein having an activity to synthesize L-fucose from L-fuculose.

14. A method for producing L-fucose comprising:
a step of synthesizing L-fuculose wherein L-fucitol is contacted with one or more selected from the group consisting of a microorganism having an ability to synthesize L-fuculose from L-fucitol to an extent so that the amount of L-fuculose thus produced accounts for 50% by weight or more of total L-fucitol oxide, a culture of the microorganism and a treated microbial cell product of the microorganism, and
a step synthesizing L-fucose wherein L-fuculose is contacted with a protein having an activity to synthesize L-fucose from L-fuculose.

15. A method for producing L-fucose comprising:
a step of synthesizing L-fuculose wherein L-fucitol is contacted with one or more selected from the group consisting of a non-genetically engineered microorganism having a protein having a dehydrogenase activity to synthesize L-fuculose from L-fucitol, the microorganism not substantially having an ability to synthesize ketohexose other than L-fuculose from L-fucitol; a culture of the non-genetically engineered microorganism; and a treated microbial cell product of the non-genetically engineered microorganism, and
a step synthesizing L-fucose wherein L-fuculose is contacted with a protein having an activity to synthesize L-fucose from L-fuculose.

16. A method for producing L-fucose comprising:
a step of synthesizing L-fuculose wherein L-fucitol is contacted with one or more selected from the group consisting of a microorganism having a protein having a dehydrogenase activity to synthesize L-fuculose from L-fucitol, wherein a gene encoding a protein having an activity to synthesize ketohexose other than L-fuculose from L-fucitol has been knocked out; a culture of the microorganism; and a treated microbial cell product of the microorganism, and
a step synthesizing L-fucose wherein L-fuculose is contacted with a protein having an activity to synthesize L-fucose from L-fuculose.

17. A method for producing L-fucose comprising:
a step of synthesizing L-fuculose wherein L-fucitol is contacted with one or more selected from the group consisting of a microorganism substantially lacking an ability to synthesize ketohexose other than L-fuculose from L-fucitol, the microorganism being transformed to be capable of expressing a first microorganism-derived protein having a dehydrogenase activity to synthesize L-fuculose from L-fucitol; a culture of the microorganism; and a treated microbial cell product of the microorganism, and
a step synthesizing L-fucose wherein L-fuculose is contacted with a protein having an activity to synthesize L-fucose from L-fuculose.

18. A method for producing L-fucose comprising contacting L-fucitol with one or more selected from the group consisting of:
a microorganism substantially lacking an ability to synthesize ketohexose other than L-fuculose from L-fucitol, the microorganism being transformed to be capable of expressing a first microorganism-derived protein having a dehydrogenase activity to synthesize L-fuculose from L-fucitol and another protein having an activity to synthesize L-fucose from L-fuculose,
a culture of the microorganism, and
a treated microbial cell product of the microorganism.

19. A protein of the following (A) or (B):
(A) a protein having an amino acid sequence according to SEQ ID NO:16, or
(B) a protein having an amino acid sequence including one or several amino acid mutations selected from the group consisting of substitution, deletion, insertion, addition and inversion in the amino acid sequence according to SEQ ID NO:16, and having a dehydrogenase activity to synthesize L-fuculose from L-fucitol.

20. A polynucleotide encoding the protein according to claim 19.

21. A polynucleotide of the following (a) or (b):
(a) a polynucleotide having a nucleotide sequence according to SEQ ID NO:15, or
(b) a polynucleotide which hybridizes with a polynucleotide having a nucleotide sequence complementary to the nucleotide sequence according to SEQ ID NO:15 under a stringent condition and encodes a protein having a dehydrogenase activity to synthesize L-fuculose from L-fucitol.

22. A protein of the following (C) or (D):
(C) a protein having an amino acid sequence according to SEQ ID NO:18, or
(D) a protein having an amino acid sequence including one or several amino acid mutations selected from the group consisting of substitution, deletion, insertion, addition and inversion in the amino acid sequence according to SEQ ID NO:18, and having an NADH oxidase activity.

23. A polynucleotide encoding the protein according to claim 22.

24. A polynucleotide of the following (c) or (d):
(c) a polynucleotide having a nucleotide sequence according to SEQ ID NO:17, or
(d) a polynucleotide which hybridizes with a polynucleotide having a nucleotide sequence complementary to the nucleotide sequence according to SEQ ID NO:17 under a stringent condition and encodes a protein having an activity to produce NAD from NADH.

25. A recombinant polynucleotide comprising the polynucleotide according to claim 20 incorporated therein.

26. A recombinant polynucleotide comprising the polynucleotide according to claim 23 incorporated therein.

27. A recombinant polynucleotide comprising the polynucleotide according to claim 20 and the polynucleotide according to claim 23 incorporated therein.

28. A transformant comprising the recombinant polynucleotide according to claim 25, the transformant being capable of expressing a protein having a dehydrogenase activity to synthesize L-fuculose from L-fucitol.

29. A method for producing a protein comprising culturing the transformant according to claim 28, wherein the transformant is a microorganism, in a medium to accumulate the protein having a dehydrogenase activity to synthesize L-fuculose from L-fucitol in the medium and/or in the microorganism.

30. A method for producing L-fuculose comprising adding one or more selected from the group consisting the transformant according to claim 28 wherein the transformant is a microorganism, a culture of the microorganism and a treated microbial cell product of the microorganism, to a reaction system containing L-fucitol to synthesize L-fuculose from L-fucitol.

31. A method for producing L-fucose comprising:
a step of synthesizing L-fuculose wherein one or more selected from the group consisting of the transformant according to claim 28 wherein the transformant is a microorganism, a culture of the microorganism and a treated microbial cell product of the microorganism are added to a reaction system containing L-fucitol to synthesize L-fuculose from L-fucitol, and
a step synthesizing L-fucose wherein L-fuculose is contacted with a protein having an activity to synthesize L-fucose from L-fuculose.

32. A transformant comprising the recombinant polynucleotide according to claim 25 and the recombinant polynucleotide according to claim 26 incorporated therein, the microorganism being capable of expressing a protein having a dehydrogenase activity to synthesize L-fuculose from L-fucitol and another protein having an activity to produce NAD from NADH.

33. A method for producing a protein comprising culturing a transformed microorganism in which the recombinant polynucleotide according to claim 26 has been introduced, the microorganism being capable of expressing an protein having an activity to produce NAD from NADH, in a medium to accumulate the protein having the activity to produce NAD from NADH in the medium and/or in the microorganism.

34. A method for producing L-fuculose comprising adding one or more selected from the group consisting of the transformant according to claim 32 wherein the transformant is a microorganism, a culture of the microorganism and a treated microbial cell product of the microorganism, to a reaction system containing L-fucitol to synthesize L-fuculose from L-fucitol.

35. A method for producing L-fucose comprising:
a step of synthesizing L-fuculose wherein one or more selected from the group consisting of the transformant according to claim 32 wherein the transformant is a microorganism, a culture of the microorganism and a treated microbial cell product of the microorganism are added to a reaction system containing L-fucitol to synthesize L-fuculose from L-fucitol, and
a step synthesizing L-fucose wherein L-fuculose is contacted with a protein having an activity to synthesize L-fucose from L-fuculose.

36. A transformant comprising the recombinant polynucleotide according to claim 27 introduced therein, the transformant being capable of expressing a protein having a dehydrogenase activity to synthesize L-fuculose from L-fucitol and another protein having an activity to produce NAD from NADH.

37. A method for producing a protein comprising culturing the transformant according to claim 36, wherein the transformant is a microorganism, in a medium to accumulate the protein having a dehydrogenase activity to synthesize L-fuculose from L-fucitol and the protein having an activity to produce NAD from NADH in the medium and/or in the microorganism.

38. A method for producing L-fuculose comprising adding one or more selected from the group consisting of the transformant according to claims 36 wherein the transformant is a microorganism, a culture of the microorganism and a treated microbial cell product of the microorganism, to a reaction system containing L-fucitol to synthesize L-fuculose from L-fucitol.

39. A method for producing L-fucose comprising:
a step of synthesizing L-fuculose wherein one or more selected from the group consisting of the transformant according to claim 36 wherein the transformant is a microorganism, a culture of the microorganism and a treated microbial cell product of the microorganism are added to a reaction system containing L-fucitol to synthesize L-fuculose from L-fucitol, and
a step synthesizing L-fucose wherein L-fuculose is contacted with a protein having an activity to synthesize L-fucose from L-fuculose.
